# EUROPÄISCHE PATENTSCHRIFT

(11) **EP 0 368 429 B1**
(45) Veröffentlichungstag und Bekanntmachung des Hinweises auf die Patenterteilung: **09.02.1994**
(21) Anmeldenummer: 89250053.9
(22) Anmeldetag: 05.10.1989
(51) Int. Cl.: C07C 311/21, C07C 311/13, C07C 311/09, C07C 311/08, C07C 237/44, C07C 233/44, C07C 235/48, A61K 31/165, A61K 31/18

(54) **Fluorsubstituierte Benzolderivate**
Fluor-substituted benzene derivatives
Dérivés de benzène fluorés

(30) Priorität: 07.10.1988 DE 3834704
(43) Veröffentlichungstag der Anmeldung: 16.05.1990
(60) Teilanmeldung: 93111849.1
(73) Patentinhaber: SCHERING AKTIENGESELLSCHAFT, 13342 Berlin (DE)
(72) Erfinder: Blaszkiewicz, Peter, Dr., D-1000 Berlin 41 (DE); Niedballa, Ulrich, Dr., D-1000 Berlin 33 (DE); Gries, Heinz, Dr., D-1000 Berlin 31 (DE); Bauer, Hans, Dr., D-1000 Berlin 42 (DE); Weinmann, Hanns-Joachim, Dr., D-1000 Berlin 38 (DE)

(56) Entgegenhaltungen:
- BE-A- 718 129
- DE-A- 1 917 821
- FR-A- 1 579 473
- US-A- 3 920 444
- US-A- 4 766 243
- JOURNAL OF AGRICULTURAL AND FOOD CHEMISTRY, Band 22, Nr. 6, November-Dezember 1974, S. 921-1166, The American Chemical Society, Washington, US; R. D. TREPKA et al: "Synthesis and herbicidal activity of fluorinated N-phenylalkanesulfonamides"

## Beschreibung

Die Erfindung betrifft den in den Patentansprüchen gekennzeichneten Gegenstand, das heißt Benzolderivate, die mindestens zwei Fluoratome enthalten, ihre Verwendung als NMR-Diagnostika, diagnostische Mittel, die diese Benzolderivate enthalten sowie Verfahren zur Herstellung dieser Verbindungen und Mittel.

Die moderne Medizintechnik ermöglicht es, kleinste morphologische Strukturen mit einer Auflösung darzustellen, die der von Gewebeschnitten aus Anatomielehrbüchern nahekommt.

Es gelingt jedoch auch mit Hilfe der Ultraschall-, der Röntgen-Diagnostik, der Nuklearmedizin und selbst der Kernspintomographie nicht, Informationen über den stoffwechselphysiologischen Zustand eines Gewebes des lebenden Organismus zu erhalten. Für eine genauere Diagnose und insbesondere für eine Planung und Verlaufskontrolle eines therapeutischen Einsatzes ist jedoch diese Kenntnis von erheblicher Bedeutung, da eine optimale Therapie nur dann erfolgreich sein kann, wenn frühzeitig eine Aussage über deren Wirkung möglich ist.

Ein wichtiger Parameter der stoffwechselphysiologischen Aktivität ist der pH-Wert. Viele pathologische Prozesse haben eine Veränderung der Wasserstoffionen-Konzentration zur Folge. Eines der bekanntesten Beispiele ist die Freisetzung von Milchsäure in Folge ungenügender Sauerstoffversorgung und dadurch bedingte anaerober Verstoffwechselung von Glucose. Eine anaerobe Glykolyse findet praktisch überall dort statt, wo eine ausreichende Versorgung mit Sauerstoff nicht mehr gewährleistet ist. Eine kurzfristige Ansäuerung ist zum Beispiel in Bereichen höchster Muskelaktivität festzustellen. Hier wird jedoch die anfallende Milchsäure in der Ruhepause relativ rasch abtransportiert, so daß im ruhenden Muskel keine Übersäuerung festzustellen ist. Anders sieht es jedoch in Bereichen permanenter Sauerstoffschuld aus. In ischaemischen Arealen (Infarkt) kommt es aufgrund der gesteigerten anaeroben Glykolyse zu einer Verschiebung des pH-Wertes. Ähnliche Effekte sind in schnell wachsenden Neoplasmen zu beobachten. Neben einer Regulationsstörung liegt im Bereich eines Tumors ein Sauerstoffmangel vor, so daß auch hier durch anaerobe Verstoffwechselung von Kohlehydraten eine Ansäuerung auftritt.

Die Bestimmung des Gewebe-pH-Werts führt somit zu wichtigen Aussagen über Funktion, Zustand und Wachstum der Zellen, so daß es generell wünschenswert ist, metabolische Azidosen zu lokalisieren. (Am. J. Physiol. 246, R 409, 1984; R. Nuccitelli, D.W. Deamer, Eds. 1982 Intracellular pH: Its Measurement, Regulation and Utilization in Cellular Functions, Liss. New York). Neben der Messung des pH-Wertes mit pH-Elektroden wurde neuerdings auch die NMR-Spektroskopie zu diesem Zweck eingesetzt. Mit deren Hilfe ist es erstmals gelungen, den pH-Wert des Gewebes ohne äußere Beeinflussung zu bestimmen.

Die Bestimmung des pH-Wertes mit Hilfe der NMR-Spektroskopie beruht auf der Messung der Signale einer chemischen Verbindung, die sich in einem pH-abhängigen, reversiblen Gleichgewicht befindet. Ist dieses Gleichgewicht langsam bezuglich der NMR-Zeitskala, so können die Signale sämtlicher Komponenten erhalten werden und die Signalintensitäten entsprechen den Konzentrationen der Gleichgewichtskomponenten. Bei einem schnellen Gleichgewicht ist dagegen nur ein Signal meßbar und die chemische Verschiebung ist durch die chemische Verschiebung der Gleichgewichtskomponenten und deren Konzentration gegeben.

In einem Zweikomponenten-Gleichgewicht kann dann, bei Kenntnis des pKₐ-Wertes und der chemischen Verschiebung der Komponenten, der pH-Wert mit Hilfe der Henderson-Hasselbalch-Gleichung berechnet werden.

Aus der folgenden Tabelle ist ersichtlich, welche Atomkerne prinzipiell für die NMR-Bildgebung bzw. -Spektroskopie in Frage kommen:

| Kern | Frequenz bei 1 Tesla MHz | rel. Meßempfindlichkeit ¹H = 1 | Konzentration in biol. Gewebe | Chemical Shift | chemische Modifikationsmöglichkeit | T₁-Relaxationszeiten |
|---|---|---|---|---|---|---|
| ¹H | 42.6 | 1,0 | 100 mol/L | klein | sehr hoch | 0.1-3 sec |
| ¹⁹F | 40.1 | 0.8 | << 1 mmol/L | sehr groß | sehr hoch | 1-5 sec |
| ²³Na | 11.3 | 0.09 | 100 mmol/L | ./. | praktisch null | < 0.1 sec |
| ³¹P | 17.2 | 0.06 | 10 mmol/L | mittel | begrenzt | 1-5 sec |
| ¹³C | 10.7 | 0.0002 | 1 mmol/L | sehr groß | sehr hoch | 1-10 sec |

Seit 15 Jahren wird der ³¹P-Kern als nicht-invasive Meßsonde für die intracelluläre pH-Wert-Messung eingesetzt (J. Biol. Chem. 248, 7276, 1973).
Das pH-empfindliche Signal ist hierbei das Signal des anorganischen Phosphats aus dem Gleichgewicht Hydrogenphosphat-Dihydrogenphosphat; als Referenz dient das ³¹P-Signal des Phosphorkreatins.
Die Verwendung des ³¹P-Kerns für die pH-Wert-Bestimmung hat jedoch auch ihre Grenzen: So ist eine exakte Bestimmung des pH-Wertes in einem gut lokalisierten Gewebevolumen beim Menschen selbst mit dem Einsatz von 2T Kernspintomographen nicht möglich. Dies liegt sowohl an den relativ niedrigen Phosphatkonzentrationen sowie an der Tatsache, daß das ³¹P-Signal meßtechnisch schwierig zu erfassen ist. Störsignale im Bereich des anorganischen Phosphats, Überlagerung des anorganischen P-Signals durch andere P-Metabolite oder das Fehlen eines Referenzsignals können eine pH-Messung verhindern. Weitere Schwierigkeiten liegen in der geringen Empfindlichkeit des Kerns und der geringen pH-Abhängigkeit der chemischen Verschiebung. Die Genauigkeit der pH-Messung wird vor allem durch die Bestimmung der chemischen Verschiebungen der Signale beeinflußt und ist nicht besser als 0,2 pH. Außerdem können Resonanzsignale bei der Verwendung endogener Phosphate gänzlich fehlen, weil die Verbindungen sich nur in so geringen Konzentrationen (z.B. im Darm oder in Ehrlich Aszites-Tumorzellen) anreichern, daß eine pH-Wert Bestimmung nicht möglich ist.
Aufgrund dieser Gegebenheiten ist nur eine recht ungenaue pH-Bestimmung in vergleichsweise großen Volumina möglich. Für die Erstellung eines befriedigenden ³¹P-Spektrums werden bei einer Akkumulationszeit von 15 Minuten Signale aus dem Meßvolumen von etwa 100 ccm aufgenommen.

Bei der Nutzung anderer Kerne als ³¹P ist der ¹⁹Fluor-Kern der Kern der Wahl, da er ein leicht meßbares NMR-Signal, das dem des Wasserstoffprotons sehr ähnlich ist (er besitzt ebenso wie ¹H einen Kernspin von 1/2), liefert, d.h. es können die gleichen Empfänger- und Sender-Spulen wie in der ¹H-NMR-Diagnostik verwendet werden, eine hohe Empfindlichkeit (ca. 83% von¹H) besitzt, in 100% Häufigkeit vorliegt und die Signale über einen großen Frequenzbereich verteilt sind. Als weitere Vorteile sind die Abwesenheit von Fluor im Organismus (mit Ausnahme der Zähne), so daß keine Komplikationen mit endogen F-Signalen auftreten können (Fehlen eines ¹⁹F-Background-Signals) - sowie die günstige chemische Zugänglichkeit anzuführen.

Informationen, die über die Verwendung von F-Molekülen in der NMR-Diagnostik erhältlich sind, können durch kein anderes diagnostisches bildgebendes bzw. quasi-bildgebendes Verfahren erzielt werden: das Signal kann sich im Körper -je nach chemischem Zustand - stark verändern, erlaubt somit die Quantifizierung biochemischer Reaktionen und ermöglicht eine direkte Beobachtung physiologischer Vorgänge. Trotz dieser verführerischen Eigenschaften muß auf die problematische Konzentration hingewiesen werden. Ein sinnvolles Experiment bedarf ¹⁹F-Konzentrationen von > 1 mmol F/l, d.h. die zu applizierenden Verbindungen müssen eine hervorragende Verträglichkeit aufweisen und eine gute Löslichkeit in Wasser besitzen, damit durch Verwendung von Lösungen hoher Konzentrationen möglichst kleine Volumina verwendet werden können.

Die Frequenz (chemical shift) einer Fluorlinie wird durch die Lage des F-Atoms im Molekül bestimmt. Prinzipiell gilt dies für alle anderen Atomkerne auch, jedoch ist im Falle des Fluoratoms der chemical shift besonders stark ausgeprägt. Um eine Verschiebung des Fluorsignals zu beobachten bzw. zu quantifizieren bedarf es einer Bezugs(Referenz)-Linie. Diese Frequenzlinie kann das ¹H-Signal, ein externer F-Standard bzw. eine sich nicht verändernde F-Linie, die sich ebenfalls in dem zu vermessenden Areal befindet, sein. Diese Referenzlinie kann sich in einem anderen, ähnlich verteilenden Molekül oder vorzugsweise in dem als Indikator benutzten Molekül selbst befinden. Die günstigste Situation liegt im letztgenannten Fall vor, da hierbei nur eine Substanz appliziert wird und keinerlei Probleme mit Suszeptibilitätseffekten auftreten, so daß eine zweifelsfreie Zuordnung der Signale möglich ist.

Es besteht daher ein Bedarf, geeignete Verbindungen zu finden, die auf eine Veränderung des pH-Werts mit einer veränderten Meßgröße (Resonanzfrequenz) im NMR-Spektrum bei gleichzeitigem Vorhandensein einer Referenzlinie reagieren. Weiterhin müssen diese Verbindungen bzw. die diese Verbindungen enthaltenden diagnostischen Mittel folgende Eigenschaften aufweisen:
a) eine große chemische Verschiebung pro pH-Einheit;
b) geeignete pK-Werte für in-vivo-Messungen;
c) eine für die Diagnostik geeignete Pharmakokinetik;
d) eine für eine Messung genügend hohe Anreicherung in den Zielorganen;
e) gute Verträglichkeit und geringe Toxizität;
f) metabolische Stabilität;
g) hohe chemische Stabilität und Lagerfähigkeit,
h) gute Wasserlöslichkeit.

Die bisher (und nur für in-vitro-Untersuchungen!) beschriebenen Verbindungen (Annals of the New York Academy of Science, S.M. Cohen, Ed. 1987, 508 33) erfüllen diese Voraussetzungen nicht. So ist mit ihnen z.B. eine genauere pH-Bestimmung als mit ³¹P nicht möglich, da die pH-Abhängigkeit der chemischen Verschiebung zu gering ist (≦ 1 ppm/pH) und/oder ihre pH-Werte liegen außerhalb des physiologischen Bereichs und/oder ihre Resonanzfrequenzen sind nicht nur vom pH sondern auch von der Feldstärke abhängig. Auch sind die beschriebenen Verbindungen auf Grund ihrer schlechten Verträglichkeit für einen tierexperimentellen oder gar klinischen Einsatz nicht geeignet.

Der Erfindung liegt somit die Aufgabe zugrunde, Verbindungen und Mittel die die oben genannten Eigenschaften aufweisen zur Verfügung zu stellen, sowie Verfahren zu ihrer Herstellung zu schaffen. Diese Aufgabe wird durch die Erfindung gelöst.

Es wurde gefunden, daß sich fluorsubstituierte Benzolderivate der allgemeinen Formel I 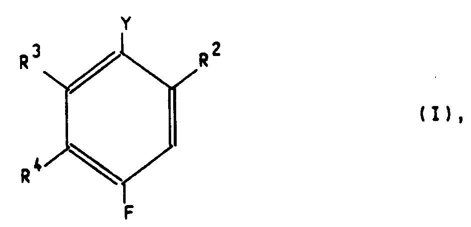
worin
Y eine OH- oder -NHSO₂R¹-Gruppe mit

- R¹: in der Bedeutung einer 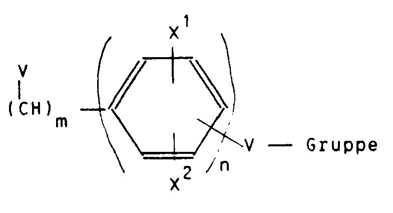 oder einer gerad- oder verzweigtkettigen Alkylengruppe mit 1 bis 10 Kohlenstoffatomen, die gegebenenfalls durch 1 bis 6 Fluoratome substituiert ist und am Ende ein Fluoratom oder den Rest V trägt,
worin V für Wasserstoff
oder die Reste U-OR⁵ oder 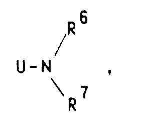 wobei
U einen CO- oder SO₂-Rest,
R⁵ Wasserstoff, einen gesättigten, ungesättigten, gerad- oder verzweigtkettigen oder cyclischen, gegebenenfalls durch 1 bis 6 Hydroxygruppen substituierten Kohlenwasserstoffrest mit bis zu 16 Kohlenstoffatomen,
R⁶ und R⁷ unabhängig voneinander Wasserstoff, eine gerad- oder verzweigtkettige, gegebenenfalls durch 1 bis 6 Hydroxygruppen oder 1 bis 12 Fluoratome substituierte Alkylgruppe mit 1 bis 16 Kohlenstoffatomen oder R⁶ und R⁷ gemeinsam mit dem Stickstoffatom einen gegebenenfalls ein weiteres Heteroatom enthaltenden gesättigten Fünf- oder Sechsring darstellen,
X¹ und X² für Fluor, den Rest V, eine gerad- oder verzweigtkettige Alkylengruppe mit 1 bis 10 Kohlenstoffatomen, die gegebenenfalls durch 1 bis 6 Fluoratome substituiert ist und am Ende ein Fluoratom oder den Rest V trägt, oder den Rest -N(R⁶)-CO-R⁸,
worin R⁸ einen gerad- oder verzweigtkettigen Alkylrest mit 1 bis 16 Kohlenstoffatomen, der gegebenenfalls durch 1 bis 6 Hydroxygruppen und 1 bis 12 Fluoratome substituiert und gegebenenfalls durch 1 bis 3 Sauerstoffatome unterbrochen ist, bedeutet,
m für die Ziffern 0, 1, 2, 3 oder 4,
n für die Ziffern 0 oder 1 stehen, wobei n und m nicht gleichzeitig 0 bedeuten sollen,
- R²: Wasserstoff, Fluor, den Rest N(R⁶)-CO-R⁸, eine 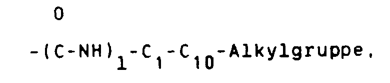 die gerad- oder verzweigtkettig und gegebenenfalls mit 1 bis 6 Fluoratomen substituiert ist, wobei l für die Ziffern 0 oder 1 steht, oder einen Rest 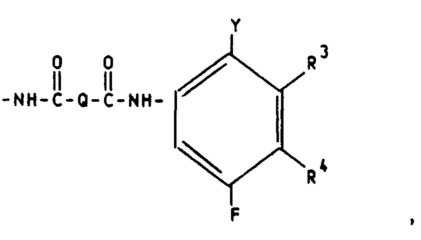 worin Q für eine (CF₂)ₖ- oder C₆F₄-Gruppe mit k in der Bedeutung der Ziffern 1, 2, 3, 4, 5 oder 6 steht,
- R³ und R⁴: unabhängig voneinander Wasserstoff, Fluor, eine gerad- oder verzweigtkettige Alkylgruppe mit 1 bis 10 Kohlenstoffatomen, die gegebenenfalls durch 1 bis 6 Fluoratome substituiert ist, den Rest V oder -N(R⁶)-CO-R⁸ bedeuten wobei die gegebenenfalls im Molekül vorhandenen Reste V gleich oder verschieden sein können und freie Säuregruppen mit organischen oder anorganischen Basen versalzt sind, unter den Maßgaben, daß mindestens 2 Fluoratome im Molekül vorhanden sind, daß für den Fall, daß R⁴ ein Wasserstoffatom und daß einer der beiden Reste R² und R³ für Fluor und der andere für Wasserstoff steht, R¹ nicht eine CH₃-Gruppe sein darf und daß für den Fall, daß R² ein Wasserstoffatom, ein Fluoratom, eine CH₃- oder CF₃-Gruppe, R³ ein Wasserstoffatom und R₄ ein Wasserstoffatom, eine CH₃- oder CF₃-Gruppe ist, das α-Kohlenstoffatom des Restes R¹ nicht fluoriert sein darf, überraschenderweise hervorragend zur Herstellung von NMR-Diagnostika eignen.

Stehen R¹ und X¹, X² bzw. R², R³ und R⁴ für eine Alkylen- bzw. Alkylgruppe, so kann diese gerad- oder verzweigtkettig sein, bis zu 10 Kohlenstoffatome aufweisen und gegebenenfalls durch 1 bis 6 Fluoratome substituiert sein. Bevorzugt sind Alkylen- bzw. Alkylsubstituenten mit 1 bis 6 Kohlenstoffatomen, die gegebenenfalls durch 1 bis 6 Fluoratome substituiert sind. Beispielsmäßig genannt seien Methylen, Difluormethylen, Ethylen, Methylethylen, Ethylethylen, Trimethylen, Tetramethylen, Pentamethylen, Hexamethylen, 1,1-Difluorethylen, 1-Fluorethylen, 1-Methyltetramethylen, 1-Methyltrimethylen, 2-Methyltrimethylen, 2-Methyltetramethylen, Trifluormethylmethylen, 2,2-Difluorethylen bzw. im Falle von R², R³ und R⁴ die entsprechenden um ein Wasserstoff- oder Fluoratom ergänzten Alkylreste wie zum Beispiel Methyl, Fluormethyl, Trifluormethyl, Ethyl, 2-Fluorethyl, 2,2,2-Trifluorethyl, 1-Methylethyl, 1-Fluormethylethyl, 2-Fluor-1-methylethyl usw.

Als Substituenten R⁵, R⁶, R⁷ und R⁸ können gesättigte, ungesättigte, gerad-oder verzweigtkettige oder cyclische, vorzugsweise gesättigte Kohlenwasserstoffreste mit bis zu 16 Kohlenstoffatomen, die gegebenenfalls durch 1 bis 6 Hydroxygruppen sowie im Falle von R⁶, R⁷ und R⁸ durch 1 bis 12 Fluoratome substituiert sind, in Betracht.
Beispielsmäßig genannt seien die Methyl-, Ethyl-, Hydroxymethyl-, 1-Hydroxyethyl-, 2-Hydroxyethyl-, 2-Hydroxy-1-(hydroxymethyl)ethyl-, 1-(Hydroxymethyl)ethyl-, Propyl-, Isopropyl-, Isopropenyl-, 2- und 3-Hydroxypropyl-, 2,3-Dihydroxypropyl-,Butyl, Isobutyl-, Isobutenyl-, 2-, 3-und 4-Hydroxybutyl-, 2-, 3- und 4-Hydroxy-2-methylbutyl-, 2- und 3-Hydroxy-isobutyl-, 2,3,4Trihydroxybutyl-, Cyclohexyl-, Pentyl-, Hexyl-, Bis-und Tris(hydroxymethyl)methyl-, 2,3-Dihydroxy-1-(hydroxymethyl)propyl-, 2,3,4,5,6-Pentahydroxyhexyl, 1,3,4-trihydroxybutyl-2-, sowie 2,2,2-Trifluorethyl-, Trifluormethyl-, 2Fluorethyl-, 1-Fluormethylethylgruppe.
Bevorzugt sind Kohlenwasserstoffreste mit bis zu 6 Kohlenstoffatomen und gegebenenfalls 1 bis 5 Hydroxygruppen sowie 1 bis 8 Fluoratome.
Im Falle von R⁸ kann der Alkylrest durch 1 bis 3 Sauerstoffatome unterbrochen sein. Als Beispiele für in R⁸ enthaltene Kettenglieder seien aufgeführt:
-CH₂-O-CH₂-, -CH₂-CH₂-O-CH₂-CH₂-, -(CH₂-O-CH₂)₂-, -(CH₂-O-CH₂)₃-, -CH₂-CH₂-(OCH₂-CH₂)₃-.

Wenn R⁶ und R⁷ gemeinsam mit dem Stickstoffatom einen gegebenenfalls ein weiteres Heteroatom enthaltenden gesättigten Fünf- oder Sechsring darstellen, steht 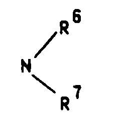
vorzugsweise für Pyrrolidin, Piperidin, Morpholin oder Piperazin.

Die in den Verbindungen der allgemeinen Formel vorhandenen aciden Wasserstoffatome können gegebenenfalls ganz oder teilweise durch Kationen anorganischer und/oder organischer Basen oder Aminosäuren ersetzt sein. Geeignete anorganische Kationen sind beispielsweise das Lithiumion, das Kaliumion, das Calciumion das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Die Herstellung der fluorsubstituierten Benzolderivate der allgemeinen Formel I erfolgt dadurch, daß man Verbindungen der allgemeinen Formel III 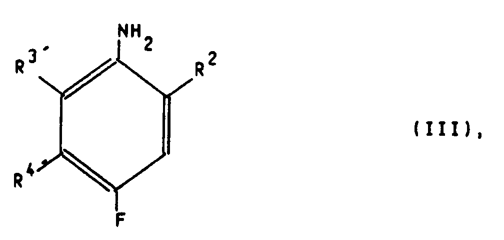
worin
R^{3'} und R^{4'} für R³ und R⁴ oder für in R³ und R⁴ umzuwandelnde Reste stehen, mit Verbindungen der allgemeinen Formel IV oder V

HalSO₂R^{1'} (IV),

(R^{1'}SO₂)₂O (V),

worin
Hal für Chlor, Brom oder Fluor und
R^{1'} für R¹ oder einen in R¹ umzuwandelnden Rest stehen,
umsetzt und gegebenenfalls in den so erhaltenen Reaktionsprodukten die Substituenten R^{1'}, R^{3'} und R^{4'} in die letztlich gewünschten Substituenten R¹, R³ und R⁴ überführt und gewünschtenfalls vorhandene freie Säuregruppen mit organischen oder anorganischen Basen versalzt.

Als Schutzgruppe Z kommen alle Hydroxylschutzgruppen infrage, die bekanntermaßen für einen intermediären Hydroxylgruppenschutz geeignet sind, d.h. die sich leicht einführen und sich später unter Rückbildung der letztlich gewünschten freien Hydroxylgruppe auch wieder leicht abspalten lassen. Bevorzugte Schutzgruppen sind Ethergruppen wie z.B. Benzyl-, Di-und Triphenyl-methyl-Ethergruppen sowie Acetal- und Ketalgruppen mit z.B. Acetaldehyd und Aceton. Geeignet ist auch oft der Schutz durch Veresterung z.B. durch Einführung des Benzoyl- oder Acyl-, insbesondere des Acetylrestes.

Die Abspaltung der Schutzgruppen Z erfolgt - ebenso wie die der in R³ und R⁴ gegebenenfalls vorhandenen Schutzgruppen - in an sich bekannter Weise, z.B. im Falle eines Benzylethers durch hydrogenolytische Spaltung in Gegenwart von z.B. Palladium-Kohle, im Falle eines Esters z.B. durch alkalische Verseifung in wäßrig-alkoholischer Lösung bei Temperaturen von 0 bis 50°C bzw. bei tert-Butylestern mit Hilfe von Trifluoressigsäure, sowie im Falle einer Ketalspaltung mit Hilfe von z.B. Kationenaustauschern oder Trifluoressigsäure.

Die Synthese der gewünschten Sulfonamide der allgemeinen Formel I (Y = NHSO₂R¹) erfolgt in an sich bekannter Weise (Houben-Weyl, "Methoden der organischen Chemie" Band IX, S. 343, 398, 547 und 557, Georg Thieme Verlag Stuttgart 1955) durch Umsetzung von Aminen der allgemeinen Formel III mit Halogensulfonylderivaten (z.B. Chlorsulfonylessigester) der allgemeinen Formel IV oder Sulfonylanhydriden (z.B. Trifluormethansulfonsäureanhydrid) der allgemeinen Formel V in Gegenwart von Säurefängern wie z.B. tertiäres Amin [z.B. Triethylamin, Pyridin, N,N-Dimethylaminopyridin, 1,1-Diazabicyclo-[4.3.0]-nonen-5(DBN), 1,5-Diazabicyclo-[5.4.0]-undecen-5(DBU)], Alkali-, Erdalkalicarbonat- oder -hydrogencarbonat (z.B. Natrium-, Magnesium-, Calcium-, Barium-, Kalium-carbonat und -hydrogencarbonat) in Lösungsmitteln wie z.B. Dioxan, Dichlorethan, Dichlormethan aber auch in Alkohol oder Wasser bei Temperaturen zwischen -20 bis +50°C, vorzugsweise -5 bis 20°C.

Die gegebenenfalls durchzuführenden Folgereaktionen zu den Endsubstanzen mit den gewünschten Substituenten R³ und R⁴, wie z.B. Verseifung von Estern, Überführung einer Carbon- oder Sulfonsäure in ein Amid sowie Abspaltung von Schutzgruppen folgt literaturbekannten Methoden. Sollen Polyhydroxyalkylamide hergestellt werden, so ist es vorteilhaft die dafür benötigten Polyhydroxyalkylamine in geschützter Form in die Reaktion einzusetzen, z.B. als O-Acylderivate oder als Ketale. Dies gilt besonders dann, wenn diese Derivate bequemer und billiger herstellbar sind als die Polyhydroxyalkylamine selbst. Ein typisches Beispiel ist das 2-Amino-1-(2,2-dimethyl-1,3-dioxolan-4-yl)-ethanol, das Acetonid des 1-Amino-2,3,4-trihydroxybutans, hergestellt nach DE-OS 31 50 917.

Die nachträgliche Entfernung der Schutzgruppen ist problemlos und kann z. B. durch Behandlung mit einem sauren Ionenaustauscher in wäßrig-ethanolischer Lösung erfolgen.

Die für die oben unter a) und b) angeführten Reaktionen benötigten Edukte der allgemeinen Formeln II, III, IV und V sind käuflich oder literaturbekannt oder können analog literaturbekannten Methoden hergestellt werden (z.B. J. Org. Chem. 27, 3134 (1962), J. of Organometallic Chem. 190 [1980], 217, J. Org. Chem. 1984,49,2792, JACS 73 [1951], 1325, J. Org. Chem. 1982, 47, 1081, Tet. Lett. 1984, 839)

Nach Herstellung der gewünschten Verbindung der allgemeinen Formel I können im Molekül vorhandene acide Wasserstoffatome durch Kationen anorganischer und/oder organischer Basen oder Aminosäuren substituiert werden.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium und Calcium und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin oder von Amiden ursprünglich neutraler oder saurer Aminosäuren.
Zur Herstellung der neutralen Salze kann man beispielsweise den sauren Benzolderivaten in wäßriger Lösung oder Suspension ein Äquivalent der gewünschten Basen zusetzen. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten.
Enthalten die sauren Verbindungen mehrere freie acide Gruppen, so ist es oft zweckmäßig, neutrale Mischsalze herzustellen, die sowohl anorganische als auch organische Kationen als Gegenionen enthalten.

Die Herstellung der erfindungsgemäßen diagnostischen Mittel erfolgt ebenfalls in an sich bekannter Weise, indem man die erfindungsgemäßen Verbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Komplexbildnern (wie zum Beispiel Diethylentriaminpentaessigsäure) oder, falls erforderlich, Elektrolyte wie zum Beispiel Natriumchlorid oder, falls erforderlich, Antioxidantien wie zum Beispiel Ascorbinsäure.
Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween^{(R)}, Myrj^{(R)} und/oder Aromastoff(en) zur Geschmackskorrektur (zum Beispiel ätherischen Ölen) gemischt.

Die erfindungsgemäßen fluorhaltigen Verbindungen können vorteilhaft in der in-vivo NMR-Diagnostik, d.h. für die NMR-Bildgebung und in der NMR-Spektroskopie als Indikator verschiedener Parameter eingesetzt werden. So können unter anderem mit Hilfe der ortsaufgelösten Spektroskopie und damit gewebsspezifisch pH, pO₂, pCO₂, Temperatur, Redox-Vorgänge, Reaktionskinetik gemessen werden.
Weiterhin wurde festgestellt, daß sich die erfindungsgemäßen Verbindungen überraschenderweise durch eine sehr gute Verträglichkeit auszeichnen. So wurde zum Beispiel für 5-[N-(4-Fluor-2-trifluormethylphenyl)sulfamoyl]-isophthalsäure (Beispiel 13) an der Maus eine intravenöse akute Verträglichkeit (LD₅₀) von 4 mmol/kg Körpergewicht ermittelt. Dieser Befund ist überraschend, da die meisten vergleichbaren fluorierten Verbindungen eine äußerst schlechte (<0.1 mmol/kg Körpergewicht) Verträglichkeit aufweisen.

Die erfindungsgemäßen pharmazeutischen Mittel werden vorzugsweise in einer Konzentration von 1 µMol-1 Mol/l hergestellt. Sie werden in der Regel in Mengen von 0,005-20 mMol/kg Körpergewicht, vorzugsweise 0,05 bis 5 mMol/kg Körpergewicht, dosiert. Sie sind zur enteralen und parenteralen Applikation bestimmt.

Die erfindungsgemäßen Mittel erfüllen die vielfältigen Voraussetzungen für die Eignung als Diagnostika für die NMR-Tomographie und -Spektroskopie. Ferner zeigen sie die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit der erfindungsgemäßen Mittel erlaubt es, hochkonzentrierte Lösungen herzustellen, damit die Volumenbelastung des Kreislaufs in vertretbaren Grenzen zu halten und die Verdünnung durch Körperflüssigkeit auszugleichen, das heißt NMR-Diagnostika müssen 100-1000-fach besser wasserlöslich sein als die in der in-vitro NMR-Spektroskopie verwendeten Mittel.

Die gute Verträglichkeit der erfindungsgemäßen Verbindungen erlaubt die Prüfung und NMR-spektroskopische Vermessung des pH-Wertes im lebenden Organismus. Hierbei ermöglicht eine Gabe von 10 µmol/kg bis 10 mmol/kg Körpergewicht eine problemlose Bestimmung der Veränderung der chemischen Verschiebung des ¹⁹F-Signals im Verhältnis zum Referenzsignal (z.B. einer intramolekularen CF₃-Gruppe) und damit des pH-Wertes. Die applizierte Lösung verteilt sich rasch im Organismus und ist somit in der Lage, Bereiche unterschiedlichen pH-Wertes nachzuweisen. Durch eine entsprechende Dosierung kann darüberhinaus eine Veränderung des pH-Wertes und damit gegebenenfalls ein therapeutischer Effekt bewirkt werden.

Um kleine Änderungen des pH-Wertes aufzeigen zu können, sind die Verbindungen von Vorteil, deren pK-Wert in der Nähe des biologischen bzw. pathologischen pH-Wertes des interessierenden Gewebes ist. In der Regel sind diejenigen Verbindungen von besonderem Interesse, deren pK-Wert zwischen 2 und 9, vorzugsweise zwischen 6 und 8, liegt. Verbindungen, die den pH-Wert des Magen-Darm-Traktes aufzeigen, haben vorteilhafterweise einen pK zwischen 2 und 8. Da die größte Genauigkeit der pH-Bestimmung im Bereich der größten Veränderung der chemischen Verschiebung pro Einheit, also beim pK-Wert der jeweiligen Verbindung, vorliegt ist eine sehr gute Analyse biologischer Vorgänge möglich. So liegt der pH des Blutes bei etwa 7.2-7.4; pathologische Bereiche können einen veränderten pH-Wert haben, der z.B. bis auf 4.0 oder niedriger sinken kann.

Für die Darstellung der Nierenfunktion bzw. Analyse des Primär- und Sekundärharnes sind Verbindungen mit einem pK-Wert zwischen 5 und 7 von Vorteil, da der pH-Wert des Urins in der Regel unter dem des Blutes liegt. Für die Bestimmung des intragastralen pH-Wertes sind die Verbindungen von Vorteil, die eine Veränderung der chemischen Verschiebung zwischen pH 2 und 6 am deutlichsten zeigen, da der pH-Wert des Magensaftes zwischen fast 1 und 7 stark schwanken kann.

Durch die Verwendung der sehr gut verträglichen neuartigen fluorierten Meßsonden ist es somit möglich geworden, in kleineren Volumina (z.B. 10 ccm) ortsaufgelöste Spektroskopie durchzuführen und physiologisch wichtige Parameter wie z.B. den pH-Wert präzise in kurzer Meßzeit ohne Störung bzw. Überlagerung durch andere Moleküle zu bestimmen.

Für ein in-vivo Imaging (NMR-Bildgebung) sind die genannten Verbindungen ebenfalls geeignet. Hierbei werden nicht nur die Informationen der veränderten chemischen Verschiebung bildlich dargestellt, sondern es werden die lokalen Konzentrationen der fluorierten Verbindungen durch die in der MRT üblichen Aufnahmesequenzen in einem Imaging wiedergegeben. Der Vorteil des ¹⁹F-Imaging gegenüber der ¹H-Tomographie beruht darin, daß die Verteilung des Pharmakons direkt ohne Überlagerung durch störende Strukturen dargestellt werden kann.

So gelingt z.B. eine hervorragende Kontrastierung des renalen Ausscheidungssystems (Niere, Urether, Blase) nach intravenöser Gabe der erfindungsgemäßen Verbindungen, welche in einer Dosis von 5 µmol/kg bis 20 mmol/kg, vorzugsweise von 0,1 mmol/kg bis 5 mmol/kg, appliziert werden. Hierbei wurde überraschenderweise auch gefunden, daß die zusätzliche Injektion einer paramagnetischen Verbindung (z.B. GdDTPA/Dimeglumin) in einer Dosis von 1 µmol/kg bis 2 mmol/kg, vorzugsweise von 50 µmol/kg bis 500 µmol/kg, zu einer deutlichen Verbesserung der Bildqualität führt.

Gekoppelt an zum Beispiel geeignete monoklonale Antikörper können die erfindungsgemäßen Verbindungen auch Verwendung als organ- und tumorspezifische Therapeutika und Diagnostika finden.

Die erfindungsgemäßen Verbindungen der allgemeinen Formel I mit Y in der Bedeutung von NHSO₂R¹ sind auch gegen alle bakteriellen Infektionen, die chemotherapeutisch mit Sulfonamiden zu behandeln sind, einzusetzen.

Die nachfolgenden Beispiele dienen zur näheren Erläuterung des Erfindungsgegenstandes.

### Beispiel 1

### 2-[N-(2,4-Difluorphenyl)sulfamoyl]-essigsäuremethylester

3.87g (30 mmol) 2,4-Difluoranilin werden unter Feuchtigkeitsausschluß in 80ml Dichlorethan gelöst, 2.5ml (30 mmol) Pyridin zugefügt und die Lösung auf 0°C gekühlt. Zur gekühlten Lösung werden 5.18g (30 mmol) Chlorsulfonylessigester, gelöst in 20ml Dichlorethan, zugetropft, 1 Stunde bei 0°C und 12 Stunden bei Raumtemperatur gerührt. Die Reaktionslösung wird dann mit 2N Salzsäure ausgeschüttelt, die Phasen getrennt, die organische Phase über Natriumsulfat getrocknet, im Vakuum eingedampft und der ölige Rückstand an 200g Kieselgel 60 (Merck) mit Essigester/Hexan chromatografiert. Man erhält 4.5g (16.97 mmol) = 56.55% d. Th. der Titelverbindung als Öl.

| Analyse : C₉H₉F₂NO₄S MG 265.24 | | | | | | | | | | | | |
|---|---|---|---|---|---|---|---|---|---|---|---|---|
| berechnet | C | 40.75 | H | 3.42 | F | 14.32 | N | 5.28 | O | 24.19 | S | 12.09% |
| gefunden | | 41.03 | | 3.61 | | 14.11 | | 5.13 | | | | 11.91% |

### Beispiel 2

### 2-[N-(2,4-Difluorphenyl)sulfamoyl]-essigsäure

2.68g (10.1 mmol) 2-[N-(2,4-Difluorphenyl)sulfamoyl]-essigsäuremethylester werden in 30ml Dioxan gelöst, 15ml 2N Natronlauge zugefügt und 30 Minuten bei Raumtemperatur gerührt. Die Reaktionslösung wird dann zur Trockne eingedampft, der Rückstand in etwas Wasser gelöst, die Lösung erneut eingedampft, der Rückstand in 30ml Wasser gelöst, mit 2N Salzsäure angesäuert und das Produkt mit Essigester extrahiert. Die organische Phase wird über Natriumsulfat getrocknet, filtriert und im Vakuum zur Trockne eingedampft. Das Rohprodukt wird aus Ether/Hexan 1:1 umkristallisiert. Man erhält 1.55g (6.17mmol) = 61.1% d. Th. Kristallisat. Fp 110-111.5°C.

### Beispiel 3

### 2-[N-(2,4-Difluorphenyl)sulfamoyl]-essigsäure-(2,3,4-trihydroxy-butylamid)

a) 2-[N-(2,4-Difluorphenyl)sulfamoyl)]-essigsäure-[2-hydroxy-2-(2,2-dimethyl-1,3-dioxolan-4-yl)-ethylamid]
1.62g ( 6.5 mmol) 2-[N-(2,4-Difluorphenyl)sulfamoyl)]-essigsäure werden unter Feuchtigkeitsausschluß und Argonatmospäre in 20ml Dimethylformamid gelöst, 1.01g (6.5 mmol) Hydroxybenztriazol und 1.05g (6.5 mmol) 2-Hydroxy-2-(2,2-dimethyl-1,3-dioxolan-4-yl)-ethylamin hinzugefügt und die Lösung auf -10°C gekühlt. Zur gekühlten Lösung werden 1.34g (6,5 mmol) Dicyclohexylcarbodiimid zugefügt, 1 Stunde bei - 10 °C nachgerührt, die Kühlung dann entfernt, so daß sich das Reaktionsgemisch langsam auf Raumtemperatur erwärmt und weitere 5 Stunden nachgerührt. Die Suspension wird dann filtriert, das Filtrat im Vakkuum zur Trockne eingedampft und der Rückstand in Essigester gelöst. Diese Lösung wird mit Wasser ausgeschüttelt, über Magnesiumsulfat getrocknet, filtriert und im Vakuum zur Trockne eingedampft. Der Rückstand wird in 50ml Dichlormethan gelöst, die Lösung filtriert, das Filtrat zur Trockne eingedampft und der Rückstand aus Diethylether kristallisiert. Man erhält 1.56g (3.96 mmol) = 60.85% d. Th. Kristallisat. Fp. 165 - 166.5°C.
b) 2-[N-(2,4-Difluorphenyl)sulfamoyl]-essigsäure-(2,3,4-trihydroxy-butylamid)
2.96g (7.5 mmol) 2-[N-(2,4-Diluorphenylsulfamoyl)-essigsäure-[2-hydroxy-2-(2,2-dimethyl-1,3-dioxolan-4-yl)-ethyl-amid] werden in 10ml Methanol gelöst, ca. 100mg Kationenaustauscher Amberlyst 15 hinzugefügt und 1 Stunde auf Siedetemperatur erwärmt. Der lonenaustauscher wird dann abfiltriert und das Filtrat im Vakuum zur Trockne eingedampft. Man erhält 2.21g (6.26 mmol) = 83.5% d. Th. des Produktes als amorphen Feststoff.

| Analyse : C₁₂H₁₅F₂N₂O₆S MG 353.32 | | | | | | | | | | | | |
|---|---|---|---|---|---|---|---|---|---|---|---|---|
| berechnet | C | 40.79 | H | 4.27 | F | 10.75 | N | 7.92 | O | 27.16 | S | 9.07% |
| gefunden | | 41.04 | | 4.52 | | 10.53 | | 8.11 | | | | 8.93% |

### Beispiel 4

### 2-[N-(2,4,6-Trifluorphenyl)sulfamoyl]-essigsäuremethylester

0.76g (5 mmol) 2,4,6-Trifluoranilin werden unter Feuchtigkeitsausschluß in 40ml Dichlorethan gelöst, 0.4ml (5 mmol) Pyridin zugefügt, bei 0°C 0.86g (5 mmol) Chlorsulfonylessigsäuremethylester, gelöst in 10ml Dichlorethan, eingetropft und anschließend 1 Stunde bei 0°C und 12 Stunden bei Raumtemperatur gerührt. Die Reaktionslösung wird dann zweimal mit je 10ml 2N Salzsäure ausgeschüttelt, die Phasen getrennt, die organische Phase über Magnesiumsulfat getrocknet, filtriert und zur Trockne engedampft. Der Rückstand wird aus Diethylether/Pentan 1:1 kristallisiert. Man erhält 790mg (2.79 mmol) = 55.79% d. Th. Kristallisat. Fp. 99-100°C.

### Beispiel 5

### 2-[N-(2,4,6-Trifluorphenyl)sulfamoyl]-essigsäure

1.37g (4.8 mmol) 2-[N-(2,4,6-Trifluorphenyl)sulfamoyl]-essigsäuremethylester werden in 15ml Dioxan gelöst, 7.5ml 2N Natronlauge hinzugefügt und eine Stunde bei Raumtempratur gerührt. Die Lösung wird dann zur Trockne eingedampft, der Rückstand in Wasser gelöst, die Lösung mit 2N Salzsäure angesäuert und zweimal mit Essigester extrahiert. Der Essigesterextrakt wird über Magnesiumsulfat getrocknet, filtriert und zur Trockne eingedampft. Der Rückstand wird aus Diethylether kristallisiert. Man erhält 1.12g (4.16 mmol) = 86.68% d. Th.. Fp. 126-127°C.

### Beispiel 6

### 2-[N-(2,4,6-Trifluorphenyl)sulfamoyl]-essigsäure-[2,3,4-trihydroxy-butylamid]

a) 2-[N-(2,4,6-Trifluorphenyl)sulfamoyl]-essigsäure-[2-hydroxy-2-(2,2-dimethyl-1,3-dioxolan-4-yl)-ethylamid]
1.38g (3.16 mmol) 2-[N-(2,4,6-Trifluorphenyl)sulfamoyl]-essigsäure werden in 20ml DMF unter Feuchtigkeitsausschluß und Argonatmosphäre gelöst, 509mg (3.16 mmol) 2-Hydroxy-2-(2,2-dimethyl-1,3-dioxolan-4-yl)-ethylamin und 484mg (3.16 mmol) Hydroxybenztriazol hinzugefügt und die Lösung auf - 10°C gekühlt. Zu der gekühlten Lösung werden 652mg (3.16 mmol) Dicyclohexylcarbodiimid zugegeben, eine Stunde bei - 10°C und 12 Stunden bei Raumtemperatur nachgerührt. Die trübe Reaktionslösung wird dann filtriert und das klare Filtrat im Vakuum zur Trockne eingedampft. Der Rückstand wird in 100ml Essigester gelöst und die Lösung fünfmal mit gesättigter Natriumhydrogencarbonatlösung und zweimal mit Wasser ausgeschüttelt. Die Essigester-Lösung wird über Natriumsulfat getrocknet, filtriert und zur Trockne eingedampft. Der Rückstand wird an Kieselgel 60 (Merck) mit Essigester/Methylenchlorid 8:2 chromatografiert. Die entsprechenden Fraktionen enthalten zusammen 0.62g ( 1.42 mmol) = 45% d. Th. Produkt als amorphen Feststoff.

| Analyse : C₁₅H₁₉F₃N₂O₆S MG 412.38 | | | | | | | | | | | | |
|---|---|---|---|---|---|---|---|---|---|---|---|---|
| berechnet | C | 43.68 | H | 4.64 | F | 13.82 | N | 6.79 | O | 23.27 | S | 7.77% |
| gefunden | | 43.42 | | 4.87 | | 13.66 | | 7.02 | | | | 7.53% |

b) 2-[N-(2,4,6-Trifluorphenyl)sulfamoyl]-essigsäure-[2,3,4-trihydroxy-butylamid]
2.22g (6 mmol) 2-[N-(2,4,6-Triluor-phenyl)sulfamoyl]-essigsäure-[2-hydroxy-2-(2,2-dimethyl-1,3-dioxolan-4-yl)-ethyl-amid] werden in einem Gemisch aus 10ml Methanol und 5ml Wasser gelöst, ca. 100mg Kationenaustauscher Amberlyst 15 hinzugefügt und 1 Stunde auf Siedetemperatur erwärmt. Der Ionenaustauscher wird dann abfiltriert und das Filtrat im Vakuum zur Trockne eingedampft. Man erhält 1.94g (5.24 mmol) = 87.3% d. Th. des Produktes als amorphen Feststoff.

| Analyse : C₁₂H₁₄F₃N₂O₆S MG 371.31 | | | | | | | | | | | | |
|---|---|---|---|---|---|---|---|---|---|---|---|---|
| berechnet | C | 38.81 | H | 3.8 | F | 15.35 | N | 7.54 | O | 25.85 | S | 8.63% |
| gefunden | | 39.04 | | 3.96 | | 15.13 | | 7.73 | | | | 8.47% |

### Beispiel 7

### 2-[N-(4-Fluor-2-trifluormethylphenyl)sulfamoyl]-essigsäuremethylester

5g (27.64 mmol) 4-Fluor-2-(trifluormethyl)-anilin werden in 25ml trockenem Pyridin gelöst und die Lösung auf 0°C gekühlt. Zur gekühlten Lösung tropft man unter Beibehaltung der Temperatur im Verlaufe von ca. 10 Minuten eine Lösung von 4.82g (27.4 mmol) Chlorsulfonylessigsäuremethylester in 20ml Dichlormethan. Anschließend wird 6 Stunden bei Raumtemperatur gerührt. Die Reaktionslösung wird dann mit 100ml Dichlormethan verdünnt, das Pyridin mit 2N Salzsäure ausgeschüttelt, die organische Phase über Magnesiumsulfat getrocknet, filtriert und zur Trockne eingedampft. Der Rückstand wird aus Diethylether/ Hexan 1 : 1 kristallisiert. Man erhält 7.35g (23.32 mmol) = 84.35% d. Th. Kristallisat. Fp. 84-86 °C.

### Beispiel 8

### 2-[N-(4-Fluor-2-trifluormethylphenyl)sulfamoyl]-essigsäure

2.2g (6.98 mmol) 2-[N-(4-Fluor-2-trifluormethylphenyl)sulfamoyl]-essigsäuremethylester werden in 20ml Dioxan gelöst, und die Lösung mit 10ml (20 mmol) 2N Natronlauge versetzt. Es tritt eine leichte Erwärmung ein und die Verseifung ist nach 10 Minuten vollständig. Die Lösung wird mit 2N Salzsäure unter pH-Kontrolle neutralisiert und im Vakuum schonend zur Trockne eingedampft. Der Rückstand wird mit 30ml Essigester extrahiert, die Lösung einmal mit 10ml Wasser ausgeschüttelt, die organische Phase über Magnesiumsulfat getrocknet, filtriert und zur Trockne eingedampft. Man erhält 1.96g (6.51 mmol) = 93.2% d. Th. Fp. 120 - 121 °C (Ether/Hexan).

| Analyse : C₉H₇F₄NO₄S MG 301.24 | | | | | | | | | | | | |
|---|---|---|---|---|---|---|---|---|---|---|---|---|
| berechnet | C | 35.89 | H | 2.34 | F | 25.23 | N | 4.65 | O | 21.25 | S | 10.64 % |
| gefunden | | 36.05 | | 2.36 | | 25.45 | | 4.66 | | | | 10.79 % |

### Beispiel 9

### 2-[N-(4-Fluor-2-(trifluormethylphenyl)sulfamoyl]-essigsäure-(2,3,4-trihydroxy-butyl-amid)

a) 2-[N-(4-Fluor-2-(trifluormethylphenyl)sulfamoyl]-essigsäure-[2-hydroxy-2-(2,2-dimethyl-1,3-dioxolan-4-yl)-ethyl-amid]
3,01 g (10 mmol) 2-[N-(4-Fluor-2-(trifluormethylphenyl)sulfamoyl]-essigsäure werden in 100 ml Dimethylformamid gelöst, 1,54 g (10 mmol) Hydroxybenztriazol und 1,61 g (10 mmol) 2-Hydroxy-2-(2,2-dimethyl-1,3-dioxolan-4-yl)-ethylamin hinzugefügt und das Gemisch auf -10 °C gekühlt. Dann gibt man 2,07 g (10 mmol) Dicyclohexylcarbodiimid hinzu, rührt 1 Stunde bei -10 °C und 12 Stunden bei Raumtemperatur. Nach Zugabe von 10 mmol Triethylamin wird weitere 5 Stunden gerührt. Die Lösung wird im Vakuum eingedampft, der Rückstand in Methylenchlorid gelöst, mit Wasser ausgeschüttelt, die organische Phase über Magnesiumsulfat getrocknet, eingedampft und der Rückstand an Kieselgel chromatographiert. Man erhält 2,76 g = 62,11 % d. Th. amorphen Feststoff, der aus Essigester kristallisiert wird. Dabei erhält man 1,05 g = 23,63 % d. Th. Kristallisat.
Fp. 125 - 126 °C.
b) 2-[N-(4-Fluor-2-(trifluormethylphenyl)sulfamoyl]-essigsäure-(2,3,4-trihydroxy-butyl-amid)
1,5 g (4,05 mmol) der nach 9a) erhaltenen Substanz werden in 68 ml eines Gemisches von Ethanol und Wasser 1:1 gelöst. Man fügt 0,1 ml konz. Salzsäure hinzu und rührt die Lösung 10 Minuten bei Raumtemperatur und 1 Stunde bei 60 °C. Die Ketalspaltung ist dann vollständig. Es wird durch Zugabe von Anionenaustauscher das Chlorid entfernt und im Vakuum eingedampft. Man erhält 570 mg = 41,45 % d. Th. als amorphen Feststoff.

| Analyse: C₁₃H₁₆F₄N₂O₆S MG 404,45 | | | | | | | | | | | | |
|---|---|---|---|---|---|---|---|---|---|---|---|---|
| berechnet | C | 38,61 | H | 3,99 | F | 18,79 | N | 6,93 | O | 23,8 | S | 7,93 % |
| gefunden | | 38,44 | | 4,63 | | 18,61 | | 6,94 | | | | 8,13 % |

### Beispiel 10

### 4-[N-(4-Fluor-2-trifluormethylphenyl)sulfamoyl]-benzoesäure

2.21g (10 mmol) 4-Chlorsulfonyl-benzoesäure werden in 30ml Dichlormethan unter Feuchtigkeitsausschluß suspendiert, die Suspension auf -5°C gekühlt und bei dieser Temperatur eine Lösung von 1.79g (10 mmol) 4-Fluor-2-(trifluormethyl)-anilin und 1ml Pyridin in 20ml Dichlormethan zugetropft. Nach beendetem Eintropfen läßt man auf Raumtemperatur kommen und rührt 12 Stunden nach. Der entstandene Niederschlag wird abgesaugt und mit Ether extrahiert. Dieser Etherextrakt und das Filtrat werden zusammen eingedampft und der Rückstand in Dichlorethan gelöst. Die Lösung wird dreimal mit je 10ml gesättigter Natriumhydrogencarbonat-Lösung extrahiert. Die vereinigten wässrigen Extrakte werden mit 2N Salzsäure angesäuert und das dabei ausfallende Produkt abfiltriert. Der Filterrückstand wird im Vakuum bei 50°C getrocknet. Man erhält 3.3g (9.08 mmol) = 90.84% d. Th.

| Analyse : C₁₄H₉F₄NO₄S MG 363.28 | | | | | | | | | | | | |
|---|---|---|---|---|---|---|---|---|---|---|---|---|
| berechnet | C | 46.29 | H | 2.5 | F | 20.92 | N | 3.86 | O | 17.62 | S | 8.83 % |
| gefunden | | 46.10 | | 2.67 | | 21.16 | | 3.84 | | | | 8.96 % |

### Beispiel 11

### 4-[N-(4-Fluor-2-trifluormethylphenyl)sulfamoyl]-benzoesäure-(2,3,4-trihydroxy-butyl)-amid

a) 4-[N-(4-Fluor-2-trifluormethylphenyl)sulfamoyl]-benzoesäure-[2-hydroxy-2-(2,2-dimethyl-1,3-dioxolan-4-yl)-ethyl-amid]
3.63g (10 mmol) 4-[N-(4-Fluor-2- trifluormethylphenyl)sulfamoyl)-benzoesäure werden in 36ml Essigester suspendiert, 2.38g (20 mmol) Thionylchlorid hinzugefügt und die Suspension auf Siedetemperatur erhitzt. Nach 15 Minuten liegt eine klare Lösung vor, nach 1 Stunde ist die Bildung des Säurechlorids vollständig. Die Lösung wird unter vermindertem Druck zur Trockne eingedampft, der Rückstand zweimal im Vakuum mit je 50ml Dichlormethan eingedampft und dann in 36ml Dioxan gelöst. Zu dieser Lösung fügt man 1.21g (12 mmol) Triethylamin und 1.93g (12 mmol) 2-Hydroxy-2-(2,2-dimethyl-1,3-dioxolan-4-yl)-ethylamin hinzu und rührt 5 Stunden bei Raumtemperatur. Die Reaktionslösung wird dann im Vakuum zur Trockne eingedampft, der Rückstand mit Essigester extrahiert, der Essigesterextrakt im Vakuum eingedampft und der Rückstand an Kieselgel 60 (Merck) mit Essigester/Hexan chromatografiert. Man erhält 3.19g (6.3 mmol) = 63% d. Th. des Produktes als amorphen Feststoff.

| Analyse : C₂₁H₂₂F₄N₂O₆S MG 506.47 | | | | | | | | | | | | |
|---|---|---|---|---|---|---|---|---|---|---|---|---|
| berechnet | C | 49.8 | H | 4.37 | F | 15.0 | N | 5.53 | O | 18.95 | S | 6.33% |
| gefunden | | 50.04 | | 4.43 | | 14.83 | | 5.32 | | | | 6.18% |

b) 4-[N-(4-Fluor-2-trifluormethylphenyl)sulfamoyl]-benzoesäure-(2,3,4-trihydroxy-butyl)-amid
2.53g (5 mmol) 4-[N-(4-Fluor-2-trifluormethylphenyl)sulfamoyl] -benzoesäure-[2-hydroxy-2-(2,2-dimethyl-1,3-dioxolan-4-yl)-ethyl-amid] werden in 20ml Methanol/Wasser 1:1 gelöst, ca. 100mg Kationenaustauscher Amberlyst 15 hinzugefügt und 1 Stunde auf Siedetemperatur erwärmt. Der Ionenaustauscher wird dann abfiltriert und das Filtrat im Vakuum zur Trockne eingedampft. Man erhält 1.74g (3.75 mmol) = 75% d. Th. des Produktes als amorphen Feststoff.

| Analyse : C₁₈H₁₇F₄N₂O₆S MG 465.4 | | | | | | | | | | | | |
|---|---|---|---|---|---|---|---|---|---|---|---|---|
| berechnet | C | 46.45 | H | 3.68 | F | 16.32 | N | 6.01 | O | 20.62 | S | 6.89% |
| gefunden | | 46.33 | | 3.87 | | 16.16 | | 5.89 | | | | 7.03% |

### Beispiel 12

### 5-[N-(4-Fluor-2-trifluormethylphenyl)sulfamoyl]-isophthalsäure-dimethylester

5g (27.64 mmol) 4-Fluor-2-(trifluormethyl)-anilin werden unter Feuchtigkeitsauschluß in 70 ml Dichlorethan gelöst, 4.38g (55.28 mmol) Pyridin hinzugefügt und die Lösung auf 0°C gekühlt. Unter Beibehaltung dieser Temperatur wird eine Lösung von 8.78g (30 mmol) 4-Chlorsulfonyl-isophthalsäuredimethylester in 50ml Dichlorethan zugetropft, 30 Minuten bei 0°C und 5 Stunden bei Raumtemperatur nachgerührt. Die Suspension wird dreimal mit 2N Salzsäure und zweimal mit Wasser ausgeschüttelt, die organische Phase über Natriumsulfat getrocknet, filtriert, eingedampft und der Rückstand an Kieselgel 60 ( Merck ) mit Essigester/Hexan 1 : 1 chromatografiert. Die eingedampften Fraktionen der entsprechenden Polarität ergeben 8.94g (20.54 mmol) = 74.3% d. Th. kristallines Produkt. Fp. 142-150°C.

### Beispiel 13

### 5-[N-(4-Fluor-2-trifluormethylphenyl)sulfamoyl]-isophthalsäure

7.03g (16.15 mmol) 5-[N-(4-Fluor-2-trifluormethylphenyl)sulfamoyl]- isophthalsäure-dimethylester werden in 50ml Dioxan gelöst, 28.27ml (56.53 mmol) 2N NaOH hinzugefügt und 3 Stunden bei 50°C gerührt. Die Lösung wird dann bei vermindertem Druck auf ca. 20ml eingeengt, mit 2N Salzsäure angesäuert und der feste Niederschlag, der dabei entsteht abgesaugt. Der Filterrückstand wird in Essigester gelöst, die Lösung zweimal mit Wasser ausgeschüttelt, über Natriumsulfat getrocknet, filtriert und zur Trockne eingedampft. Der Rückstand wird aus Essigester/Hexan 1 : 1 kristallisiert. Man erhält 5.78g (14.19 mmol) = 87.87% d. Th. als Kristallisat. Fp. 250-254°C.

### Beispiel 14

### 5-[N-(4-Fluor-2-trifluormethylphenyl)sulfamoyl]-isophthalsäure-bis(2,3,4-trihydroxy-butyl)-amid

a) 5-[N-(4-Fluor-2-trifluormethylphenyl)sulfamoyl]-isophthalsäure-bis[2-hydroxy-2-(2,2-dimethyl-1,3-dioxolan-4-yl)-ethylamid]
4.89g (12 mmol) 5-[N-(4-Fluor-2-trifluormethylphenyl)sulfamoyl]- isophthalsäure werden unter Argonatmosphäre in 50ml trockenem Dimethylformamid gelöst, 2.76g (18 mmol) Hydroxybenztriazol und 2.9g (18 mmol) 2-Hydroxy-2-(2,2-dimethyl-1,3-dioxolan-4-yl)-ethylamin hinzugefügt und die Lösung auf -10°C gekühlt. Bei dieser Temperatur werden der Lösung 3.71g (18 mmol) Dicyclohexylcarbodiimid zugesetzt, 1 Stunde bei -10°C und 10 Stunden bei Raumtemperatur gerührt. Die trübe Reaktionslösung wird filtriert und das Filtrat im Vakuum eingedampft. Der Rückstand wird in 100ml Essigester gelöst, die Lösung fünfmal mit gesättigter Natriumbicarbonatlösung und zweimal mit Wasser ausgeschüttelt, die organische Phase über Natriumsulfat getrocknet, filtriert und eingedampft. Der Rückstand wird aus Essigester/Hexan 1 : 1 kristallisiert. Man erhält 3.85g (5.55 mmol) = 46.25% d. Th. Kristallisat. Fp. 75-80°C.

| Analyse : C₂₉H₃₅F₄N₃O₁₀S MG 693.67 | | | | | | | | | | | | |
|---|---|---|---|---|---|---|---|---|---|---|---|---|
| berechnet | C | 50.21 | H | 5.09 | F | 10.95 | N | 6.06 | O | 23.06 | S | 4.62% |
| gefunden | | 50.35 | | 5.16 | | 10.67 | | 6.09 | | | | 4.41% |

b) 5-[N-(4-Fluor-2-trifluormethylphenyl)sulfamoyl]-isophthalsäure-bis(2,3,4-trihydroxy-butyl-amid)
3.79g (5.46 mmol) 5-[N-(4-Fluor-2-trifluormethyl-phenyl)sulfamoyl]- isophthalsäure-bis[2-hydroxy-2-(2,2-dimethyl-1,3-dioxolan-4-yl)-ethylamid] werden in einem Gemisch aus 40ml Ethanol und 10ml Wasser gelöst und die Lösung mit verdünnter Salzsäure auf pH 1 angesäuert. Bei diesem pH wird 4 Stunden bei 60°C gerührt. Die Lösung wird dann mit Anionenaustauscher Amberlite IRA 67 auf pH 6.1 gestellt, filtriert und im Vakuum zur Trockne eingedampft. Der feste amorphe Rückstand wird im Vakuum bei 50°C getrocknet. Man erhält 2.55g (4.16 mmol) = 76.12 % d. Th. als amorphen Feststoff.

| Analyse : C₂₃H₂₇F₄N₃O₁₀S MG 613.54 | | | | | | | | | | | | |
|---|---|---|---|---|---|---|---|---|---|---|---|---|
| berechnet | C | 45.03 | H | 4.44 | F | 12.39 | N | 6.85 | O | 26.08 | S | 5.23% |
| gefunden | | 45.28 | | 4.67 | | 12.12 | | 6.68 | | | | 4.97% |

### Beispiel 15

### 3-[N-(4-Fluor-2-(trifluormethylphenyl)sulfamoyl]-benzoesäure

2.21g (10 mmol) 3-Chlorsulfonyl-benzoesäure werden in 30ml Dichlormethan unter Feuchtigkeitsausschluß suspendiert, die Suspension auf -5°C gekühlt und bei dieser Temperatur eine Lösung von 1.79g (10 mmol) 4-Fluor-2-(trifluormethyl)-anilin und 1ml Pyridin in 20ml Dichlormethan zugetropft. Nach beendetem Eintropfen läßt man auf Raumtemperatur kommen und rührt 12 Stunden nach. Der entstandene Niederschlag wird abgesaugt und mit Ether extrahiert. Dieser Etherextrakt und das Filtrat werden zusammen eingedampft und der Rückstand in Dichlorethan gelöst. Die Lösung wird dreimal mit je 10ml gesättigter Natriumhydrogencarbonat-Lösung extrahiert. Die vereinigten wässrigen Extrakte werden mit 2N Salzsäure angesäuert und das dabei ausfallende Produkt abfiltriert. Der Filterrückstand wird im Vakuum bei 50°C getrocknet. Man erhält 3.04g ( 8.37 mmol) = 83.7% d. Th.

| Analyse : C₁₄H₉F₄NO₄S MG 363.28 | | | | | | | | | | | | |
|---|---|---|---|---|---|---|---|---|---|---|---|---|
| berechnet | C | 46.22 | H | 2.5 | F | 20.92 | N | 3.86 | O | 17.62 | S | 8.96% |
| gefunden | | 45.97 | | 2.68 | | 21.14 | | 4.02 | | | | 8.67% |

### Beispiel 16

### 3-[N-(4-Fluor-2-trifluormethylphenyl)sulfamoyl]-benzoesäure-[2,3,4-trihydroxy-butyl)-amid)

a) 3-[N-(4-Fluor-2-trifluormethylphenyl)sulfamoyl]-benzoesäure-[2-hydroxy-2-(2,2-dimethyl-1,3-dioxolan-4-yl)-ethyl-amid]
3.63g (10 mmol) 3-[N-(4-Fluor-2-trifluormethylphenyl)sulfamoyl]-benzoesäure werden in 36ml Essigester suspendiert, 2.38g (20 mmol) Thionylchlorid hinzugefügt und die Suspension auf Siedetemperatur erhitzt. Nach 15 Minuten liegt eine klare Lösung vor, nach 1 Stunde ist die Bildung des Säurechlorids vollständig. Die Lösung wird unter vermindertem Druck zur Trockne eingedampft, der Rückstand zweimal im Vakuum mit je 50ml Dichlormethan eingedampft und dann in 36ml Dioxan gelöst. Zu dieser Lösung fügt man 1.21g (12 mmol) Triethylamin und 1.93g (12 mmol) 2-Hydroxy-2-(2,2-dimethyl-1,3-dioxolan-4-yl)-ethylamin hinzu und rührt 5 Stunden bei Raumtemperatur. Die Reaktionslösung wird dann im Vakuum zur Trockne eingedampft, der Rückstand mit Essigester extrahiert, der Essigesterextrakt im Vakuum eingedampft und der Rückstand an Kieselgel 60 (Merck) mit Essigester/Hexan chromatografiert. Man erhält 3.42g (6.75mmol) = 67.5% d. Th. des Produktes als amorphen Feststoff.

| Analyse : C₂₁H₂₂F₄N₂O₆S MG 506.47 | | | | | | | | | | | | |
|---|---|---|---|---|---|---|---|---|---|---|---|---|
| berechnet | C | 49.8 | H | 4.37 | F | 15.0 | N | 5.53 | O | 18.95 | S | 6.33% |
| gefunden | | 50.04 | | 4.53 | | 14.86 | | 5.77 | | | | 6.17% |

b) 3-[N-(4-Fluor-2-trifluormethylphenyl)sulfamoyl]-benzoesäure-[2,3,4-trihydroxy-butyl)-amid
2.53g (5 mmol) 3-[N-(4-Fluor-2-trifluormethyl-phenyl)sulfamoyl]-benzoesäure-[2-hydroxy-2-(2,2-dimethyl-1,3-dioxolan-4-yl)-ethyl-amid] werden in 20ml Methanol/Wasser 1 : 1 gelöst, ca. 100mg Kationenaustauscher Amberlyst 15 hinzugefügt und 1 Stunde auf Siedetemperatur erwärmt. Der Ionenaustauscher wird dann abfiltriert und das Filtrat im Vakuum zur Trockne eingedampft. Man erhält 1.94g (4.18 mmol ) = 83.5% d. Th. des Produktes als amorphen Feststoff.

| Analyse : C₁₈H₁₇F₄N₂O₆S MG 465.4 | | | | | | | | | | | | |
|---|---|---|---|---|---|---|---|---|---|---|---|---|
| berechnet | C | 46.45 | H | 3.68 | F | 16.32 | N | 6.01 | O | 20.62 | S | 6.89% |
| gefunden | | 46.63 | | 3.92 | | 16.11 | | 5.87 | | | | 6.72% |

### Beispiel 17

### 3-[N-(4-Fluor-2-trifluormethylphenyl)sulfamoyl]-benzolsulfonsäure-(2,3,4-trihydroxy-butyl)-N-methyl-amid

a) 3-[N-(4-Fluor-2-trifluormethyl-phenyl)sulfamoyl]-benzolsulfonsäurechlorid
8.78g (30 mmol) 4-Fluor-2-(trifluormethyl)-anilin werden unter Feuchtigkeitsausschluß in 88 ml Dichlorethan gelöst, 5.55g (70 mmol) Pyridin hinzugefügt und die Lösung auf 0°C gekühlt. Unter Beibehaltung dieser Temperatur wird eine Lösung von 9.9g (36 mmol) 1,3-Benzoidisulfonsäuredichlorid in 50ml Dichlorethan zugetropft, 30 Minuten bei 0°C und 5 Stunden bei Raumtemperatur nachgerührt. Die Suspension wird dreimal mit 2N Salzsäure und zweimal mit Wasser ausgeschüttelt, die organische Phase über Natriumsulfat getrocknet, filtriert, eingedampft und der Rückstand an Kieselgel 60 ( Merck ) mit Essigester/Hexan 1 : 1 chromatografiert. Die eingedampften Fraktionen der entsprechenden Polarität ergeben 9.7 g (23.22 mmol) = 77.4% d. Th. teilweise kristallines Produkt.

| Analyse : C₁₃H₈ClF₄NO₄S₂ MG 417.78 | | | | | | | | | | | | | | |
|---|---|---|---|---|---|---|---|---|---|---|---|---|---|---|
| berechnet | C | 37.37 | H | 1.93 | Cl | 8.48 | F | 18.19 | N | 3.35 | O | 15.31 | S | 15.35% |
| gefunden | C | 37.54 | H | 2.12 | Cl | 8.22 | F | 17.96 | N | 3.55 | | | S | 15.16% |

b) 3-[N-(4-Fluor-2-trifluormethyl-phenyl)sulfamoyl]-benzolsulfonsäure-[2-hydroxy-2-(2,2-dimethyl-1,3-dioxolan-4-yl)-N-methyl-ethylamid]
8.36g (20 mmol) 3-[N-(4-Fluor-2-trifluormethylphenyl)sulfamoyl]-benzolsulfonsäurechlorid werden in 40ml Tetrahydrofuran gelöst, 2.52g (25 mmol) Triethylamin und 4.03g (25 mmol) 2-Hydroxy-2-(2,2-dimethyl-1,3-dioxolan-4-yl)-N-methyl-ethylamin hinzugefügt und 3 Stunden bei 45°C gerührt. Das Reaktionsgemisch wird dann unter vermindertem Druck eingedampft, der Rückstand mit Essigester extrahiert und der Extrakt an Kieselgel 60 (Merck) mit Essigester/Hexan chromatografiert. Man erhält 9.35g (16.8 mmol) = 84% d. Th. als amorphen Feststoff.

| Analyse : C₂₁H₂₄F₄N₂O₇S₂ MG 556.55 | | | | | | | | | | | | |
|---|---|---|---|---|---|---|---|---|---|---|---|---|
| berechnet | C | 45.32 | H | 4.34 | F | 13.65 | N | 5.03 | O | 20.12 | S | 11.52% |
| gefunden | | 45.57 | | 4.61 | | 13.37 | | 5.22 | | | | 11.71% |

c) 3-[N-(4-Fluor-2-trifluormethyl-phenyl)sulfamoyl]-benzolsulfonsäure-(2,3,4-trihydroxy-butyl)amid
2.78g (5 mmol) 3-[N-(4-Fluor-2-trifluormethylphenyl)sulfamoyl]-benzolsulfonsäure-[2-hydroxy-2-(2,2-dimethyl-1,3-dioxolan-4-yl)-ethylamid] werden in 10ml Methanol gelöst, 10ml Wasser und eine katalytische Menge Kationenaustaucher Amberlyst 15 hinzugefügt und 1 Stunde gekocht. Der Ionenaustauscher wird dann abfiltriert und das Filtrat zur Trockne eingedampft. Man erhält 2.12g (4.12 mmol) = 82.3% d. Th. des Produktes als amorphen Feststoff.

| Analyse : C₁₈H₁₉F₄N₂O₇S₂ MG 515.48 | | | | | | | | | | | | |
|---|---|---|---|---|---|---|---|---|---|---|---|---|
| berechnet | C | 41.94 | H | 3.71 | F | 14.74 | N | 5.43 | O | 21.72 | S | 12.44% |
| gefunden | | 41.7 | | 3.96 | | 14.55 | | 5.32 | | | | 12.57% |

### Beispiel 18

### 4-[N-(4-Fluor-2-trifluormethylphenyl)sulfamoylmethyl]-benzoesäure-methylester

5.29g (29.5 mmol) 4-Fluor-2-(trifluormethyl)-anilin und 5ml Pyridin werden unter Feuchtigkeitsausschluß In 200ml Dichlormethan gelöst und die Lösung auf 0°C gekühlt. Zu dieser Lösung werden portionsweise 7.5g (29.5 mmol) 4-Chlorsulfonylmethyl-benzoesäure-methylester hinzugefügt und 1 Stunde bei 0°C gerührt. Die Reaktionslösung wird mit 2N Salzsäure pyridinfrei gewaschen, zweimal mit Wasser ausgeschüttelt, die organische Phase über Natriumsulfat getrocknet, filtriert und zur Trockne eingedampft. Der Rückstand wird im Vakuum getrocknet. Man erhält 10.27g (26.24 mmol) = 89% d. Th.

| Analyse : C₁₆H₁₃F₄NO₄S MG 391.34 | | | | | | | | | | | | |
|---|---|---|---|---|---|---|---|---|---|---|---|---|
| berechnet | C | 49.11 | H | 3.35 | F | 19.42 | N | 3.58 | O | 16.35 | S | 8.19% |
| gefunden | | 49.39 | | 3.23 | | 19.67 | | 3.66 | | | | 8.15% |

### Beispiel 19

### 4-[N-(4-Fluor-2-trifluormethylphenyl)sulfamoylmethyl]-benzoesäure

8.6g (21.98 mmol) 4-[N-(4-Fluor-6-trifluormethylphenyl)sulfamoylmethyl]-benzoesäure-methylester werden in 150ml Dioxan gelöst, bzw. suspendiert, 25ml (50 mmol) 2N Natronlauge hinzugefügt und 15 Minuten bei 60°C gerührt. Die Lösung wird dann mit konzentrierter Salzsäure angesäuert, wobei die Säure als Feststoff ausfällt. Sie wird abgesaugt, mit Wasser gewaschen und in 50ml Essigester gelöst. Diese Lösung wird über Natriumsulfat getrocknet, filtriert, eingedampft und der Rückstand im Vakuum getrocknet. Man erhält 7.35g (19.48 mmol) = 88.62% d. Th.

| Analyse : C₁₅H₁₁F₄NO₄S MG 377.31 | | | | | | | | | | | | |
|---|---|---|---|---|---|---|---|---|---|---|---|---|
| berechnet | C | 47.75 | H | 2.94 | F | 20.14 | N | 3.71 | O | 16.96 | S | 8.5% |
| gefunden | | 47.88 | | 3.07 | | 20.25 | | 3.56 | | | | 8.35% |

### Beispiel 20

### 4-[N-(4-Fluor-2-trifluormethylphenyl)sulfamoylmethyl]-benzoesäure-(2,3,4-trihydroxy-butyl)amid

a) 4-[N-(4-Fluor-2-trifluormethylphenyl)sulfamoylmethyl]-benzoesäure-[2-hydroxy-2-(2,2-dimethyl-1,3-dioxolan-4-yl)-ethylamid]
3.24g (8.59 mmol) 4-[N-(4-Fluor-2-trifluormethylphenyl)sulfamoylmethyl]-benzoesäure werden unter Feuchtigkeitsausschluß in 30ml DMF gelöst, 1.32g (8.59 mmol) Hydroxybenztriazol und 1.39g (8.59 mmol) 2-Hydroxy-2-(2,2-dimethyl-1,3-dioxolan-4-yl)-ethylamin hinzugefügt und die Lösung auf -10°C gekühlt. Zur gekühlten Lösung werden portionsweise 1.77g (8.59 mmol) Dicyclohexylcarbodiimid hinzugegeben, 1 Stunde bei -10°C und 5 Stunden bei Raumtemperatur nachgerührt. Der Niederschlag des Dicyclohexylharnstoffs wird dann abgesaugt und das Filtrat im Vakuum eingedampft. Der Rückstand wird in 100ml Essigester gelöst, die Lösung mit gesättigter Bicarbonatlösung und mit Wasser ausgeschüttelt, über Natriumsulfat getrocknet, filtriert und zur Trockne eingedampft. Der Rückstand wird im Vakuum getrocknet. Man erhält 3.99g (7.67 mmol) = 89.24% d. Th.

| Analyse : C₂₂H₂₄F₄N₂O₆S MG 520.5 | | | | | | | | | | | | |
|---|---|---|---|---|---|---|---|---|---|---|---|---|
| berechnet | C | 50.77 | H | 4.65 | F | 14.6 | N | 5.38 | O | 18.44 | S | 6.16% |
| gefunden | | 50.94 | | 4.71 | | 14.42 | | 5.42 | | | | 6.14% |

b) 4-[N-(4-Fluor-2-trifluormethylphenyl)sulfamoylmethyl]-benzoesäure-(2,3,4-trihydroxy-butyl)amid
2.6g (5 mmol) 4-[N-(4-Fluor-2-trifluormethylphenyl)sulfamoylmethyl]-benzoesäure-[2-hydroxy-2-(2,2-dimethyl-1,3-dioxolan-4-yl)-ethylamid] werden in 20ml Wasser/Ethanol 1 : 1 suspendiert, mit Salzsäure auf pH 1 angesäuert und 1 Stunde bei 60°C gerührt. Die Reaktionslösung wird mit Anionenaustauscher Amberlite IRA 67 neutralisiert, der Ionenaustauscher abfiltriert und das Filtrat im Vakuum zur Trockne eingedampft. Der Rückstand wird im Vakuum getrocknet. Man erhält 2.2g (4.58 mmol) = 91.58% d. Th.

| Analyse : C₁₉H₂₀F₄N₂O₆S MG 480.43 | | | | | | | | | | | | |
|---|---|---|---|---|---|---|---|---|---|---|---|---|
| berechnet | C | 47.5 | H | 4.2 | F | 15.82 | N | 5.83 | O | 19.98 | S | 6.67% |
| gefunden | | 47.55 | | 4.4 | | 15.65 | | 5.91 | | | | 6.73% |

### Beispiel 21

### 2-Fluor-5-trifluormethylsulfamoyl-benzoesäure

a) 2-Fluor-5-(trifluormethylsulfamoyl)-benzoesäuremethylester
3.83g (20 mmol) 5-Amino-2-fluor-benzoesäure-hydrochlorid werden in 30ml Dioxan gelöst, 3.56g (45 mmol) Pyridin hinzugefügt, die Lösung auf 5°C gekühlt und unter Beibehaltung dieser Temperatur und Feuchtigkeitsausschluß eine Lösung von 8.46g (30 mmol) Trifluormethansulfonsäureanhydrid in 30ml Dioxan zugetropft. Es wird 2 Stunden bei 5°C und 2 Stunden bei Raumtemperatur nachgerührt, die Reaktionslösung unter vermindertem Druck eingedampft und der Rückstand in 100ml Methanol gelöst. Diese Lösung wird mit 1ml konzentrierter Schwefelsäure versetzt und anschließend 3 Stunden gekocht. Das Methanol wird dann im Vakuum zum größten Teil abdestilliert, das Konzentrat in 100ml Dichlorethan gelöst und dreimal mit je 30ml Wasser ausgeschüttelt. Die organische Phase wird über Natriumsulfat getrocknet, eingedampft und der Rückstand an Kieselgel 60 (Merck) mit Essigester/Hexan chromatografiert. Man erhält 3.97g (13.18 mmol) = 65.9% d. Th. überwiegend kristallines Produkt.

| Analyse : C₉H₇F₄NO₄S MG 301.22 | | | | | | | | | | | | |
|---|---|---|---|---|---|---|---|---|---|---|---|---|
| berechnet | C | 35.88 | H | 2.34 | F | 25.22 | N | 4.65 | O | 21.24 | S | 10.64% |
| gefunden | | 36.03 | | 2.55 | | 24.97 | | 4.53 | | | | 10.47% |

b) 2-Fluor-5-(trifluormethylsulfamoyl)-benzoesäure
3g (10 mmol) 2-Fluor-5-(trifluormethylsulfamoyl)-benzoesäuremethylester werden in 30ml Dioxan gelöst, 7.5ml (15 mmol) 2N Natronlauge zugefügt und 1 Stunde bei 50°C gerührt. Die Lösung wird dann im Vakuum eingedampft, der Rückstand in 30ml Wasser gelöst und die Lösung mit konzentrierter Salzsäure angesäuert. Die saure wässrige Lösung wird mit Essigester extrahiert. Der Essigesterextrakt wird über Natriumsulfat getrocknet, filtriert und eingedampft. Der Rückstand wird aus Essigester kristallisiert. Man erhält 2.12g (7.38 mmol) 73.8% d. Th.Kristallisat.

| Analyse : C₈H₅F₄NO₄S MG 287.19 | | | | | | | | | | | | |
|---|---|---|---|---|---|---|---|---|---|---|---|---|
| berechnet | C | 33.45 | H | 1.75 | F | 26.46 | N | 4.87 | O | 22.28 | S | 11.16% |
| gefunden | | 33.67 | | 1.98 | | 26.27 | | 5.03 | | | | 10.92 |

### Beispiel 22

### 2-Fluor-5-(trifluormethylsulfamoyl)-benzoesäure-(2,3,4-trihydroxy-butyl)amid

a) 2-Fluor-5-(trifluormethylsulfamoyl)-benzoesäure-[2-hydroxy-2-(2,2-dimethyl-1,3-dioxolan-4-yl)-ethylamid
2.87g (10 mmol) 2-Fluor-5-(trifluormethylsulfamoyl)-benzoesäure werden unter Feuchtigkeitsausschluß in 30ml DMF gelöst, 2.03g (15 mmol) Hydroxybenztriazol und 2.42g (15 mmol) 2-Hydroxy-2-(2,2-dimethyl-1,3-dioxolan-4-yl)-ethylamin hinzugefügt und die Lösung auf -10°C gekühlt. Zur gekühlten Lösung werden portionsweise 3.09g (15 mmol) Dicyclohexylcarbodiimid hinzugegeben, 1 Stunde bei -10°C und 5 Stunden bei Raumtemperatur nachgerührt. Der Niederschlag des Dicyclohexylharnstoffs wird dann abgesaugt und das Filtrat im Vakuum eingedampft. Der Rückstand wird in 100ml Essigester gelöst, die Lösung mit gesättigter Bicarbonatlösung und mit Wasser ausgeschüttelt, über Natriumsulfat getrocknet, filtriert und zur Trockne eingedampft. Man erhält 3.58g (8.32 mmol) = 83.2% d. Th. amorphen Feststoff.

| Analyse : C₁₅H₁₈F₄N₂O₆S MG 430.77 | | | | | | | | | | | | |
|---|---|---|---|---|---|---|---|---|---|---|---|---|
| berechnet | C | 41.86 | H | 4.21 | F | 17.65 | N | 6.5 | O | 22.3 | S | 7.45% |
| gefunden | | 41.63 | | 4.46 | | 17.87 | | 6.53 | | | | 7.63% |

b) 2-Fluor-5-(trifluormethylsulfamoyl)-benzoesäure-(2,3,4-trihydroxy-butyl)amid
2.15g (5 mmol) 2-Fluor-5-(trifluormethylsulfamoyl)-benzoesäure-[2-hydroxy-2-(2,2-dimethyl-1,3-dioxolan-4-yl)-ethylamid] werden in 20ml Wasser/Methanol 1 : 1 suspendiert, mit einer katalytischen Menge Kationenaustauscher Amberlyst 15 versetzt und 3 Stunden gekocht. Die Reaktionslösung wird filtriert und das Filtrat im Vakuum zur Trockne eingedampft. Man erhält 1.7g (4.37 mmol) = 87.4% d. Th. amorphen Feststoff.

| Analyse : C₁₂H₁₃F₄N₂O₆S MG 389.3 | | | | | | | | | | | | |
|---|---|---|---|---|---|---|---|---|---|---|---|---|
| berechnet | C | 37.02 | H | 3.36 | F | 19.52 | N | 7.19 | O | 24.65 | S | 8.23% |
| gefunden | | 37.26 | | 3.51 | | 19.37 | | 6.96 | | | | 8.37% |

### Beispiel 23

### 5-Fluor-2-[N-(2,2,2-trifluorethylsulfonyl)amido]-benzoesäure

8.62g (45 mmol) 2-Amino-5-fluor-benzoesäure-hydrochlorid werden unter Feuchtigkeitsausschluß in 150ml Dichlormethan gelöst, bzw. suspendiert, 5ml Pyridin hinzugefügt und das Gemisch auf -8°C gekühlt. Unter Beibehaltung dieser Temperatur wird eine Lösung von 8.21g (45 mmol) 2,2,2-Trifluorethylsulfochlorid in 20ml Dichlormethan zugetropft. Man rührt anschließend 1 Stunde bei - 8°C und 1 Stunde bei Raumtemperatur nach. Das Reaktionsgenisch wird dann zweimal mit je 30ml 2N Salzsäure und zweimal mit Wasser ausgeschüttelt, die organische Phase über Natriumsulfat getrocknet, filtriert und zur Trockne eingedampft. Der Rückstand wird aus Diethylether, dem man etwas Pentan zusetzt, teils kristallin, teils amorph abgeschieden. Man erhält 8.65g (28.71 mmol) = 63.8% d. Th..

| Analyse : C₉H₇F₄NO₄S MG 301.22 | | | | | | | | | | | | |
|---|---|---|---|---|---|---|---|---|---|---|---|---|
| berechnet | C | 35.88 | H | 2.34 | F | 25.22 | N | 4.65 | O | 21.24 | S | 10.64% |
| gefunden | | 35.96 | | 2.51 | | 25.03 | | 4.47 | | | | 10.43% |

### Beispiel 24

### 5-Fluor-2-[N-(2,2,2-trifluorethylsulfonyl)amido]-benzoesäure-(2,3,4-trihydroxy-butyl)amid

a) 5-Fluor-2-[N-(2,2,2-trifluorethylsulfonyl)amido]-benzoesäure-[2-hydroxy-2-(2,2-dimethyl-1,3-dioxolan-4-yl)-ethylamid]
3.01g (10 mmol) 5-Fluor-2-[N-(2,2,2-trifluorethylsulfonyl)amido]-benzoesäure werden unter Feuchtigkeitsausschluß in 30ml DMF gelöst, 2.03g (15 mmol) Hydroxybenztriazol und 2.42g (15 mmol) 2-Hydroxy-2-(2,2-dimethyl-1,3-dioxolan-4-yl)-ethylamin hinzugefügt und die Lösung auf -10°C gekühlt. Zur gekühlten Lösung werden portionsweise 3.09g (15 mmol) Dicyclohexylcarbodiimid hinzugegeben, 1 Stunde bei -10°C und 5 Stunden bei Raumtemperatur nachgerührt. Der Niederschlag des Dicyclohexylharnstoffs wird dann abgesaugt und das Filtrat im Vakuum eingedampft. Der Rückstand wird in 100ml Essigester gelöst, die Lösung mit gesättigter Bicarbonatlösung und mit Wasser ausgeschüttelt, über Natriumsulfat getrocknet filtriert und zur Trockne eingedampft. Man erhält 3.53g (7.95 mmol) = 79.5% d. Th. der Verbindung als amorphen Feststoff.

| Analyse : C₁₆H₂₀F₄N₂O₆S MG 444.4 | | | | | | | | | | | | |
|---|---|---|---|---|---|---|---|---|---|---|---|---|
| berechnet | C | 43.24 | H | 4.53 | F | 17.1 | N | 6.3 | O | 21.6 | S | 10.17% |
| gefunden | | 43.55 | | 4.71 | | 16.87 | | 6.15 | | | | 9.75% |

b) 5-Fluor-2-[N-(2,2,2-trifluorethylsulfonyl)amido]-benzoesäure-(2,3,4-trihydroxy-butyl)amid
2.22g (5 mmol) 5-Fluor-2-[N-(2,2,2-trifluorethylsulfonyl)amido]-benzoesäure-[2-hydroxy-2-(2,2-dimethyl-1,3-dioxolan-4-yl)-ethylamid] werden in 20ml Wasser/Methanol 1 : 1 suspendiert, mit einer katalytischen Menge Kationenaustauscher Amberlyst 15 versetzt und 3 Stunden gekocht. Die Reaktionslösung wird filtriert und das Filtrat im Vakuum zur Trockne eingedampft. Man erhält 1.68g (4.17 mmol) = 83.4% d. Th. amorphen Feststoff.

| Analyse : C₁₃H₁₅F₄N₂O₆S MG 403.33 | | | | | | | | | | | | |
|---|---|---|---|---|---|---|---|---|---|---|---|---|
| berechnet | C | 38.71 | H | 3.74 | F | 18.84 | N | 6.94 | O | 23.8 | S | 7.95% |
| gefunden | | 38.92 | | 3.87 | | 18.56 | | 7.13 | | | | 7.72% |

### Beispiel 25

### 4-[N-(2,4,6-Trifluorphenyl)sulfamoyl]-benzoesäure-(2,3,4-trihydroxy-butyl)amid

a) 4-[N-(2,4,6-Trifluorphenyl)sulfamoyl]-benzoesäuremethylester
2.35g (10 mmol) 4-Chlorsulfonyl-benzoesäuremethylester werden unter Feuchtigkeitsausschluß in 30ml Dichlormethan suspendiert, die Suspension auf -50°C gekühlt und bei dieser Temperatur eine Lösung von 1.47g (10 mmol) 2,4,6- Trifluoranilin und 1ml Pyridin in 20ml Dichlormethan zugetropft. Nach beendetem Eintropfen läßt man auf Raumtemperatur kommen und rührt 12 Stunden nach. Der entstandene Niederschlag wird abgesaugt und mit Ether extrahiert. Der Etherextrakt und das Filtrat werden zusammen eingedampft, der Rückstand in Dichlorethan gelöst und an Kieselgel 60 (Merck) mit Essigester/Hexan chromatografiert. Man erhält 2.71g (7.96 mmol) = 78.6% d. Th. teils kristallines, teils amorphes Produkt.

| Analyse : C₁₄H₁₀F₃NO₄S MG 345.3 | | | | | | | | | | | | |
|---|---|---|---|---|---|---|---|---|---|---|---|---|
| berechnet | C | 48.69 | H | 2.91 | F | 16.5 | N | 4.05 | O | 18.53 | S | 9.28% |
| gefunden | | 48.43 | | 3.97 | | 16.32 | | 3.87 | | | | 9.44% |

b) 4-[N-(2,4,6-Trifluorphenyl)sulfamoyl]-benzoesäure
6.22g (18 mmol) 4-[N-(2,4,6-Trifluorphenyl)sulfamoyl]-benzoesäuremethylester werden in 50ml Dioxan gelöst, 18ml (32 mmol) 2N NaOH hinzugefügt und 3 Stunden bei 50°C gerührt. Die Lösung wird dann bei vermindertem Druck auf ca. 10ml eingeengt, mit 2N Salzsäure angesäuert und der feste Niederschlag, der dabei entsteht abgesaugt. Der Filterrückstand wird in Essigester gelöst, die Lösung zweimal mit Wasser ausgeschüttelt, über Natriumsulfat getrocknet, filtriert und zur Trockne eingedampft. Man erhält 5.58g (16.85 mmol) = 76.9% d. Th. als amorphen Feststoff.

| Analyse : C₁₃H₈F₃NO₄S MG 331.27 | | | | | | | | | | | | |
|---|---|---|---|---|---|---|---|---|---|---|---|---|
| berechnet | C | 47.13 | H | 2.43 | F | 17.2 | N | 4.22 | O | 19.31 | S | 9.67% |
| gefunden | | 47.37 | | 2.56 | | 16.94 | | 4.4 | | | | 9.45% |

c) 4-[N-(2,4,6-Trifluorphenyl)sulfamoyl]-benzoesäure-[2-hydroxy-2-(2,2-dimethyl-1,3-dioxolan-4-yl)-ethylamid]
4.97g (15 mmol) 4-[N-(2,4,6-Trifluorphenyl)sulfamoyl]-benzoesäure werden unter Argonatmosphäre in 50ml trockenem Dimethylformamid gelöst, 2.03g (15 mmol) Hydroxybenztriazol und 2.42g (15 mmol) 2-Hydroxy-2-(2,2-dimethyl-1,3-dioxolan-4-yl)-ethylamin hinzugefügt und die Lösung auf -10°C gekühlt. Bei dieser Temperatur werden der Lösung 3.09g (15 mmol) Dicyclohexylcarbodiimid zugesetzt. Anschließend wird 1 Stunde bei -10°C und 10 Stunden bei Raumtemperatur gerührt. Die trübe Reaktionslösung wird filtriert und das Filtrat im Vakuum eingedampft. Der Rückstand wird in 100ml Essigester gelöst, die Lösung fünfmal mit gesättigter Natriumbicarbonatlösung und zweimal mit Wasser ausgeschüttelt, die organische Phase über Natriumsulfat getrocknet, filtriert und eingedampft. Man erhält 3.46g (7.29 mmol) = 48.6% d. Th. Feststoff.

| Analyse : C₂₀H₂₁F₃N₂O₆S MG 474.46 | | | | | | | | | | | | |
|---|---|---|---|---|---|---|---|---|---|---|---|---|
| berechnet | C | 50.63 | H | 4.46 | F | 12.01 | N | 5.9 | O | 20.23 | S | 6.75% |
| gefunden | | 50.78 | | 4.63 | | 11.87 | | 6.05 | | | | 6.83% |

d) 4-[N-(2,4,6-Trifluorphenyl)sulfamoyl]-benzoesäure-(2,3,4-trihydroxy-butyl)amid
4.74g (10 mmol) 4-[N-(2,4,6-Trifluorphenyl)sulfamoyl]-benzoesäure-[2-hydroxy-2-(2,2-dimethyl-1,3-dioxolan-4-yl)-ethylamid] werden in einem Gemisch aus 50ml Ethanol und 20ml Wasser gelöst und die Lösung mit verdünnter Salzsäure auf pH 1 angesäuert. Bei diesem pH wird 4 Stunden bei 60°C gerührt. Die Lösung wird dann mit Anionenaustauscher Amberlite IRA 67 auf pH 6.3 gestellt, filtriert und im Vakuum zur Trockne eingedampft. Der feste amorphe Rückstand wird im Vakuum bei 50°C getrocknet. Man erhält 3.4g (7.83 mmol) = 78.3 % d. Th. als amorphen Feststoff.

| Analyse : C₁₇H₁₇F₃N₂O₆S MG 434.39 | | | | | | | | | | | | |
|---|---|---|---|---|---|---|---|---|---|---|---|---|
| berechnet | C | 47.0 | H | 3.94 | F | 13.12 | N | 6.44 | O | 22.09 | S | 7.38% |
| gefunden | | 47.26 | | 4.12 | | 12.9 | | 6.23 | | | | 7.27% |

### Beispiel 26

### 3-[N-(2,4,6-Trifluorphenyl)sulfamoyl]-benzoesäure-(2,3,4-trihydroxy-butyl)amid

a) 3-[N-(2,4,6-Trifluorphenyl)sulfamoyl]-benzoesäuremethylester
2.35g (10 mmol) 3-Chlorsulfonyl-benzoesäuremethylester werden unter Feuchtigkeitsausschluß in 30ml Dichlormethan suspendiert, die Suspension auf -5°C gekühlt und bei dieser Temperatur eine Lösung von 1.47g (10 mmol) 2,4,6- Trifluoranilin und 1ml Pyridin in 20ml Dichlormethan zugetropft. Nach beendetem Eintropfen läßt man auf Raumtemperatur kommen und rührt 12 Stunden nach. Der entstandene Niederschlag wird abgesaugt und mit Ether extrahiert. Der Etherextrakt und das Filtrat werden zusammen eingedampft und der Rückstand in Dichlorethan gelöst und an Kieselgel 60 (Merck) mit Essigester/Hexan chromatografiert. Man erhält 2.8g (8.12 mmol) = 81.2% d. Th. amorphes Produkt.

| Analyse : C₁₄H₁₀F₃NO₄S MG 345.3 | | | | | | | | | | | | |
|---|---|---|---|---|---|---|---|---|---|---|---|---|
| berechnet | C | 48.69 | H | 2.91 | F | 16.5 | N | 4.05 | O | 18.53 | S | 9.28% |
| gefunden | | 48.84 | | 3.25 | | 16.57 | | 4.23 | | | | 9.03% |

b) 3-[N-(2,4,6-Trifluorphenyl)sulfamoyl]-benzoesäure
4.49g (13 mmol) 3-[N-(2,4,6-Trifluorphenyl)sulfamoyl]-benzoesäuredimethylester werden in 50ml Dioxan gelöst, 13ml (26 mmol) 2N NaOH hinzugefügt und 3 Stunden bei 50°C gerührt. Die Lösung wird dann bei vermindertem Druck auf ca. 10ml eingeengt, mit 2N Salzsäure angesäuert und der feste Niederschlag, der dabei entsteht abgesaugt. Der Filterrückstand wird in Essigester gelöst, die Lösung zweimal mit Wasser ausgeschüttelt, über Natriumsulfat getrocknet, filtriert und zur Trockne eingedampft. Man erhält 2.42g (7.32 mmol) = 73.2% d. Th. amorphen Feststoff.

| Analyse : C₁₃H₈F₃NO₄S MG 331.27 | | | | | | | | | | | | |
|---|---|---|---|---|---|---|---|---|---|---|---|---|
| berechnet | C | 47.13 | H | 2.43 | F | 17.2 | N | 4.22 | O | 19.31 | S | 9.67% |
| gefunden | | 46.88 | | 2.64 | | 16.98 | | 4.13 | | | | 9.83% |

c) 3-[N-(2,4,6-Trifluorphenyl)sulfamoyl]-benzoesäure-[2-hydroxy-2-(2,2-dimethyl-1,3-dioxolan-4-yl)-ethylamid]
5.96g (18 mmol) 3-[N-(2,4,6-Trifluorphenyl)sulfamoyl]-benzoesäure werden unter Argonatmosphäre in 50ml trockenem Dimethylformamid gelöst, 2.76g (18 mmol) Hydroxybenztriazol und 2.9g (18 mmol) 2-Hydroxy-2-(2,2-dimethyl-1,3-dioxolan-4-yl)-ethylamin hinzugefügt und die Lösung auf -10°C gekühlt. Bei dieser Temperatur werden der Lösung 3.71g (18 mmol) Dicyclohexylcarbodiimid zugesetzt. Anschließend wird 1 Stunde bei -10°C und 6 Stunden bei Raumtemperatur gerührt. Die trübe Reaktionslösung wird filtriert und das Filtrat im Vakuum eingedampft. Der Rückstand wird in 100ml Essigester gelöst, die Lösung fünfmal mit gesättigter Natriumbicarbonatlösung und zweimal mit Wasser ausgeschüttelt, die organische Phase über Natriumsulfat getrocknet, filtriert und eingedampft. Der Rückstand wird an Kieselgel 60 (Merck) mit Essigester/Hexan chromatografiert. Man erhält 4.91g (10.35 mmol) = 57.5% d. Th. amorphen Feststoff.

| Analyse : C₂₀H₂₁F₃N₂O₆S MG 474.46 | | | | | | | | | | | | |
|---|---|---|---|---|---|---|---|---|---|---|---|---|
| berechnet | C | 50.63 | H | 4.46 | F | 12.01 | N | 5.9 | O | 20.23 | S | 6.75% |
| gefunden | | 50.55 | | 4.67 | | 12.19 | | 6.16 | | | | 6.53% |

d) 3-[N-(2,4,6-Trifluorphenyl)sulfamoyl]-benzoesäure-(2,3,4-trihydroxy-butyl)amid
4.74g (10 mmol) 3-[N-(2,4,6-Trifluorphenyl)sulfamoyl]-benzoesäure-[2-hydroxy-2-(2,2-dimethyl-1,3-dioxolan-4-yl)-ethylamid] werden in einem Gemisch aus 50ml Ethanol und 20ml Wasser gelöst und die Lösung mit verdünnter Salzsäure auf pH 1 angesäuert. Bei diesem pH wird 4 Stunden bei 60°C gerührt. Die Lösung wird dann mit Anionenaustauscher Amberlite IRA 67 auf pH 6.6 gestellt, filtriert und im Vakuum zur Trockne eingedampft. Der feste amorphe Rückstand wird im Vakuum bei 50°C getrocknet. Man erhält 3.61g (8.32 mmol) = 83.2.% d. Th. als amorphen Feststoff.

| Analyse : C₁₇H₁₇F₃N₂O₆S MG 434.39 | | | | | | | | | | | | |
|---|---|---|---|---|---|---|---|---|---|---|---|---|
| berechnet | C | 47.0 | H | 3.94 | F | 13.12 | N | 6.44 | O | 22.09 | S | 7.38% |
| gefunden | | 46.83 | | 4.15 | | 13.27 | | 6.46 | | | | 7.53% |

### Beispiel 27

### 5-[N-(2,4,6-Trifluorphenyl)sulfamoyl]-isophthalsäure-bis(2,3,4-trihydroxybutyl)-diamid

a) 5-[N-(2,4,6-Trifluorphenyl)sulfamoyl]-isophthalsäuredimethylester
2.94g (20 mmol) 2,4,6-Trifluoranilin werden unter Feuchtigkeitsausschluß in 50 ml Dichlorethan gelöst, 3.16g (40 mmol) Pyridin hinzugefügt und die Lösung auf 0°C gekühlt. Unter Beibehaltung dieser Temperatur wird eine Lösung von 5.85g (20 mmol) 5-Chlorsulfonyl-isophthalsäuredimethylester in 50ml Dichlorethan zugetropft, 30 Minuten bei 0°C und 5 Stunden bei Raumtemperatur nachgerührt. Die Suspension wird dreimal mit 2N Salzsäure und zweimal mit Wasser ausgeschüttelt, die organische Phase über Natriumsulfat getrocknet, filtriert, eingedampft und der Rückstand an Kieselgel 60 ( Merck ) mit Essigester/Hexan chromatografiert. Die eingedampften Fraktionen der entsprechenden Polarität ergeben 6.34g (15.72 mmol) = 78.6% d. Th. teilweise kristallines Produkt.

| Analyse : C₁₆H₁₂F₃NO₆S MG 403.33 | | | | | | | | | | | | |
|---|---|---|---|---|---|---|---|---|---|---|---|---|
| berechnet | C | 47.64 | H | 2.99 | F | 14.13 | N | 3.47 | O | 23.8 | S | 7.95% |
| gefunden | | 47.77 | | 3.16 | | 13.94 | | 3.58 | | | | 8.2 % |

b) 5-[N-(2,4,6-Trifluorphenyl)sulfamoyl]-isophthalsäure
6.05g (15 mmol) 5-[N-(2,4,6-Trifluorphenyl)sulfamoyl]-isophthalsäuredimethylester werden in 60ml Dioxan gelöst, 15ml (30 mmol) 2N NaOH hinzugefügt und 3 Stunden bei 50°C gerührt. Die Lösung wird dann bei vermindertem Druck auf ca. 15ml eingeengt, mit 2N Salzsäure angesäuert und der feste Niederschlag, der dabei entsteht, abgesaugt. Der Filterrückstand wird in Essigester gelöst, die Lösung zweimal mit Wasser ausgeschüttelt, über Natriumsulfat getrocknet, filtriert und zur Trockne eingedampft. Man erhält 4.8g (12.78 mmol) = 85.22% d. Th. als amorphen Feststoff.

| Analyse : C₁₄H₈F₃NO₆S MG 375.28 | | | | | | | | | | | | |
|---|---|---|---|---|---|---|---|---|---|---|---|---|
| berechnet | C | 44.8 | H | 2.14 | F | 15.18 | N | 3.73 | O | 25.57 | S | 8.54% |
| gefunden | | 45.03 | | 2.32 | | 14.93 | | 4.0 | | | | 8.33% |

c) 5-[N-(2,4,6-Trifluorphenyl)sulfamoyl]-isophthalsäure-bis[2-hydroxy-2-(2,2-dimethyl-1,3-dioxolan-4-yl)-ethylamid]
4.5g (12 mmol) 5-[N-(2,4,6-Trifluorphenyl)sulfamoyl]-isophthalsäure werden unter Argonatmosphäre in 50ml trockenem Dimethylformamid gelöst, 2.03g (15 mmol) Hydroxybenztriazol und 2.42g (15 mmol) 2-Hydroxy-2-(2,2-dimethyl-1,3-dioxolan-4-yl)-ethylamin hinzugefügt und die Lösung auf -10°C gekühlt. Bei dieser Temperatur werden der Lösung 3.09g (15 mmol) Dicyclohexylcarbodiimid zugesetzt, 1 Stunde bei -10°C und 8 Stunden bei Raumtemperatur gerührt. Die trübe Reaktionslösung wird filtriert und das Filtrat im Vakuum eingedampft. Der Rückstand wird in 100ml Essigester gelöst, die Lösung fünfmal mit gesättigter Natriumbicarbonatlösung und zweimal mit Wasser ausgeschüttelt, die organische Phase über Natriumsulfat getrocknet, filtriert und eingedampft. Der Rückstand wird an Kieselgel 60 (Merck) mit Essigester/Hexan chromatografiert. Man erhält 4.15g (6.28 mmol) = 52.33% d. Th. amorphen Feststoff.

| Analyse : C₂₈H₃₄F₃N₃O₁₀S MG 661.65 | | | | | | | | | | | | |
|---|---|---|---|---|---|---|---|---|---|---|---|---|
| berechnet | C | 50.82 | H | 5.18 | F | 8.61 | N | 6.35 | O | 24.18 | S | 4.84% |
| gefunden | | 51.05 | | 5.32 | | 8.47 | | 6.23 | | | | 4.65% |

d) 5-[N-(2,4,6-Trifluorphenyl)sulfamoyl]-isophthalsäure-bis(2,3,4-trihydroxybutyl)-diamid
3.31g (5 mmol) 5-[N-(2,4,6-Trifluorphenyl)sulfamoyl]-isophthalsäure-bis[2-hydroxy-2-(2,2-dimethyl-1,3-dioxolan-4-yl)-ethylamid] werden in einem Gemisch aus 20ml Ethanol und 10ml Wasser gelöst und die Lösung mit verdünnter Salzsäure auf pH 1 angesäuert. Bei diesem pH wird 4 Stunden bei 60°C gerührt. Die Lösung wird dann mit Anionenaustauscher Amberlite IRA 67 auf pH 6.4 gestellt, filtriert und im Vakuum zur Trockne eingedampft. Der feste amorphe Rückstand wird im Vakuum bei 50°C getrocknet. Man erhält 2.37g (4.07 mmol) = 81.37 % d. Th. als amorphen Feststoff.

| Analyse : C₂₂H₂₆F₃N₃O₁₀S MG 581.52 | | | | | | | | | | | | |
|---|---|---|---|---|---|---|---|---|---|---|---|---|
| berechnet | C | 45.44 | H | 4.5 | F | 9.8 | N | 7.22 | O | 27.51 | S | 5.51% |
| gefunden | | 45.67 | | 4.72 | | 9.66 | | 7.13 | | | | 5.77% |

### Beispiel 28

### 2-{N-[(4-Fluor-2-hydroxycarbonyl)phenyl]sulfamoyl}-essigsäure-[di-(2,2,2-trifluorethyl)amid]

a) 2-{N-[(4-Fluor-2-hydroxycarbonyl)phenyl]sulfamoyl}-essigsäuremethylester
In 150ml Dichlormethan werden 9,58g (50 mmol) 2-Amino-4-fluor-benzoesäure-Hydrochlorid und 5ml (61.8 mmol) Pyridin vorgelegt. Bei 0°C werden unter Feuchtigkeitsausschluß 8.63g (50 mmol) Chlorsulfonylessigsäuremethylester, gelöst in 20ml Dichlormethan, zugetropft. Man läßt über Nacht bei Raumtemperatur rühren, wascht die Lösung mit 2N Salzsäure und mit Wasser, trocknet über Natriumsulfat und engt im Vakuum zur Trockne ein. Das Produkt wird als amorpher Feststoff erhalten. Die Ausbeute beträgt 12.26g (42.09 mmol) = 84.2% d. Th.

| Analyse: C₁₀H₁₀FNO₆S MG 291.26 | | | | | | | | | | |
|---|---|---|---|---|---|---|---|---|---|---|
| berechnet | C | 41.24 | H | 3.46 | F | 6.52 | N | 4.81 | S | 11.01 % |
| gefunden | | 41.12 | | 3.51 | | 6.47 | | 4.88 | | 10.89 % |

b) 2-{N-[(4-Fluor-2-hydroxycarbonyl)phenyl] sulfamoyl}-essigsäure-[di-(2,2,2-trifluorethyl)amid]
230mg (10 mmol) Natrium werden in 50ml trockenem Methanol gelöst und zu dieser Lösung 2.91g (10 mmol) 2-[N-(4-Fluor-2-hydroxycarbonyl)sulfamoyl]-essigsäuremethylester hinzugefügt. Die Lösung wird zur Trockne eingedampft, unter Feuchtigkeitsausschluß in 10ml Bis(2,2,2-trifluorethyl)amin suspendiert und die Suspension 6 Stunden am Rückfluß gekocht. Das überschüssige Amin wird dann in eine Kühlfalle destilliert und der Rückstand in einem Gemisch aus Wasser und Ethanol gelöst. Die Lösung wird mit Salzsäure angesäuert und Produkt fällt als Feststoff aus, der im Vakuum getrocknet wird. Man erhält 3.46g (7.86 mmol) = 78.6% d. Th..

| Analyse : C₁₃H₁₁F₇N₂O₅S MG 440.3 | | | | | | | | | | |
|---|---|---|---|---|---|---|---|---|---|---|
| berechnet | C | 35.46 | H | 2.52 | F | 30.2 | N | 6.36 | S | 7.28 % |
| gefunden | | 35.35 | | 2.59 | | 30.1 | | 6.42 | | 7.17 % |

### Beispiel 29

### 5-Fluor-2-hydroxy-3-(2,2,2-trifluorethylcarbamoyl)-benzoesäure

a) (2,6-Dibrom-4-fluor-phenyl)-benzylether
2.21g (96 mmol) Natrium werden unter Feuchtigkeitsausschluß in 66ml Ethanol gelöst. Zu dieser Lösung fügt man 21,6g (80 mmol) 2,6-Dibrom-4-fluorphenol hinzu. Es entsteht eine Suspension. Dieser werden 20.5g (120 mmol) Benzylbromid zugesetzt, 30 Minuten bei Raumtemperatur gerührt und anschließend 1 Stunde am Rück fluß gekocht. Eine Probe der Suspension reagiert dann auf feuchtem Indikatorpapier neutral. Der Niederschlag des Natriumbromids wird abgesaugt, das Filtrat zur Trockne eingedampft und der Rückstand aus Ethanol kristallisiert. Man erhält 23.7g (66 mmol) = 82.5% d. Th. Kristallisat. Fp. 83°C.
b) 2-Benzyloxy-5-fluor-isophthalsäure-diethylester
10.8g (30 mmol) (2,6-Dibrom-4-fluor-phenyl)-benzylether werden in einer Argonatmospäre unter Feuchtigkeitsausschluß in 150ml trockenem Tetrahydrofuran gelöst und die Lösung auf -100°C gekühlt. Bei dieser Temperatur werden 225ml (180 mmol) einer 0.8-molaren Lösung von Butyllithium in Hexan zugetropft. Nach beendeter Zugabe rührt man 5 Minuten nach und tropft dann eine Lösung von 21.24g (180 mmol) Diethylcarbonat in 50ml trockenem Tetrahydrofuran ein. Die Temperatur darf dabei -40°C nicht übersteigen. Anschließend rührt man nach, bis das Reaktionsgemisch Raumtemperatur erreicht hat und weitere 2 Stunden bei dieser Temperatur. Das Reaktionsgemisch wird unter vermindertem Druck eingedampft, der Rückstand in Essigester gelöst und die Lösung mehrmals mit Wasser ausgeschüttelt. Die organische Phase wird über Natriumsulfat getrocknet, im Vakuum eingedampft und der Rückstand an Kieselgel 60 (Merck) mit Essigester/Hexan chromatografiert. Man erhält 5.43g (15.69 mmol) 52.3% d. Th des Diesters als amorpen Feststoff.

| Analyse : C₁₉H₁₉FO₅ MG 346.35 | | | | | | | | |
|---|---|---|---|---|---|---|---|---|
| berechnet | C | 65.99 | H | 5.53 | F | 5.48 | O | 23.09% |
| gefunden | | 66.17 | | 5.77 | | 5.26 | | % |

c) 2-Benzyloxy-5-fluor-isophthalsäure-monoethylester
25.63g (74 mmol) 2-Benzyloxy-5-fluor-isophthalsäure-diethylester werden unter Feuchtigkeitsausschluß in 200ml trockenem Ethanol gelöst und in diese Lösung im Verlaufe von 2 Stunden bei Raumtemperatur eine Lösung von 1.48g Natriumhydroxyd in 50ml Ethanol eingetropft. Das Mono-Natriumsalz fällt allmählich aus. Nach beendetem Eintropfen wird 1 Stunde nachgerührt, der Niederschlag abgesaugt und das Filtrat auf ca. die Hälfte eingeengt. Dabei fällt weiteres Mono-Natriumsalz, das ebenfalls abgesaugt wird. Beide Filterrückstände werden vereinigt, in Wasser suspendiert und die Suspension mit konzentrierter Salzsäure angesäuert. Die Monosäure wird mit Essigester ausgeschüttelt, die Essigesterlösung über Natriumsulfat getrocknet, filtriert und eingedampft. Der feste Rückstand wird im Vakuum bei 50°C getrocknet. Man erhält 14.93g (46.92 mmol) = 63.4% d. Th. als teils kristallinen, teils amorphen Feststoff.

| Analyse : C₁₇H₁₅FO₅ MG 318.3 | | | | | | | | |
|---|---|---|---|---|---|---|---|---|
| berechnet | C | 64.14 | H | 4.75 | F | 5.96 | O | 25.13% |
| gefunden | | 64.35 | | 4.51 | | 6.13 | | % |

d) 2-Benzyloxy-5-fluor-3-(2,2,2-trifluorethylcarbamoyl)-benzoesäure-ethylester
7.96g (25 mmol) 2-Benzyloxy-5-fluor-isophthalsäure-monoethylester werden in 50ml trockenem Essigester gelöst, bzw. suspendiert, 3.57g (30 mmol) Thionylchlorid hinzugefügt und 1 Stunde bei 50°C gerührt. Die Reaktionslösung wird dann unter vermindertem Druck zur Trockne eingedampft, der Rückstand zweimal mit je 50ml Dichlormethan abgedampft und in 30ml trockenem Tetrahydrofuran gelöst. Diese Lösung wird mit 3.03g (30 mmol) Triethylamin versetzt und anschließend eine Lösung von 2.97g (30 mmol) 2,2,2-Trifluorethylamin in 10ml trockenem Tetrahydrofuran eingetropft. Man laßt 5 Stunden bei Raumtemperatur nachrühren, dampft die Reaktionslösung unter vermindertem Druck zum Öl ein, löst dieses in 80ml Essigester und schüttelt zweimal mit je 10ml 1N Salzsäure und einmal mit 10ml Wasser aus. Die organische Phase wird über Magnesiumsulfat getrocknet, filtriert, eingedampft und der Rückstand an Kieselgel 60 (Merck) mit Essigester/Hexan chromatografiert. Man erhält 7.25g (18.15 mmol)= 72.6% d. Th. der Verbindung als amorphen Feststoff.

| Analyse : C₁₉H₁₇F₄NO₄ MG 399.34 | | | | | | | | | | |
|---|---|---|---|---|---|---|---|---|---|---|
| berechnet | C | 57.14 | H | 4.29 | F | 19.03 | N | 3.5 | O | 16.02% |
| gefunden | | 57.37 | | 4.53 | | 18.87 | | 3.61 | | % |

e) 2-Benzyloxy-5-fluor-3-(2,2,2-trifluorethylcarbamoyl)-benzoesäure
10.78g (27 mmol) 2-Benzyloxy-5-fluor-3-(2,2,2-trifluorethylcarbamoyl)-benzoesäure-ethylester werden in 60ml Dioxan gelöst, die Lösung mit 17.5ml (35 mmol) 2N Natronlauge versetzt, 1 Stunde bei Raumtemperatur und 1 Stunde bei 40°C gerührt. Das Reaktionsgemisch wird unter vermindertem Druck eingedampft, der Rückstand in 80ml Wasser aufgenommen, mit konzentrierter Salzsäure auf pH 1 angesäuert. Die Säure fällt dabei größtenteils als amorpher Feststoff aus. Er wird abgesaugt und mit wenig Wasser gewaschen. Die wässrige Phase wird mehrfach mit Essigester ausgeschüttelt. Der Essigesterextrakt wird eingedampft und der Rückstand mit der Fällung aus Wasser vereinigt. Man trocknet 48 Stunden bei 50°C im Vakuum. Ausbeute: 8.38g (22.57 mmol) = 83.6% d. Th. als amorpher Feststoff.

| Analyse : C₁₇H₁₃F₄NO₄ MG 371.29 | | | | | | | | | | |
|---|---|---|---|---|---|---|---|---|---|---|
| berechnet | C | 54.99 | H | 3.52 | F | 20.46 | N | 3.77 | O | 17.23% |
| gefunden | | 55.22 | | 3.78 | | 20.27 | | 3.58 | | % |

f) 5-Fluor-2-hydroxy-3-(2,2,2-trifluorethylcarbamoyl)-benzoesäure
5.2g (14 mmol) 2-Benzyloxy-5-fluor-3-(2,2,2-trifluorethylcarbamoyl)-benzoesäure werden in 30ml Ethanol gelöst, 250mg Palladium-Kohle (10%-ig) hinzugefügt und 1 Stunde bei Normaltemperatur hydriert. Der Katalysator wird anschließend abfiltriert und das Filtrat im Vakuum zur Trockne eingedampft. Der Rückstand wird an Kieselgel 60 (Merck) mit Dichlormethan/Methanol chromatografiert. Man erhält 3.03g (11.34 mmol) = 81% d. Th. der Verbindung als amorphen Feststoff.

| Analyse : C₁₀H₇F₄O₄ MG 267.16 | | | | | | | | |
|---|---|---|---|---|---|---|---|---|
| berechnet | C | 44.95 | H | 2.64 | F | 28.44 | O | 23.95% |
| gefunden | | 44.76 | | 2.83 | | 28.21 | | % |

### Beispiel 30

### 5-Fluor-2-hydroxy-3-(2,2,2-trifluorethylcarbamoyl)-benzoesäure-(2,3,4-trihydroxy-butyl)-amid

a) 2-Benzyloxy-5-fluor-3-(2,2,2-trifluorethylcarbamoyl)-benzoesäure-[2-hydroxy-2-(2,2-dimethyl-1,3-dioxolan-4-yl)-ethylamid]
4.46g (12 mmol) 2-Benzyloxy-5-fluor-3-(2,2,2-trifluorethylcarbamoyl)-benzoesäure werden unter Feuchtigkeitsausschluß in 30ml trockenem N,N-Dimethylformamid gelöst, 1.85g (12 mmol) Hydroxybenztriazol, 2.43g (15 mmol) 2-Hydroxy-2-(2,2-dimethyl-1,3-dioxolan-4-yl)-ethylamin hinzugefügt und die Lösung auf -10°C gekühlt. Zur gekühlten Lösung werden protionsweise 2.68g (13 mmol) Dicyclohexylcarbodiimid hinzugegeben, 1 Stunde bei -10°C und 6 Stunden bei Raumtemperatur gerührt. Der Niederschlag des Dicyclohexylharnstoffs wird dann abgesaugt, das Filtrat im Vakuum zur Trockne eingedampft und der Rückstand in ca. 100ml Essigester gelöst. Die Lösung wird mit gesättigter Bicarbonatlösung und mit Wasser ausgeschüttelt, über Natriumsulfat getrocknet, filtriert und eingeengt und an Kieselgel 60 (Merck) mit Essigester/Hexan chromatografiert. Man erhält 4.22g (8.2 mmol) = 68.3% d. Th. der Verbindung als amorphen Feststoff.

| Analyse : C₂₄H₂₆F₄N₂O₆ MG 514.47 | | | | | | | | | | |
|---|---|---|---|---|---|---|---|---|---|---|
| berechnet | C | 56.03 | H | 5.09 | F | 14.77 | N | 5.44 | O | 18.65% |
| gefunden | | 55.81 | | 4.9 | | 14.92 | | 5.23 | | % |

b) 5-Fluor-2-hydroxy-3-(2,2,2-trifluorethylcarbamoyl)-benzoesäure-(2,3,4-trihydroxy-butyl)-amid
6.95g (13.5 mmol) 2-Benzyloxy-5-fluor-3-(2,2,2-trifluorethylcarbamoyl)-benzoesäure-[2-hydroxy-2-(2,2-dimethyl-1,3-dioxolan-4-yl)-ethylamid] werden in 20ml Methanol gelöst, 5ml Wasser und 5ml 1N Salzsäure hinzugefügt und 3 Stunden bei Raumtemperatur gerührt. Die Lösung wird dann durch Zugabe von Anionenaustauscher Amberlite IRA 67 auf pH 6.3 gestellt, 200mg Palladium-Kohle (10%-ig) hinzugefügt und 1 Stunde bei Normaldruck hydriert. Der Katalysator wird anschließend abfiltriert, das Filtrat im Vakuum zur Trockne eingedampft und der Rückstand an Kieselgel 60 (Merck) mit Dichlormethan/Methanol chromatografiert. Man erhält 3.76g (9.77 mmol) = 72.4% d. Th. der Verbindung als amorphen Feststoff.

| Analyse : C₁₄H₁₆F₄N₂O₆ MG 384.28 | | | | | | | | | | |
|---|---|---|---|---|---|---|---|---|---|---|
| berechnet | C | 43.75 | H | 4.19 | F | 19.77 | N | 7.29 | O | 24.98% |
| gefunden | | 43.96 | | 4.32 | | 19.58 | | 7.13 | | % |

### Beispiel 31

### 5-Fluor-2-hydroxy-3-(2,2,2-trifluorethylcarbamoyl)-benzoesäure-N-methyl-N-(2,3,4-trihydroxy-butyl)-amid

a) 2-Benzyloxy-5-fluor-3-(2,2,2-trifluorethylcarbamoyl)-benzoesäure-N-methyl-N-[2-hydroxy-2-(2,2-dimethyl-1,3-dioxolan-4-yl)-ethylamid]
2.97g (8 mmol) 2-Benzyloxy-5-fluor-3-(2,2,2-trifluorethylcarbamoyl)-benzoesäure werden unter Feuchtigkeitsausschluß in 20ml trockenem N,N-Dimethylformamid gelöst, 1.08g (8 mmol) Hydroxybenztriazol, 1.76g (10 mmol) N- Methyl-[2-Hydroxy-2-(2,2-dimethyl-1,3-dioxolan-4-yl)]- ethylamin hinzugefügt und die Lösung auf -10°C gekühlt. Zur gekühlten Lösung werden protionsweise 1.85g (9 mmol) Dicyclohexylcarbodiimid hinzugegeben, 1 Stunde bei -10°C und 5 Stunden bei Raumtemperatur gerührt. Der Niederschlag des Dicyclohexylharnstoffs wird dann abgesaugt, das Filtrat im Vakuum zur Trockne eingedampft und der Rückstand in ca. 100ml Essigester gelöst. Die Lösung wird mit gesättigter Bicarbonatlösung und mit Wasser ausgeschüttelt, über Natriumsulfat getrocknet, filtriert, eingeengt und an Kieselgel 60 (Merck) mit Essigester/Hexan chromatografiert. Man erhält 2.69g (5.09 mmol) = 63.6% d. Th. der Verbindung als amorphen Feststoff

| Analyse : C₂₅H₂₈F₄N₂O₆ MG 528.5 | | | | | | | | | | |
|---|---|---|---|---|---|---|---|---|---|---|
| berechnet | C | 56.81 | H | 5.34 | F | 14.37 | N | 5.3 | O | 18.16% |
| gefunden | | 56.64 | | 5.47 | | 14.51 | | 5.43 | | % |

b) 5-Fluor-2-hydroxy-3-(2,2,2-trifluorethylcarbamoyl)-benzoesäure-N-methyl-N-(2,3,4-trihydroxy-butyl)-amid
3.86g (7.3 mmol) 2-Benzyloxy-5-fluor-3-(2,2,2-trifluorethylcarbamoyl)-benzoesäure-N-methyl-N-[2-hydroxy-2-(2,2-dimethyl-1,3-dioxolan-4-yl)-ethylamid] werden in 10ml Methanol gelöst, 10m Wasser und 200mg Kationenaustauscher Amberlyst 15 hinzugefügt und 2 Stunden am Rückfluß gekocht. Der Ionenaustauscher wird dann abfiltriert, dem Filtrat 150mg Palladium-Kohle (10%-ig) zugefügt und 1 Stunde bei Normaldruck hydriert. Der Katalysator wird danach abfiltriert, das Filtrat im Vakuum zur Trockne eingedampft und der Rückstand an Kieselgel 60 (Merck) mit Dichlormethan/Methanol chromatografiert. Man erhält 2.16g (5.42 mmol) = 74.3% d. Th. der Verbindung als amorphen Feststoff.

| Analyse : C₁₅H₁₈F₄N₂O₆ MG 398.31 | | | | | | | | | | |
|---|---|---|---|---|---|---|---|---|---|---|
| berechnet | C | 45.23 | H | 4.55 | F | 19.07 | N | 7.03 | O | 24.1% |
| gefunden | | 45.47 | | 4.4 | | 18.91 | | 6.87 | | % |

### Beispiel 32

### 5-Fluor-2-hydroxy-3-(trifluormethyl)-benzoesäure

a) 2-Brom-4-fluor-6-(trifluormethyl)-phenol
9g (50 mmol) 4-Fluor-2-(trifluormethyl)-phenol werden in 100ml Dichlorethan gelöst, die Lösung bei 50°C gerührt und eine Lösung von 9.6g (60 mmol) Brom in 30ml Dichlorethan im Verlaufe von 2 Stunden zugetropft. Nach beendetem Zutropfen wird 1 Stunde bei 50°C nachgerührt und dann im Vakuum eingedampft. Der ölige Rückstand wird in 100ml Dichlorethan gelöst und dreimal mit je 20ml Wasser ausgeschüttelt. Die organische Phase wird über Magnesiumsulfat getrocknet, filtriert, eingedampft und der Rückstand an Kieselgel 60 mit Essigester/Hexan chromatografiert. Man erhält 8.07g (31.15 mmol) = 62.3% d. Th. als teilweise kristallinen Feststoff.

| Analyse : C₇H₃BrF₄O MG 259.0 | | | | | | | | | | |
|---|---|---|---|---|---|---|---|---|---|---|
| berechnet | C | 32.46 | H | 1.16 | Br | 30.85 | F | 29.34 | O | 6.17% |
| gefunden | | 32.23 | | 1.05 | | 31.03 | | 29.11 | | % |

b) [2-Brom-4-Fluor-6-(trifluormethyl)-phenyl]-benzylether
0.65g (28 mmol) Natrium werden in 35ml trockenem Ethanol gelöst, 5.96g (23 mmol) 2-Brom-4-fluor-6-(trifluormethyl)-phenol hinzugefügt, 20 Minuten bei Raumtemperatur gerührt, 7.17g (42 mmol) Benzylbromid zugegeben und 1 Stunde am Rückfluß gekocht. Aus der Lösung fällt Natriumbromid kristallin aus. Die warme Lösung wird filtriert, das Filtrat zur Trockne eingedampft und der Rückstand an Kieselgel 60 mit Dichlormethan/Hexan chromatografiert. Man erhält 6.29g (18 mmol) = 78.3% d. Th. als überwiegend kristallinen Feststoff.

| Analyse : C₁₄H₉BrF₄O MG 349.13 | | | | | | | | | | |
|---|---|---|---|---|---|---|---|---|---|---|
| berechnet | C | 48.16 | H | 2.59 | Br | 22.88 | F | 21.76 | O | 4.58% |
| gefunden | | 48.33 | | 2.73 | | 22.67 | | 21.59 | | % |

c) 2-Benzyloxy-5-fluor-3-(trifluormethyl)-benzoesäureethylester
6.11g (17.5 mmol) [2-Brom-4-Fluor-6-(trifluormethyl)-phenyl]-benzylether werden in einer Argonatmosphäre unter Feuchtigkeitsausschluß in 60ml trockenem Tetrahydrofuran gelöst und die Lösung auf -100°C gekühlt. Bei dieser Temperatur werden 65.63ml (52.5 mmol) einer 0.8-molaren Lösung von Butyllithium in Hexan zugetropft. Nach beendeter Zugabe rührt man 5 Minuten nach und tropft dann eine Lösung von 3.54g (30 mmol) Diethylcarbonat in 20ml trockenem Tetrahydrofuran ein. Die Temperatur wird dabei unter -40°C gehalten. Anschließend rührt man nach, bis das Reaktionsgemisch Raumtemperatur erreicht hat und weitere 3 Stunden bei dieser Temperatur. Das Reaktionsgemisch wird unter vermindertem Druck eingedampft, der Rückstand in Essigester gelöst und die Lösung mehrmals mit Wasser ausgeschüttelt. Die organische Phase wird über Magnesiumsulfat getrocknet, im Vakuum eingedampft und der Rückstand an Kieselgel 60 (Merck) mit Essigester/Hexan chromatografiert. Man erhält 5.43g (10.26 mmol) 58.6% d. Th. der Verbindung als amorpen Feststoff.

| Analyse : C₁₇H₁₄F₄O₃ MG 342.29 | | | | | | | | |
|---|---|---|---|---|---|---|---|---|
| berechnet | C | 59.65 | H | 4.12 | F | 22.2 | O | 14.02% |
| gefunden | | 59.83 | | 4.27 | | 21.93 | | % |

d) 5-Fluor-2-hydroxy-3-(trifluormethyl)-benzoesäure
4.28g (12.3 mmol) 2-Benzyloxy-5-fluor-3-(trifluormethyl)-benzoesäureethylester werden in 20ml Dioxan gelöst, 7.5ml (15 mmol) 2N Natronlauge hinzugefügt und 1 Stunde bei 50°C gerührt. Die Reaktionslösung wird dann mit 200mg Palladium-Kohle (10%-ig) versetzt und 1 Stunde bei Normaltemperatur hydriert. Der Katalysator wird anschließend abfiltriert, das alkalische Filtrat mit 30ml Wasser versetzt und mit Dichlormethan extrahiert. Die Wasserphase wird mit konzentrierter Salzsäure auf pH1 angesäuert und mit Essigester extrahiert. Der Essigesterextrakt wird über Magnesiumsulfat getrocknet, filtriert und zur Trockne eingedampft. Der kristalline Rückstand wird im Vakuum bei 50°C 24 Stunden getrocknet. Man erhält 2.27g (10.15 mmol) 82.5% d. Th. Schmelzbereich 76-83°C.

| Analyse : C₈H₄F₄O₃ MG 224.11 | | | | | | | | |
|---|---|---|---|---|---|---|---|---|
| berechnet | C | 42.87 | H | 1.79 | F | 33.9 | O | 21.41% |
| gefunden | | 42.66 | | 1.93 | | 33.76 | | % |

### Beispiel 33

### 5-Fluor-2-hydroxy-3-(trifluormethyl)-benzoesäure-(2,3,4-trihydroxy-butyl)-amid

a) 2-Benzyloxy-5-fluor-(trifluormethyl)-benzoesäure-[2-hydroxy-2-(2,2-dimethyl-1,3-dioxolan-4-yl)-ethylamid]
3.42g (10 mmol) 2-Benzyloxy-5-fluor-3-(trifluormethyl)-benzoesäureethylester werden in 10ml Ethanol gelöst, 2.43g (15 mmol) 2-Hydroxy-2-(2,2-dimethyl-1,3-dioxolan-4-yl)-ethylamin hinzugefügt, das Ethanol abdestilliert und das zurückbleibende Öl 2 Stunden im Vakuum auf 100°C erwärmt. Das Reaktionsgemisch wird dann in 50ml Essigester gelöst, zweimal mit je 10ml 1N Salzsäure ausgeschüttelt, die organische Phase über Natriumsulfat getrocknet, eingedampft und der Rückstand an Kieselgel 60 mit Essigester/Hexan chromatografiert. Man erhält 2.18g (7.18 mmol) = 47.6.8% d. Th. als amorphen Feststoff.

| Analyse : C₂₂H₂₃F₄NO₅ MG 457.42 | | | | | | | | | | |
|---|---|---|---|---|---|---|---|---|---|---|
| berechnet | C | 57.76 | H | 5.06 | F | 16.61 | N | 3.06 | O | 17.48% |
| gefunden | | 57.92 | | 5.23 | | 16.45 | | 2.87 | | % |

b) 5-Fluor-2-hydroxy-3-(trifluormethyl)-benzoesäure-(2,3,4-trihydroxy-butyl)-amid
6.86g (15 mmol) 5-Fluor-2-hydroxy-3-(trifluormethyl)-benzoesäure[2-hydroxy-2-(2,2-dimethyl-1,3-dioxolan-4-yl)-ethylamid] werden in 30ml Methanol gelöst, 30ml Wasser und 300mg Kationenaustauscher Amberlyst 15 hinzugefügt und 2 Stunden am Rückfluß gekocht. Der Ionenaustauscher wird dann abfiltriert, dem Filtrat 250mg Palladium-Kohle (10%-ig) zugesetzt und 1 Stunde bei Normaldruck hydriert. Der Katalysator wird danach abfiltriert, das Filtrat im Vakuum zur Trockne eingedampft und der Rückstand an Kieselgel 60 (Merck) mit Dichlormethan/Methanol chromatografiert. Man erhält 3.08g (9.41 mmol) = 62.7% d. Th. der Verbindung als amorphen Feststoff.

| Analyse : C₁₂H₁₃F₄NO₅ MG 327.23 | | | | | | | | | | |
|---|---|---|---|---|---|---|---|---|---|---|
| berechnet | C | 44.04 | H | 4.0 | F | 23.22 | N | 4.28 | O | 24.44% |
| gefunden | | 43.88 | | 4.16 | | 22.95 | | 4.43 | | % |

### Beispiel 34

### 5-Fluor-2-hydroxy-3-(trifluormethyl)-benzoesäure-N-methyl-N-(2,3,4-trihydroxy-butyl)-amid

a) 2-Benzyloxy-5-fluor-3-(trifluormethyl)-benzoesäure-N-methyl-N-[2-hydroxy-2-(2,2-dimethyl-1,3-dioxolan-4-yl)-ethyl]-amid
4.66g (13.6 mmol) 2-Benzyloxy-5-fluor-3-(trifluormethyl)-benzoesäureethylester werden in 15ml Ethanol gelöst, 3.59g (20.4 mmol) N-Methyl-N-[2-hydroxy-2-(2,2-dimethyl-1,3-dioxolan-4-yl)]-ethylamin hinzugefügt, das Ethanol abdestilliert und das zurückbleibende Öl 2 Stunden im Vakuum auf 100°C erwärmt. Das Reaktionsgemisch wird dann in 80ml Essigester gelöst, zweimal mit je 10ml 1N Salzsäure ausgeschüttelt, die organische Phase über Magnesiumsulfat getrocknet, eingedampft und der Rückstand an Kieselgel 60 mit Essigester/Hexan chromatografiert. Man erhält 3.31g (7.03 mmol) = 51.7% d. Th. als amorphen Feststoff.

| Analyse : C₂₃H₂₅F₄NO₅ MG 471.45 | | | | | | | | | | |
|---|---|---|---|---|---|---|---|---|---|---|
| berechnet | C | 58.59 | H | 5.34 | F | 16.11 | N | 2.97 | O | 16.96% |
| gefunden | | 58.47 | | 5.51 | | 15.92 | | 3.11 | | % |

b) 5-Fluor-2-hydroxy-3-(trifluormethyl)-benzoesäure-N-methyl-N-(2,3,4-trihydroxy-butyl)-amid
8.01g (17 mmol) 5-Fluor-2-hydroxy-3-(trifluormethyl)-benzoesäure-N-methyl-N-[2-hydroxy-2-(2,2-dimethyl-1,3-dioxolan-4-yl)-ethyl]-amid werden in 35ml Methanol gelöst, 35ml Wasser und 350 mg Kationenaustauscher Amberlyst 15 hinzugefügt und 2 Stunden am Rückfluß gekocht. Der Ionenaustauscher wird dann abfiltriert, dem Filtrat 350mg Palladium-Kohle (10%-ig) zugesetzt und 1 Stunde bei Normaldruck hydriert. Der Katalysator wird anschließend abfiltriert, das Filtrat im Vakuum zur Trockne eingedampft und der Rückstand an Kieselgel 60 (Merck) mit Dichlormethan/Methanol chromatografiert. Man erhält 3.9g (11.44 mmol) = 67.3% d. Th. der Verbindung als amorphen Feststoff.

| Analyse : C₁₃H₁₅F₄NO₅ MG 341.26 | | | | | | | | | | |
|---|---|---|---|---|---|---|---|---|---|---|
| berechnet | C | 45.75 | H | 4.43 | F | 22.26 | N | 4.1 | O | 23.44% |
| gefunden | | 46.03 | | 4.57 | | 21.98 | | 4.22 | | % |

### Beispiel 35

### Hexafluorglutarsäure-bis[(5-fluor-2-hydroxy-3-hydroxycarbonyl)-anilid]

a) Hexafluorglutarsäure-bis[(2-hydroxy-3-hydroxycarbonyl-5-nitro)-anilid]
9.91g (50 mmol) 3-Amino-2-hydroxy-5-nitro-benzoesäure (J. Chem. Soc.111, 540 [1917]) werden in 30ml trockenem Dimethylformamid gelöst, 10.12g (0.1mol) Triethylamin hinzugefügt, die Lösung auf 0°C gekühlt und bei dieser Temperatur 6.9g (25 mmol) Hexafluorglutarsäuredichlorid eingetropft. Es wird 30 Minuten bei 0°C und 3 Stunden bei Raumtemperatur nachgerührt. Der Niederschlag von Triethylaminhydrochlorid wird abgesaugt und da Filtrat in 300ml Dichlormethan gefällt. Die Fällung wird abgesaugt, in Methanol gelöst und an Kieselgel 60 mit Dichlormethan/Methanol chromatografiert. Man erhält 13.09g (21.8 mmol) = 43.6% d. Th. der Verbindung als teils amorphen, teils kristallinen Feststoff.

| Analyse : C₁₉H₁₀F₆N₄O₁₂ MG 600.3 | | | | | | | | | | |
|---|---|---|---|---|---|---|---|---|---|---|
| berechnet | C | 38.01 | H | 1.67 | F | 18.98 | N | 9.98 | O | 31.98% |
| gefunden | | 37.8 | | 1.83 | | 19.2 | | 9.75 | | % |

b) Hexafluorglutarsäure-bis[(2-hydroxy-3-ethoxycarbonyl-5-nitro)-anilid]
12g (20 mmol) Hexafluorglutarsäure-bis[(2-hydroxy-3-hydroxycarbonyl-5-nitro)-anilid] werden in 120ml trockenem Ethanol gelöst, 2ml konzentrierte Schwefelsäure hinzugefügt und die Lösung 6 Stunden am Rückfluß gekocht. Die Schwefelsäure wird durch Zugabe von festem Natriumhydrogencarbonat abgepuffert, bis die überstehende Lösung auf wasserfeuchtem pH-Papier pH 8 anzeigt. Der Feststoff wird abgesaugt und das Filtrat zur Trockne eingedampft. Der Rückstand wird an Kiesegel 60 mit Hexan/Essigester chromatografiert. Man erhält 7.04g (10.72 mmol) = 53.6% d. Th. als amorphen Feststoff.

| Analyse : C₂₃H₁₈F₆N₄O₁₂ MG 656.4 | | | | | | | | | | |
|---|---|---|---|---|---|---|---|---|---|---|
| berechnet | C | 42.08 | H | 2.76 | F | 17.36 | N | 8.53 | O | 29.24% |
| gefunden | | 42.25 | | 2.93 | | 17.13 | | 8.38 | | % |

c) Hexafluorglutarsäure-bis[(2-benzyloxy-3-ethoxycarbonyl-5-nitro)-anilid]
1.5g (65 mmol) Natrium werden in einer Stickstoffatmosphäre unter Feuchtigkeitsausschluß in 100ml trockenem Ethanol gelöst. Zu dieser Lösung werden 17.7g (27 mmol) Hexafluorglutarsäure-bis[(2-hydroxy-3-ethoxycarbonyl-5-nitro)-anilid] hinzugefügt 15 Minuten bei Raumtemperatur gerührt, 11.12g (65 mmol) Benzylbromid hinzugefügt und 4 Stunden am Rückfluß gekocht. Die Reaktionslösung wird dann zur Trockne eingedampft, der Rückstand in Essigester aufgenommen, filtriert und das Filtrat an Kieselgel 60 mit Hexan/Essigester chromatografiert. Man erhält 10.73g (12.83 mmol) = 47.5% d. Th. der Verbindung als amorphen Feststoff.

| Analyse : C₃₇H₃₀F₆N₄O₁₂ MG 836.65 | | | | | | | | | | |
|---|---|---|---|---|---|---|---|---|---|---|
| berechnet | C | 53.11 | H | 3.61 | F | 13.62 | N | 6.69 | O | 22.94% |
| gefunden | | 53.32 | | 3.77 | | 13.48 | | 6.5 | | % |

d) Hexafluorglutarsäure-bis[(5-amino-2-benzyloxy-3-ethoxycarbonyl)-anilid]
8.37g (10 mmol) Hexafluorglutarsäure-bis[(2-benzyloxy-3-ethoxycarbonyl-5-nitro)-anilid] werden in 84ml Ethanol gelöst, 11.3g (50 mmol) Zinn-II-chlorid * H₂O hinzugefügt und die Suspension bei 70°C Badtemperatur 4 Stunden gerührt. Das Reaktionsgemisch wird zu einem Öl eingedampft und dieses an Kieselgel 60 mit Dichlormethan/Essigester chromatografiert. Man erhält 5.64g (7.26 mmol) = 72.6% der Verbindung als amorphen Feststoff.

| Analyse : C₃₇H₃₄F₆N₄O₈ MG 776.69 | | | | | | | | | | |
|---|---|---|---|---|---|---|---|---|---|---|
| berechnet | C | 57.21 | H | 4.41 | F | 14.67 | N | 7.21 | O | 16.47% |
| gefunden | | 57.43 | | 4.6 | | 14.43 | | 6.96 | | % |

e) Hexafluorglutarsäure-bis[(2-benzyloxy-3-ethoxycarbonyl-5-fluor)-anilid]
9.32g (12 mmol) Hexafluorglutarsäure-bis[(5-amino-2-benzyloxy-3-ethoxycarbonyl)-anilid] werden in einem Gemisch aus 9.6ml (60 mmol) 42%-iger Tetrafluorborsäure und 10ml Wasser suspendiert. Zu dieser Suspension tropft man bei 10-15°C Innentemperatur eine Losung von 2.07g (30 mmol) Natriumnitrit in 10ml Wasser. Man rührt 30 Minuten bei 0-5°C nach, saugt den Niederschlag ab, wäscht ihn mit 20ml kalter 5%-iger Tetrafluorborsäure, 20ml kaltem Ethanol und 50ml kaltem Ether. Das Salz wird dann in 100ml m-Xylol suspendiert und die Suspension im Verlaufe von ca. 2 Stunden allmählich zum Sieden erhitzt. Das Xylol wird unter vermindertem Druck abdestilliert. Der Rückstand wird in 50ml Ethanol aufgenommen, 1.5ml konzentrierte Schwefelsäure zugegeben und 3 Stunden am Rückfluß gekocht. Man kühlt auf Raumtemperatur, fügt Natriumhydrogencarbonat hinzu, bis der Überstand neutrales pH anzeigt und filtriert den Niederschlag ab. Das Filtrat wird weitgehend eingedampft und der Rückstand an Kieselgel 60 mit Dichlormethan/Essigester chromatografiert. Man erhält 3.34g (4.27 mmol) = 35.6% d. Th. der Verbindung als amorphen Feststoff.

| Analyse : C₃₇H₃₀F₈N₂O₈ MG 782.64 | | | | | | | | | | |
|---|---|---|---|---|---|---|---|---|---|---|
| berechnet | C | 56.78 | H | 3.86 | F | 19.42 | N | 3.57 | O | 16.35% |
| gefunden | | 56.62 | | 4.03 | | 19.23 | | 3.43 | | % |

f) Hexafluorglutarsäure-bis[(5-fluor-2-hydroxy-3-hydroxycarbonyl)-anilid]
5.87g (7.5 mmol) Hexafluorglutarsäure-bis[(2-benzyloxy-3-ethoxycarbonyl-5-fluor) -anilid] werden in 25ml Dioxan gelöst, 150mg Pd-Kohle (10%-ig) hinzugefügt und 2 Stunden bei Normaldruck hydriert. Der Katalysator wird abgesaugt und das Filtrat mit 15ml 2N Natronlauge 2 Stunden bei 50°C gerührt. Die Lösung wird mit konzentrierter Salzsäure auf ca. pH 8 gestellt und im Vakuum weitgehend eingedampft. Der ölige Rückstand wird in Wasser gelöst und auf pH 1 angesäuert. Das Produkt fällt größtenteils als amorpher Niederschlag aus. Die Wasserphase wird mit Chloroform ausgeschüttelt. Niederschlag und Chloroformextrakt werden vereinigt, eingedampft und der Rückstand an Kieselgel 60 mit Dichlormethan/Aceton chromatografiert. Man erhält (4.76 mmol) = 63.4% d. Th. als amorphen Feststoff.

| Analyse : C₁₉H₁₀F₈N₂O₈ MG 546.28 | | | | | | | | | | |
|---|---|---|---|---|---|---|---|---|---|---|
| berechnet | C | 41.77 | H | 1.84 | F | 27.82 | N | 5.12 | O | 23.43% |
| gefunden | | 41.96 | | 2.04 | | 27.66 | | 4.93 | | % |

### Beispiel 36

### Hexafluorglutarsäure-bis{[5-fluor-2-hydroxy-3-(2,3,4-trihydroxybutylcarbamoyl)]-anilid}

5.0g (6.4 mmol) Hexafluorglutarsäure-bis[(5-amino-2-benzyloxy-3-ethoxycarbonyl)-anilid] werden in 15ml Ethanol gelöst, 3.24 g (20 mmol) 2-Hydroxy-2-(2,2-dimethyl-1,3-dioxolan-4-yl)-ethylamin hinzugefügt, das Ethanol abdestilliert und das verbleibende Öl 3 Stunden im Vakuum bei 100-110°C erwärmt. Das Reaktionsgemisch wird anschließend auf Raumtemperatur gekühlt, in Aceton aufgenommen und an Kieselgel 60 mit Hexan/Aceton chromatografiert. Die zusammengefaßten Fraktionen des intermediären Produktes mit den Benzyl- und Ketalschutzgruppen werden eingedampft und der Rückstand in 50 Methanol gelöst. Dieser Lösung werden 200mg Pd-Kohle (10%-ig) zugefügt und 2 Stunden bei Normaldruck hydriert. Der Katalsator wird abfiltriert, dem Filtrat 30ml Wasser und 500mg Kationenaustauscher Amberlyst 15 zugesetzt und 3 Stunden bei 60°C gerührt. Der Ionenaustauscher wird dann abfiltriert, das Filtrat im Vakuum zur Trockne eingedampft und der Rückstand an Kieselgel mit Dichlormethan/Methanol chromatografiert. Man erhält 2g (2.66 mmol) = 41.5% d. Th. als amorphen Feststoff.

| Analyse : C₂₇H₂₈F₈N₄O₁₂ MG 752.52 | | | | | | | | | | |
|---|---|---|---|---|---|---|---|---|---|---|
| berechnet | C | 43.29 | H | 3.75 | F | 20.19 | N | 7.44 | O | 25.51% |
| gefunden | | 43.42 | | 3.9 | | 19.93 | | 7.27 | | % |

### Beispiel 37

### 5-Fluor-3-hydroxyacetamido-2-hydroxy-benzoesäure-(2,2,2-trifluor-ethyl)amid

a) 5-Fluor-3-nitro-salicylsäure
6,24 g (40 mmol) 5-Fluor-salicylsäure werden unter Erwärmen in 62,4 ml Acetonitril gelöst. die Lösung auf 0 °C gekühlt und unter Beibehaltung dieser Temperatur eine Lösung von 2,96 ml (44 mmol) 65 %iger HNO₃ in 14,8 ml Acetonitril zugetropft. Das Reaktionsgemisch wird 24 Stunden bei 0 °C gerührt, dann über 7,5 g Kieselgel 60 filtriert und zur Trockne eingedampft. Man erhält 6,1 g = 75,8 % d. Th. Rohprodukt, das ohne Reinigung in die nächste Reaktion eingesetzt wird.
b) 5-Fluor-2-hydroxy-3-nitro-benzoesäure-(2,2,2-trifluor-ethyl)amid
5,4 g (26,85 mmol) 5-Fluor-2-hydroxy-3-nitro-benzoesäure werden in 27 ml Thionylchlorid suspendiert und bei 50 °C gerührt. Es entsteht eine nahezu klare Lösung. Nach 1 Stunde wird die Lösung im Vakuum zur Trockne eingedampft, der Rückstand in 27 ml THF gelöst und 27 ml 2,2,2-Trifluorethylamin hinzugefügt. Die Lösung wird 15 Stunden bei Raumtemperatur gerührt, dann filtriert, das Filtrat eingedampft und an Kieselgel 60 mit Methylenchlorid/Essigester chromatographiert. Man erhält 3,51 g = 46,33 % d. Th. als amorphen Feststoff. Dieser ist aus Dioxan kristallisierbar.

| Analyse: C₉H₆F₄N₂O₄ MG 282,15 | | | | | | | | | | |
|---|---|---|---|---|---|---|---|---|---|---|
| berechnet | C | 38,31 | H | 2,14 | F | 26,93 | N | 9,92 | O | 22,68 % |
| gefunden | | 38,05 | | 2,32 | | 26,65 | | 9,63 | | |

c) 3-Amino-5-fluor-2-hydroxy-benzoesäure-(2,2,2-trifluor-ethyl)amid
7,05 g (25 mmol) 5-Fluor-2-hydroxy-3-nitro-benzoesäure-(2,2,2-trifluor-ethyl)amid werden in 70 ml Ethanol gelöst, 5,65 (25 mmol) SnCl₂.H₂O hinzugefügt und die Suspension 2 Stunden bei 70 °C gerührt. Das Reaktionsgemisch wird dann zu einem Öl eingedampft und ohne Reinigung in die nächste Reaktion eingesetzt.
d) 3-Acetoxyacetamido-5-fluor-2-hydroxy-benzoesäure-(2,2,2-trifluorethyl)amid
6,3 g (25 mmol) rohes 3-Amino-5-fluor-2-hydroxy-benzoesäure-(2,2,2-trifluor-ethyl)amid werden in 30 ml THF gelöst, 3,04 g (30 mmol) Triethylamin sowie 4,1 g (30 mmol) Acetoxyacetylchlorid hinzugefügt und 12 Stunden bei Raumtemperatur gerührt. Die Reaktionslösung wird dann im Vakuum eingedampft und der Rückstand an Kieselgel 60 mit Essigester/Hexan chromatographiert. Man erhält 5,52 g = 62,7 % d. Th. als amorphen Feststoff.

| Analyse: C₁₃H₁₂F₄N₂O₅ MG 352,24 | | | | | | | | | | |
|---|---|---|---|---|---|---|---|---|---|---|
| berechnet | C | 44,32 | H | 3,43 | F | 21,57 | N | 7,95 | O | 22,71 % |
| gefunden | | 44,67 | | 3,52 | | 21,31 | | 7,78 | | |

e) 5-Fluor-3-hydroxyacetamido-2-hydroxy-benzoesäure-(2,2,2-trifluor-ethyl)amid
5,9 g (17 mmol) 3-Acetoxyacetamido-5-fluor-2-hydroxy-benzoesäure-(2,2,2-trifluor-ethyl)amid werden in 34 ml 1N NaOH gelöst und 30 Minuten bei 40 °C gerührt. Die Reaktionslösung wird dann auf Raumtemperatur gekühlt und über 200 ml Kationenaustauscher filtriert. Das Eluat wird im Vakuum zur Trockne eingedampft und der Rückstand an Kieselgel 60 mit Essigester/Aceton chromatographiert. Man erhält 3,3 g = 62,7 % d. Th. als amorphes Material.

| Analyse: C₁₁H₁₀F₄N₂O₄ | | | | | | | | | | |
|---|---|---|---|---|---|---|---|---|---|---|
| berechnet | C | 42,59 | H | 3,24 | F | 24,49 | N | 9,03 | O | 20,63 % |
| gefunden | | 42,83 | | 3,52 | | 24,21 | | 8,87 | | |

### Beispiel 38

### 5-Fluor-3-[N-(2,3-dihydroxy-propyl)-hydroxyacetamido-2-hydroxy-benzoesäure-(2,2,2-trifluor-ethyl)amid

a) 5-Fluor-3-acetoxyacetamido-2-benzyloxy-benzoesäure-(2,2,2-trifluor-ethyl)-amid
7,75 g (22 mmol) 3-Acetoxyacetamido-5-fluor-2-hydroxy-benzoesäure-(2,2,2-trifluor-ethyl)amid [Beispiel 37d)] werden in 50 ml trockenem THF gelöst, 1,35 g (25 mmol) Natriummethylat hinzugefügt, 15 Minuten bei Raumtemperatur gerührt, 4,27 g (25 mmol) Benzylbromid zugefügt und 1 Stunde am Rückfluß gekocht. Die Reaktionslösung wird dann auf Raumtemperatur gekühlt, NaBr abfiltriert, das Filtrat im Vakuum eingedampft und der Rückstand an Kieselgel 60 mit Essigester/Hexan chromatographiert. Man erhält 5,2 g = 53,4 % d. Th. als amorphen Feststoff.

| Analyse: C₂₀H₁₈F₄N₂O₅ MG 442,37 | | | | | | | | | | |
|---|---|---|---|---|---|---|---|---|---|---|
| berechnet | C | 54,3 | H | 4,1 | F | 17,17 | N | 6,33 | O | 18,08 % |
| gefunden | | 54,57 | | 4,32 | | 16,88 | | 6,17 | | |

b) 5-Fluor-3-[N-(2,2-dimethyl-1,3-dioxolan-4-ylmethyl)-acetoxyacetamido-2-benzyloxy-benzoesäure-(2,2,2-trifluor-ethyl)amid
6,64 g (15 mmol) 5-Fluor-3-acetoxyacetamido-2-benzyloxy-benzoesäure-(2,2,2-trifluor-ethyl)amid werden in 50 ml THF gelöst, 480 mg (20 mmol) NaH hinzugefügt, 30 Minuten bei Raumtemperatur gerührt, 3,01 g (20 mmol) 4-Chlormethyl-2,2-dimethyl-dioxolan hinzugefügt und 3 Stunden am Rückfluß gekocht. Die Reaktionslösung wird dann im Vakuum eingedampft und der Rückstand an Kieselgel 60 mit Essigester/Hexan chromatographiert. Man erhält 4,03 g = 48,3 % d. Th. als amorphen Feststoff.

| Analyse: C₂₆H₂₈F₄N₂O₇ MG 556,51 | | | | | | | | | | |
|---|---|---|---|---|---|---|---|---|---|---|
| berechnet | C | 56,11 | H | 5,07 | F | 13,65 | N | 5,07 | O | 20,12 % |
| gefunden | | 56,32 | | 5,32 | | 13,44 | | 4,93 | | |

c) 5-Fluor-3-[N-(2,3-dihydroxy-propyl)-hydroxyacetamido-2-hydroxy-benzoesäure-(2,2,2-trifluor-ethyl)amid
6,68 g (12 mmol) 5-Fluor-3-[N-(2,2-dimethyl-1,3-dioxolan-4-ylmethyl)-acetoxyacetamido-2-benzyloxy-benzoesäure-(2,2,2-trifluor-ethyl)amid werden in 40 ml Methanol gelost, 15 ml 1N methanolische NaOH zugefügt und 30 Minuten bei 40 °C gerührt. Die Lösung wird dann mit 40 ml Ionenaustauscher Amberlyst 15 3 Stunden bei Raumtemperatur gerührt, filtriert, das Filtrat im Vakuum eingedampft, der Rückstand in 40 ml Methanol gelöst, 200 mg Palladium-Kohle (10 %ig) zugefügt und 1 Stunde bei Normaldruck hydriert. Der Katalysator wird anschließend abgesaugt, das Filtrat zur Trockne eingedampft und der Rückstand an Kieselgel 60 mit Methylenchlorid/Methanol chromatographiert. Man erhält 2,6 g = 56,6 % d. Th. als amorphen Feststoff.

| Analyse: C₁₄H₁₆F₄N₂O₆ MG 384,28 | | | | | | | | | | |
|---|---|---|---|---|---|---|---|---|---|---|
| berechnet | C | 43,75 | H | 4,19 | F | 19,77 | N | 7,29 | O | 24,98 % |
| gefunden | | 43,92 | | 4,37 | | 19,53 | | 7,02 | | |

### Beispiel 39

### 5-Fluor-3-(D-gluconamido)-2-hydroxy-benzoesäure-(2,2,2-trifluorethyl)amid

a) 5-Fluor-3-(pentaacetoxy-D-gluconamido)-2-hydroxy-benzoesäure-(2,2,2-trifluor-ethyl)amid
4,29 g (17 mmol) 3-Amino-5-fluor-2-hydroxy-benzoesäure-(2,2,2-trifluor-ethyl)amid [Beispiel 37c)] werden in 40 ml THF gelöst, 2,03 g (20 mmol) Triethylamin und 8,5 g (20 mmol) Pentaacetoxy-D-gluconsäurechlorid hinzugefügt und 12 Stunden bei Raumtemperatur gerührt. Das Reaktionsgemisch wird dann im Vakuum eingedampft, der Rückstand in Essigester gelöst und mit Wasser ausgeschüttelt. Die organische Phase wird über MgSO₄ getrocknet, filtriert, eingedampft und der Rückstand an Kieselgel 60 mit Essigester/Hexan chromatographiert. Man erhalt 68,2 % d. Th. als amorphen Feststoff.

| Analyse: C₂₅H₂₈F₄N₂O₈ MG 560,5 | | | | | | | | | | |
|---|---|---|---|---|---|---|---|---|---|---|
| berechnet | C | 53,37 | H | 5,03 | F | 13,55 | N | 4,99 | O | 22,83 % |
| gefunden | | 53,58 | | 5,26 | | 13,37 | | 4,72 | | |

b) 5-Fluor-3-(gluconamido)-2-hydroxy-benzoesäure-(2,2,2-trifluor-ethyl)amid
(8.5 mmol) 5-Fluor-3-(pentaacetoxy-D-gluconamido)-2-hydroxy-benzoesäure-(2,2,2-trifluor-ethyl)amid werden in 30 ml Methanol gelöst, 10 ml 1N methanolische NaOH zugefügt und 1 Stunde bei 40 °C gerührt. Dann werden 30 ml Kationenaustauscher Amberlyst 15 zugefügt und 1 Stunde bei Raumtemperatur gerührt. Der Ionenaustauscher wird abfiltriert, das Filtrat zur Trockne eingedampft, der Rückstand mehrfach mit Methylenchlorid extrahiert und anschließend im Vakuum bei 50 °C getrocknet. Man erhält 76,3 % d. Th. als amorphen Feststoff.

| Analyse: C₁₅H₁₈F₄N₂O₈ MG 430,31 | | | | | | | | | | |
|---|---|---|---|---|---|---|---|---|---|---|
| berechnet | C | 41,86 | H | 4,21 | F | 17,66 | N | 6,51 | O | 29,74 % |
| gefunden | | 42,07 | | 4,42 | | 17,49 | | 5,32 | | |

### Beispiel 40

### 2-[N-(4-Fluor-3-(trifluormethylphenyl)sulfamoyl]-essigsäure-(2,3,4-trihydroxybutyl-amid

a) 2-[N-(4-Fluor-3-trifluormethylphenyl)sulfamoyl]-essigsäuremethylester
Analog der Vorschrift für Beispiel 7 werden 5 g (27,64 mmol) 4-Fluor-3-(trifluormethyl)-anilin in 25 ml trockenem Pyridin mit 4,82 g (27,4 mmol) Chlorsulfonylessigsäuremethylester in 100 ml trockenem Dichlorethan umgesetzt. Nach Kristallisation aus Ether/Hexan erhält man 7,35 g = 84,35 % d. Th.
Fp. 111 - 112 °C.
b) 2-[N-(4-Fluor-3-trifluormethylphenyl)sulfamoyl]-essigsäure
Analog der Vorschrift für Beispiel 8 werden 2,2 g (6,98 mmol) 2-[N-(4-Fluor-3-trifluormethylphenyl)sulfamoyl]-essigsäuremethylester in Dioxan mit wäßriger NaOH verseift. Nach Ansäuern, Extraktion und Kristallisation aus Essigester/Hexan erhält man 1,96 g = 93,2 % d. Th. Kristalle.
Fp. 138 - 140 °C.
c) 2-[N-(4-Fluor-3-trifluormethylphenyl)sulfamoyl]-essigsäure-[2-hydroxy-2-(2,2-dimethyl-1,3-dioxolan-4-yl)-ethyl-amid]
Analog der Vorschrift für Beispiel 9a) werden 2,21 g (7 mmol) 2-[N-(4-Fluor-3-trifluormethylphenyl)sulfamoyl]-essigsäure in Dioxan mit 1,13 g (7 mmol) 2-Hydroxy-2-(2,2-dimethyl-1,3-dioxolan-4-yl)-ethylamin ca. 1 Stunde bei 90 °C umgesetzt. Man erhält nach Eindampfen und Kristallisation aus Ethanol/Ether 2,87 g = 92,3 % d. Th. Kristallisat.
Fp. 144 - 146 °C.
d) 2-[N-(4-Fluor-3-(trifluormethylphenyl)sulfamoyl]-essigsäure-(2,3,4-trihydroxy-butyl-amid
Analog der Vorschrift für Beispiel 9b) worden 1,8 g (4,05 mmol) 2-[N-(4-Fluor-3-trifluormethylphenyl)sulfamoyl]-essigsäure-[2-hydroxy-2-(2,2-dimethyl-1,3-dioxolan-4-yl)-ethyl-amid] in 10 ml Trifluoressigsäure in die Titelverbindung überführt. Man erhält nach Kristallisation aus Ethanol/Diethylether 1,3 g = 79,4 % d. Th. hygroskopische Kristalle.

| Analyse: C₁₃H₁₆F₄N₂O₆S MG 404,45 | | | | | | | | | | | | |
|---|---|---|---|---|---|---|---|---|---|---|---|---|
| berechnet | C | 38,61 | H | 3,99 | F | 18,79 | N | 6,93 | O | 23,8 | S | 7,93 % |
| gefunden | | 38,83 | | 4,17 | | 18,93 | | 6,76 | | | | 7,67 |

### Beispiel 41

### 4-[N-(4-Fluor-3-trifluormethylphenyl)sulfamoyl]-benzoesäure-(2,3,4-trihydroxy-butyl-amid)

a) 4-[N-(4-Fluor-3-trifluormethylphenyl)sulfamoyl]-benzoesäure
Analog Beispiel 10 werden 2,21 g (10 mmol) 4-Chlorsulfonyl-benzoesäure mit 1,79 g (10 mmol) 4-Fluor-3-(trifluormethyl)-anilin in Pyridin umgesetzt und das Rohprodukt aus Ethanol kristallisiert. Man erhält 3,3 g = 90,84 mmol kristallines Produkt.
Fp. 238 - 241 °C.
b) 4-[N-(4-Fluor-3-trifluormethylphenyl)sulfamoyl]-benzoesäure-[2-hydroxy-2-(2,2-dimethyl-1,3-dioxolan-4-yl)-ethyl-amid]
Analog Beispiel 11a) werden 3,63 g (10 mmol) 4-[N-(4-Fluor-3-trifluormethylphenyl)sulfamoyl]benzoesäure in Essigester mit SOCl₂ in das Säurechlorid überführt und dieses mit 1,93 g (12 mmol) 2-Hydroxy-2-(2,2-dimethyl-1,3-dioxolan-2-yl)-ethylamin umgesetzt und das Rohprodukt durch Chromatographie gereinigt. Man erhält 4,2 g = 82,9 % d. Th. der Verbindung als amorphen Feststoff.

| Analyse: C₂₁H₂₂F₄N₂O₆S MG 506,47 | | | | | | | | | | | | |
|---|---|---|---|---|---|---|---|---|---|---|---|---|
| berechnet | C | 49,8 | H | 4,37 | F | 15,0 | N | 5,53 | O | 18,95 | S | 6,33 % |
| gefunden | | 49,62 | | 4,21 | | 15,13 | | 5,67 | | | | 6,27 |

c) 4-[N-(4-Fluor-3-trifluormethylphenyl)sulfamoyl]-benzoesäure-(2,3,4-trihydroxy-butyl-amid)
Analog Beispiel 11b) werden 3,8 g (7,5 mmol) 4-[N-(4-Fluor-3-trifluormethylphenyl)sulfamoyl]-benzoesäure-[2-hydroxy-2-(2,2-dimethyl-1,3-dioxolan-4-yl)-ethyl-amid] in Methanol/Wasser mit Kationenaustauscher in die Titelverbindung überführt. Man erhält 1,74 g = 83 % d.Th. als amorphen Feststoff.

| Analyse: C₁₈H₁₇F₄N₂O₆S MG 465,4 | | | | | | | | | | | | |
|---|---|---|---|---|---|---|---|---|---|---|---|---|
| berechnet | C | 46,45 | H | 3,68 | F | 16,32 | N | 6,01 | O | 20,62 | S | 6,89 % |
| gefunden | | 46,57 | | 3,82 | | 16,48 | | 5,79 | | | | 7,13 |

### Beispiel 42

### 5-[N-(4-Fluor-3-trifluormethylphenyl)sulfamoyl]-isophthalsäure-bis(2,3,4-trihydroxy-butyl-amid)

a) 5-[N-(4-Fluor-3-trifluormethylphenyl)sulfamoyl]-isophthalsäure-dimethylester
Analog Beispiel 12 werden 5 g (27,64 mmol) 4-Fluor-3-(trifluormethyl)-anilin mit 7,32 g (25 mmol) 4-Chlorsulfonyl-isophthalsäuredimethylester umgesetzt und das Rohprodukt durch Chromatographie und anschließende Kristallisation aus Essigester gereinigt. Man erhalt 5,48 g = 45,55 % d. Th.
Fp. 123 - 124 °C.
b) 5-[N-(4-Fluor-3-trifluormethylphenyl)sulfamoyl]-isophthalsäure
Analog Beispiel 13 werden 4,35 g (10 mmol) 5-[N-(4-Fluor-3-trifluormethylphenyl)sulfamoyl]-isophthalsäure-dimethylester alkalisch verseift und das Rohprodukt aus Essigester/Hexan 1:1 kristallisiert. Man erhält 3,49 g = 85,6 % d. Th.
Fp. 286 - 288 °C.
c) 5-[N-(4-Fluor-3-trifluormethylphenyl)sulfamoyl]-isophthalsäure-bis-[2-hydroxy-2-(2,2-dimethyl-1,3-dioxolan-4-yl)-ethylamid]
Analog Beispiel 14a) werden 4,07 g (10 mmol) 5-[N-(4-Fluor-3-trifluormethylphenyl]sulfamoyl]-isophthalsäure mit 2,42 (15 mmol) 2-Hydroxy-2-(2,2-dimethyl-1,3-dioxolan-4-yl)-ethylamin umgesetzt. Das Rohprodukt wird aus Essigester/Hexan kristallisiert. Man erhält 3,02 g = 43,5 % d. Th. Kristallisat.
Fp. 217 - 218 °C.
d) 5-[N-(4-Fluor-3-trifluormethylphenyl)sulfamoyl]-isophthalsäure-bis(2,3,4-trihydroxy-butyl-amid)
Analog Beispiel 14b) werden 4,86 g (7 mmol) 5-[N-(4-Fluor-3-trifluormethyl-phenyl)sulfamoyl]-isophthalsäure-bis[2-hydroxy-2-(2,2-dimethyl-1,3-dioxolan-4-yl)-ethylamid] in Ethanol/Wasser mit verdünnter HCl in die Hydroxy-Verbindung überführt, mit Anionenaustauscher entsalzt und zur Trockne eingedampft. Man erhält 3,37 g = 78,6 % d. Th. als amorphen Feststoff.

| Analyse: C₂₃H₂₇F₄N₃O₁₀S MG 613,54 | | | | | | | | | | | | |
|---|---|---|---|---|---|---|---|---|---|---|---|---|
| berechnet | C | 45,03 | H | 4,44 | F | 12,39 | N | 6,85 | O | 26,08 | S | 5,23 % |
| gefunden | | 44,92 | | 4,28 | | 12,18 | | 6,97 | | | | 5,34 |

### Beispiel 43

### 3-Fluor-6-trifluormethylsulfamoyl-benzoesäure-2,3,4-trihydroxybutyl)amid

a) 3-Fluor-6-(trifluormethylsulfamoyl)-benzoesäuremethylester
Analog Beispiel 21a) werden 3,83 g (20 mmol) 2-Amino-5-fluor-benzoesäurehydrochlorid mit 8,46 g (30 mmol) Trifluormethansulfonsäureanhydrid umgesetzt, die intermediäre Säure in den Methylester überführt und das Rohprodukt durch Chromatographie gereinigt. Man erhält 3,23 g = 53,6 % d. Th. überwiegend kristallines Produkt.

| Analyse: C₉H₇F₄NO₄S MG 301,22 | | | | | | | | | | | | |
|---|---|---|---|---|---|---|---|---|---|---|---|---|
| berechnet | C | 35,88 | H | 2,34 | F | 25,22 | N | 4,65 | O | 21,24 | S | 10,64 % |
| gefunden | | 35,57 | | 2,22 | | 25,48 | | 4,44 | | | | 10,72 |

b) 3-Fluor-6-(trifluormethylsulfamoyl)-benzoesäure
Analog Beispiel 21b) werden 2,41 g (8 mmol) 3-Fluor-6-(trifluormethylsulfamoyl)-benzoesäuremethylester in wäßrigem Dioxan mit NaOH verseift und isoliert, Man erhalt 1,55 g = 67,3 % d. Th. teilweise kristallines Produkt.

| Analyse: C₈H₅F₄NO₄S MG 287,19 | | | | | | | | | | | | |
|---|---|---|---|---|---|---|---|---|---|---|---|---|
| berechnet | C | 33,45 | H | 1,75 | F | 26,46 | N | 4,87 | O | 22,28 | S | 11,16 % |
| gefunden | | 33,73 | | 1,88 | | 26,57 | | 4,62 | | | | 11,37 |

c) 3-Fluor-6-(trifluormethylsulfamoyl)-benzoesäure-[2-hydroxy-2-(2,2-dimethyl-1,3-dioxolan-4-yl)-ethylamid
Analog Beispiel 22a) werden 4,31 g (15 mmol) 3-Fluor-6-(trifluormethylsulfamoyl]-benzoesäure mit 3,22 g (20 mmol) 2-Hydroxy-2-(2,2-dimethyl-1,3-dioxolan-4-yl]-ethylamin umgesetzt und das Rohprodukt durch Chromatographie an Kieselgel mit Essigester/Hexan gereinigt. Man erhält 4,93 g = 76,3 % d. Th. amorphen Feststoff.

| Analyse: C₁₅H₁₈F₄N₂O₆S MG 430,77 | | | | | | | | | | | | |
|---|---|---|---|---|---|---|---|---|---|---|---|---|
| berechnet | C | 41,86 | H | 4,21 | F | 17,65 | N | 6,5 | O | 22,3 | S | 7,45 % |
| gefunden | | 42,07 | | 4,33 | | 17,52 | | 6,67 | | | | 7,27 |

d) 3-Fluor-6-(trifluormethylsulfamoyl)-benzoesäure-(2,3,4-trihydroxy-butyl)amid
Analog Beispiel 22b) werden 5,38 g (12,5 mmol) 3-Fluor-6-(trifluormethylsulfamoyl)-benzoesäure-[2-hydroxy-2-(2,2-dimethyl-1,3-dioxolan-4-yl)-ethyl-amid] in die Titelverbindung überführt. Man erhält 3,52 g = 72,3 % d. Th. amorphen Feststoff.

| Analyse: C₁₂H₁₃F₄N₂O₆S MG 389,3 | | | | | | | | | | | | |
|---|---|---|---|---|---|---|---|---|---|---|---|---|
| berechnet | C | 37,02 | H | 3,36 | F | 19,52 | N | 7,19 | O | 24,65 | S | 8,23 % |
| gefunden | | 36,87 | | 3,6 | | 19,75 | | 7,32 | | | | 7,96 |

### Beispiel 44

### 3-Fluor-6-(trifluormethylsulfamoyl)-benzoesäure-(2,3,4-trihydroxy-N-methylbutyl)amid

a) 3-Fluor-6-(trifluormethylsulfamoyl)-benzoesäure-[2-hydroxy-2-(2,2-dimethyl-1,3-dioxolan-4-yl)-ethyl-N-methyl-amid
Analog Beispiel 22a) werden 4,31 g (15 mmol) 3-Fluor-6-(trifluormethylsulfamoyl)-benzoesäure mit 3,22 g (20 mmol) 2-Methylamino-1-(2,2-dimethyl-1,3-dioxolan-4-yl)-ethanol umgesetzt und das Rohprodukt durch Chromatographie an Kieselgel mit Essigester/Hexan gereinigt. Man erhält 4,93 g = 76,3 % d. Th. amorphen Feststoff.

| Analyse: C₁₆H₂₀F₄N₂O₆S MG 444,4 | | | | | | | | | | | | |
|---|---|---|---|---|---|---|---|---|---|---|---|---|
| berechnet | C | 43,24 | H | 4,53 | F | 17,10 | N | 6,3 | O | 21,6 | S | 7,21 % |
| gefunden | | 42,06 | | 4,33 | | 17,38 | | 6,54 | | | | 7,27 |

b) 3-Fluor-6-(trifluormethylsulfamoyl)-benzoesäure-(2,3,4-trihydroxy-N-methylbutyl)amid
Analog Beispiel 22b) werden 3,11 g (7 mmol) 3-Fluor-6-(trifluormethylsulfamoyl)-benzoesäure-[2-hydroxy-2-(2,2-dimethyl-1,3-dioxolan-4-yl)-ethyl-N-methyl-amid in wäßrigem Methanol mit Ionenaustauscher in die Titelverbindung überführt. Man erhält 2,32 g = 82,3 % d. Th. als amorphen Feststoff.

| Analyse: C₁₃H₁₅F₄N₂O₆S MG 403,33 | | | | | | | | | | | | |
|---|---|---|---|---|---|---|---|---|---|---|---|---|
| berechnet | C | 38,71 | H | 3,74 | F | 18,84 | N | 6,94 | O | 23,8 | S | 7,95 % |
| gefunden | | 38,52 | | 3,95 | | 18,59 | | 7,13 | | | | 7,68 |

### Beispiel 45

### Herstellung einer Lösung von 2-[N-(4-Fluor-3-(trifluormethylphenyl)sulfamoyl]-essigsäure-(2,3,4-trihydroxy-butyl-amid)

202,225 g (0,5 Mol) der in Beispiel 40d) beschriebenen Verbindung werden in 600 ml Wasser p.i. gelöst, 1,211 g Tromethamin und 100 mg CaNa₂EDTA hinzugefügt. Es wird mit 1N HCl auf ph 7,2 eingestellt, mit Wasser p.i. auf 1000 ml aufgefüllt, die Lösung filtriert, in Multivials und/oder Ampullen abgefüllt und hitzesterilisiert.

### Beispiel 46

### Herstellung einer Lösung von 5-[N-(4-Fluor-3-trifluormethylphenyl)sulfamoyl]-isophthalsäure-bis(2,3,4-trihydroxy-butyl-amid)

306,77 g (0,5 Mol) der in Beispiel 42b) beschriebenen Verbindung werden in 600 ml Wasser p.i. gelöst. Die Lösung wird sterilfiltriert und im Multivials und/oder Ampullen abgefüllt.

### Beispiel 47

### Herstellung einer Lösung von 5-[N-(4-Fluor-2-trifluormethylphenyl)sulfamoyl]-isophthalsäure

203,645 g (0,5 Mol) der in Beispiel 13 beschriebenen Verbindung werden in 500 ml Wasser p.i. suspendiert. Durch Zugabe von 5-normaler Natronlauge wird eine Lösung hergestellt, die anschließend mit 0,1-normaler Natronlauge auf pH 7,2 eingestellt wird. Nach Zugabe von 100 mg CaNa₂EDTA wird mit Wasser p.i. auf 1000 ml aufgefüllt, filtriert, in Multivials und/oder Ampullen abgefüllt und hitzesterilisiert.

### Beispiel 48

### 4-[N-(4-Fluor-3-trifluormethylphenyl)-sulfamoyl]-benzoesäure-(2,3-dihydroxy-1-hydroxymethyl-propyl)-amid

a) 4-[N-(4-Fluor-3-trifluormethylphenyl)-sulfamoyl]-benzoesäure-(6-hydroxy-2,2-dimethyl-1,3-dioxepan-5-yl)-amid
In 160 ml Dimethylformamid werden 3 g (8,26 mmol) 4-[N-(Fluor-3-trifluormethylphenyl)-sulfamoyl]-benzoesäure (Beispiel 41a) gegeben und mit 1,27 g (8,26 mmol) Hydroxybenztriazol sowie 1.33 g (8,26 mmol) 6-Amino-2,2-dimethyl-1,3-dioxepan-5-ol (EP 0 033 426) unter Rühren versetzt. Man kühlt auf -10 °C, versetzt mit 1,7 g (8,26 mmol) Dicyclohexylcarbodiimid, läßt 1 Stunde bei dieser Temperatur und weitere 72 Stunden bei Raumtemperatur rühren. Man saugt über ein Glasfaserfilter ab, engt im Vakuum zur Trockne ein und nimmt den Rückstand in Essigester auf. Nach Waschen mit Natriumbicarbonatlösung und Wasser wird die Lösung über Natriumsulfat getrocknet und zur Trockne eingedampft. Der Rückstand wird durch Chromatografie an Kieselgel gereinigt. Man erhält 3,64 g = 87 % d. Th. der Verbindung als amorphen Feststoff.

| Analyse: C₂₁H₂₂F₄N₂O₆S MG 506,47 | | | | | | | | | | | | |
|---|---|---|---|---|---|---|---|---|---|---|---|---|
| Berechnet | C | 49,8 | H | 4,38 | F | 15,0 | N | 5,53 | O | 18,95 | S | 6,33 % |
| Gefunden | | 49,91 | | 4,46 | | 14,87 | | 5,67 | | | | 6,22 % |

b) 4-[N-(4-Fluor-3-trifluormethylphenyl)-sulfamoyl]-benzoesäure-(2,3-dihydroxy-1-hydroxymethyl-propyl)-amid
In eine Lösung aus 10 ml Ethanol und 5 ml destilliertem Wasser werden 2 g (3,95 mmol) 4-[N-(4-Fluor-3-trifluormethylphenyl)-sulfamoyl]-benzoesäure-(6-hydroxy-2,2-dimethyl-1,3-dioxepan-5-yl)-amid sowie 2 ml Kationenaustauscher in der H⁺-Form gegeben und 90 Minuten auf dem Wasserbad erwärmt. Man filtriert vom Austauscher ab, wäscht mit destilliertem Wasser nach, engt im Vakuum zur Trockne ein, nimmt erneut in Wasser auf und erhält durch Gefriertrocknung dieser Lösung 1,55 g = 84,2 % d. Th. als amorphen Feststoff.

| Analyse: C₁₈H₁₈F₄N₂O₆S MG 466,4 | | | | | | | | | | | | |
|---|---|---|---|---|---|---|---|---|---|---|---|---|
| Berechnet | C | 46,36 | H | 3,89 | F | 16,29 | N | 6,01 | O | 20,58 | S | 6,87 % |
| Gefunden | | 46,27 | | 4,02 | | 16,3 | | 5,83 | | | | 6,68 % |

### Durchführung von in-vivo ¹⁹F-NMR-spektroskopischen pH-Bestimmungen mit 5-[N-(4-Fluor-2-trifluormethylphenyl)sulfamoyl]-isophthalsäure) (Beispiel 13)

Es wird eine wäßrige Lösung der Fluorverbindung mit einer Konzentration von 0,4 Mol/l hergestellt. Der pH-Wert der wäßrigen Lösung wurde mit NaOH auf 7.2 eingestellt.

Die NMR-Spektren werden mit einem tierexperimentellen Kernspintomographen (General Electric) gemessen, der mit einer Feldstärke von 2 Tesla arbeitet (entspricht einer RF-Resonanzfrequenz von 80.5 MHz). Durch die Ganzkörperspule (Tiergröße) bzw. Oberflächenspulen können Signale erfaßt werden.

Die Experimente werden mit weiblichen Ratten der Zucht Wistar-Han-Schering (SPF) mit einem Körpergewicht von 200 g durchgeführt. Das Tier wird durch eine intramuskuläre Injektion von Rompun/Ketavet (1:1, 1 ml/kg Körpergewicht) narkotisiert. Anschließend wird dem Tier eine Flügelkanüle in eine der Schwanzvenen gelegt. Das Tier wird in eine Ganzkörper-Spule gelegt und diese auf die Fluor-Resonanzfrequenz abgestimmt. Mit dieser Spule wird das Spektrum des ganzen Tieres erstellt, also keine ortsaufgelöste Spektroskopie durchgeführt.

Die fluorierte Verbindung wird innerhalb von 30 Sekunden in einer Dosis von 0,5 mmol/kg intravenös appliziert und innerhalb von 4 Minuten ein F-Spektrum registriert.

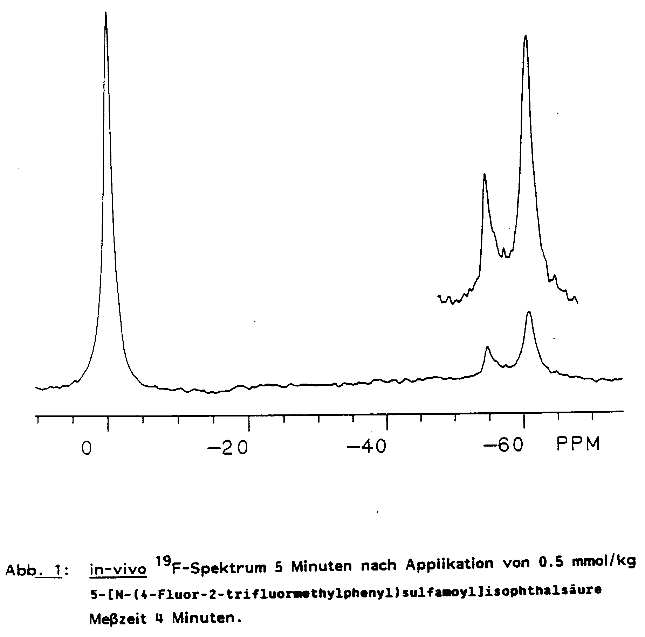

Abbildung 1 zeigt das Fluor-Spektrum, das 5 Minuten nach Applikation erhalten wurde. Das Spektrum besteht aus 3 Signalen, wobei das intensivste Signal dem Signal der CF₃-Gruppe, dem Referenzsignal, entspricht. Die beiden Signale im aromatischen Bereich zeigen, daß sich die Fluorverbindung in 2 Umgebungen mit unterschiedlichem pH-Wert befindet. Die pH-Werte berechnen sich aus den chemischen Verschiebungen zu 6.5 und 7.7.

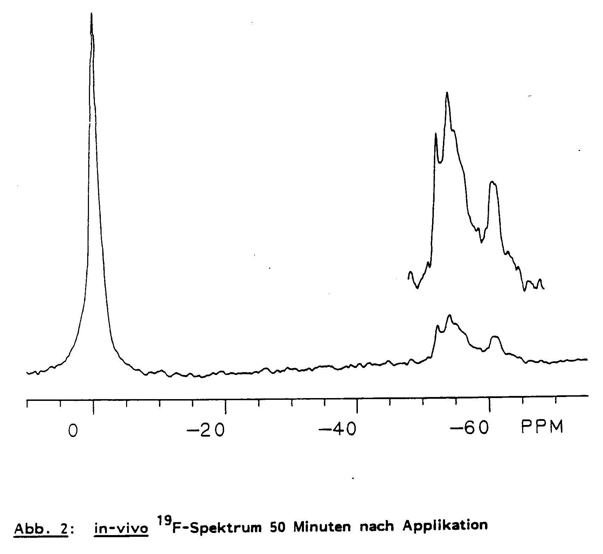

50 Minuten nach Applikation (Abb. 2) ist das Signal aus dem alkalischen Bereich nahezu verschwunden, während das Signal im sauren Bereich in mehrere Signale aufgespalten wurde. Dieses Spektrum zeigt, daß die Verbindung, die sich zu Beginn des Experiments nur im Blut befand, extravaskulär verteilt wurde, und daß unterschiedliche Bereiche im Gewebe verschiedene pH-Werte zeigen. Die Bereiche korrelieren mit dem extrazellulären Raum, intrazellulären Raum (in diesem Fall speziell intrazelluläre Verteilung in Zellen des Nierenparenchyms) sowie Arealen des Nierenparenchyms, der Urether und der Harnblase.

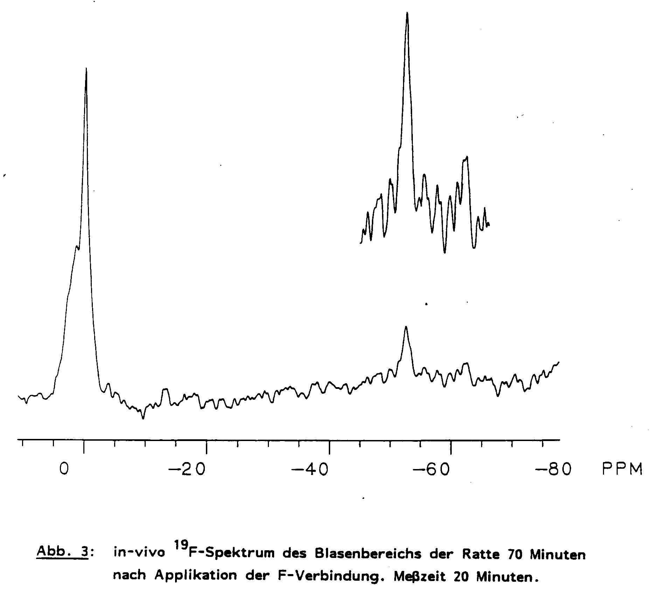

Nach der Messung des gesamten Tieres wird das Tier aus der Spule entnommen und so auf einer Oberflächenspule positioniert, daß der Blasenbereich von der Spule erfaßt wird. Abb. 3 zeigt dieses ortsaufgelöste Spektrum, das in 20 Minuten Meßzeit erhalten wurde. Die intensiven Signale stammen von der Fluorverbindung in der Blase. Die chemische Verschiebung im Aromatenbereich ist identisch mit dem Tieffeldsignal des Aromatenbereichs in Abb. 2. Dieses Spektrum zeigt, daß die Verbindung renal ausgeschieden wird und in der Blase der tiefste (sauerste) pH-Wert im Tier vorliegt.

### Durchführung von ¹⁹F-Imaging

Die Herstellung der Losung der fluorierten Substanz sowie die Vorbereitung des Tieres erfolgt wie bei der Aufnahme der ¹⁹F-NMR-Spektren.

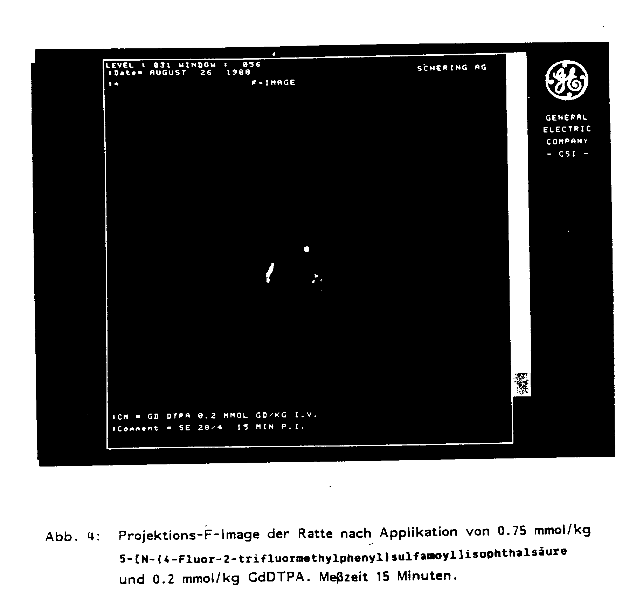

Das Versuchstier wird in die Meßspule gelegt und die fluorierte Verbindung in einer Dosis von 0,75 mmol/kg Körpergewicht über die Schwanzvene in 1 Minute appliziert. Anschließend erfolgt eine intravenöse Applikation, ebenfalls über die Kanüle in der Schwanzvene, von GdDTPA in einer Dosis von 0.2 mmol/kg. Die Aufnahme des Bildes erfolgt mit einer Projektions-Spin-Echo-Sequenz und den Parametern: TR = 28 msec (Wiederholungsrate), TE = 4 msec (Echo-Zeit), NEX = 256 (Anzahl der Mittlungen); die Gesamtmeßzeit beträgt 15 Minuten. Das Image (Abb. 4) zeigt die Niere, die Blase und die Urether, da die untersuchte Verbindung renal ausgeschieden wird und durch Rückresorption von Wasser in diesen Regionen eine hohe F-Konzentration erreicht wird.

## Patentansprüche

1. Fluorsubstituierte Benzolderivate der allgemeinen Formel I 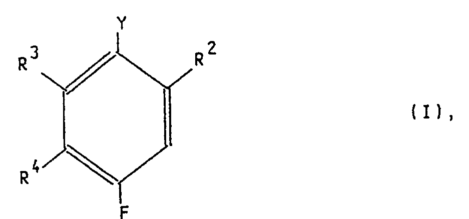 worin
Y eine -NHSO₂R¹-Gruppe mit
R¹ in der Bedeutung einer 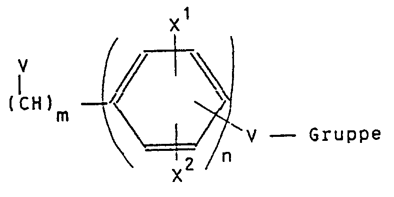 oder einer gerad- oder verzweigtkettigen Alkylengruppe mit 1 bis 10 Kohlenstoffatomen, die gegebenenfalls durch 1 bis 6 Fluoratome substituiert ist und am Ende ein Fluoratom oder den Rest V trägt,
worin V für Wasserstoff
oder die Reste U-OR⁵ oder 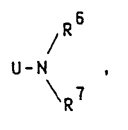 wobei
U einen CO- oder SO₂-Rest,
R⁵ Wasserstoff, einen gesättigten, ungesättigten, gerad- oder verzweigtkettigen oder cyclischen, gegebenenfalls durch 1 bis 6 Hydroxygruppen substituierten Kohlenwasserstoffrest mit bis zu 16 Kohlenstoffatomen,
R⁶ und R⁷ unabhängig voneinander Wasserstoff, eine gerad- oder verzweigtkettige, gegebenenfalls durch 1 bis 6 Hydroxygruppen oder 1 bis 12 Fluoratome substituierte Alkylgruppe mit 1 bis 16 Kohlenstoffatomen oder R⁶ und R⁷ gemeinsam mit dem Stickstoffatom einen gegebenenfalls ein weiteres Heteroatom enthaltenden gesättigten Fünf- oder Sechsring darstellen,
X¹ und X² für Fluor, den Rest V, eine gerad- oder verzweigtkettige Alkylengruppe mit 1 bis 10 Kohlenstoffatomen, die gegebenenfalls durch 1 bis 6 Fluoratome substituiert ist und am Ende ein Fluoratom oder den Rest v trägt, oder den Rest -N(R⁶)-CO-R⁸.
worin R⁸ einen gerad- oder verzweigtkettigen Alkylrest mit 1 bis 16 Kohlenstoffatomen, der gegebenenfalls durch 1 bis 6 Hydroxygruppen und 1 bis 12 Fluoratome substituiert und gegebenenfalls durch 1 bis 3 Sauerstoffatome unterbrochen ist, bedeutet,
m für die Ziffern 0, 1, 2, 3 oder 4,
n für die Ziffern 0 oder 1 stehen, wobei n und m nicht gleichzeitig 0 bedeuten sollen,
R² Wasserstoff, Fluor, den Rest N(R⁶)-CO-R⁸, eine 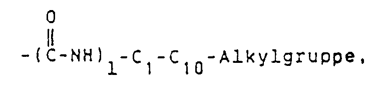 die gerad- oder verzweigtkettig und gegebenenfalls mit 1 bis 6 Fluoratomen substituiert ist, wobei 1 für die Ziffern 0 oder 1 steht, oder einen Rest 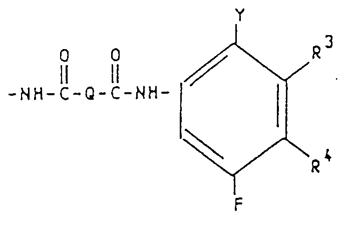 worin Q für eine (CF₂)ₖ- oder C₆F₄-Gruppe mit k in der Bedeutung der Ziffern 1, 2, 3, 4, 5 oder 6 steht,
R³ und R⁴ unabhängig voneinander Wasserstoff, Fluor, eine gerad- oder verzweigtkettige Alkylgruppe mit 1 bis 10 Kohlenstoffatomen, die gegebenenfalls durch 1 bis 6 Fluoratome substituiert ist, den Rest V oder -N(R⁶)-CO-R⁸ bedeuten,
wobei die gegebenenfalls im Molelkül vorhandenen Reste V gleich oder verschieden sein können und freie Säuregruppen mit organischen oder anorganischen Basen versalzt sind, unter den Maßgaben, daß mindestens 2 Fluoratome im Molelkül vorhanden sind, daß für den Fall, daß R⁴ ein Wasserstoffatom und daß einer der beiden Reste R² und R³ für Fluor und der andere für Wasserstoff steht, R¹ nicht eine CH₃-Gruppe sein darf und daß für den Fall, daß R² ein Wasserstoffatom, ein Fluoratom, eine CH₃- oder CF₃-Gruppe, R³ ein Wasserstoffatom und R₄ ein Wasserstoffatom, eine CH₃- oder CF₃-Gruppe ist, das α-Kohlenstoffatom des Restes R¹ nicht fluoriert sein darf.

2. Diagnostische Mittel, enthaltend mindestens eine Verbindung der allgemeinen Formel I. 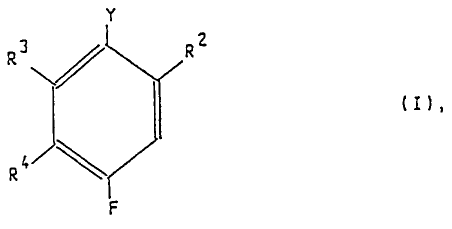 worin
Y eine -NHSO₂R¹-Gruppe mit
R¹ in der Bedeutung einer 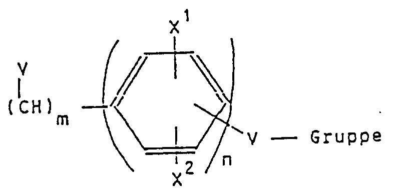 oder einer gerad- oder verzweigtkettigen Alkylengruppe mit 1 bis 10 Kohlenstoffatomen, die gegebenenfalls durch 1 bis 6 Fluoratome substituiert ist und am Ende ein Fluoratom oder den Rest V trägt,
worin V für Wasserstoff
oder die Reste U-OR⁵ oder 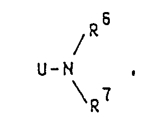 wobei
U einen CO- oder SO₂-Rest,
R⁵ Wasserstoff, einen gesättigten, ungesättigten, gerad- oder verzweigtkettigen oder cyclischen, gegebenenfalls durch 1 bis 6 Hydroxygruppen substituierten Kohlenwasserstoffrest mit bis zu 16 Kohlenstoffatomen,
R⁶ und R⁷ unabhängig voneinander Wasserstoff, eine gerad- oder verzweigtkettige, gegebenenfalls durch 1 bis 6 Hydroxygruppen oder 1 bis 12 Fluoratome substituierte Alkylgruppe mit 1 bis 16 Kohlenstoffatomen oder R⁶ und R⁷ gemeinsam mit dem Stickstoffatom einen gegebenenfalls ein weiteres Heteroatom enthaltenden gesättigten Fünf- oder Sechsring darstellen,
X¹ und X² für Fluor, den Rest V, eine gerad- oder verzweigtkettige Alkylengruppe mit 1 bis 10 Kohlenstoffatomen, die gegebenenfalls durch 1 bis 6 Fluoratome substituiert ist und am Ende ein Fluoratom oder den Rest V trägt, oder den Rest -N(R⁶)-CO-R⁸,
worin R⁸ einen gerad- oder verzweigtkettigen Alkylrest mit 1 bis 16 Kohlenstoffatomen, der gegebenenfalls durch 1 bis 6 Hydroxygruppen und 1 bis 12 Fluoratome substituiert und gegebenenfalls durch 1 bis 3 Sauerstoffatome unterbrochen ist, bedeutet,
m für die Ziffern 0, 1, 2, 3 oder 4,
n für die Ziffern 0 oder 1 stehen, wobei n und m nicht gleichzeitig 0 bedeuten sollen,
R² Wasserstoff, Fluor, den Rest N(R⁶)-CO-R⁸, eine 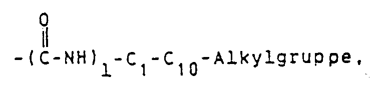 die gerad- oder verzweigtkettig und gegebenenfalls mit 1 bis 6 Fluoratomen substituiert ist, wobei 1 für die Ziffern 0 oder 1 steht, oder einen Rest 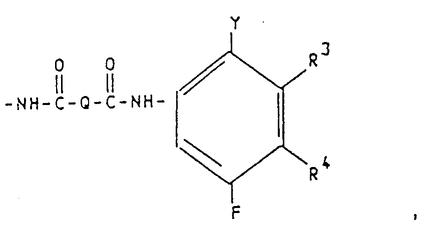 worin Q für eine (CF₂)ₖ- oder C₆F₄-Gruppe mit k in der Bedeutung der Ziffern 1, 2, 3, 4, 5 oder 6 steht,
R³ und R⁴ unabhängig voneinander Wasserstoff, Fluor, eine gerad- oder verzweigtkettige Alkylgruppe mit 1 bis 10 Kohlenstoffatomen, die gegebenenfalls durch 1 bis 6 Fluoratome substituiert ist, den Rest V oder -N(R⁶)-CO-R⁸ bedeuten,
wobei die gegebenenfalls im Molekül vorhandenen Reste V gleich oder verschieden sein können und freie Säuregruppen mit organischen oder anorganischen Basen versalzt sind, unter den Maßgaben, daß mindestens 2 Fluoratome im Molekül vorhanden sind, daß für den Fall, daß R⁴ ein Wasserstoffatom und daß einer der beiden Reste R² und R³ für Fluor und der andere für Wasserstoff steht, R¹ nicht eine CH₃-Gruppe sein darf und daß für den Fall, daß R² ein Wasserstoffatom, ein Fluoratom, eine CH₃- oder CF₃-Gruppe, R³ ein Wasserstoffatom und R₄ ein Wasserstoffatom, eine CH₃- oder CF₃-Gruppe ist, das α-Kohlenstoffatom des Restes R¹ nicht fluoriert sein darf.

3. Verwendung von Verbindungen der allgemeinen Formel I als NMR-Diagnostika. 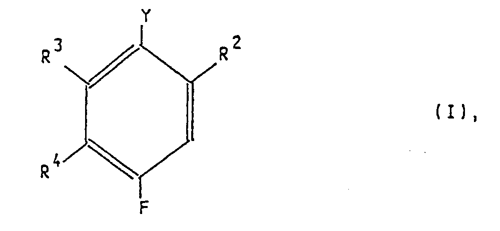 worin
Y eine -NHSO₂R¹-Gruppe mit
R¹ in der Bedeutung einer 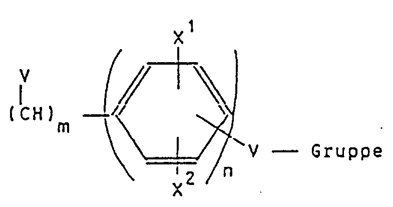 oder einer gerad- oder verzweigtkettigen Alkylengruppe mit 1 bis 10 Kohlenstoffatomen, die gegebenenfalls durch 1 bis 6 Fluoratome substituiert ist und am Ende ein Fluoratom oder den Rest V trägt,
worin V für Wasserstoff
oder die Reste U-OR⁵ oder 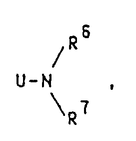 wobei
U einen CO- oder SO₂-Rest,
R⁵ Wasserstoff, einen gesättigten, ungesättigten, gerad- oder verzweigtkettigen oder cyclischen, gegebenenfalls durch 1 bis 6 Hydroxygruppen substituierten Kohlenwasserstoffrest mit bis zu 16 Kohlenstoffatomen,
R⁶ und R⁷ unabhängig voneinander Wasserstoff, eine gerad- oder verzweigtkettige, gegebenenfalls durch 1 bis 6 Hydroxygruppen oder 1 bis 12 Fluoratome substituierte Alkylgruppe mit 1 bis 16 Kohlenstoffatomen oder R⁶ und R⁷ gemeinsam mit dem Stickstoffatom einen gegebenenfalls ein weiteres Heteroatom enthaltenden gesättigten Fünf- oder Sechsring darstellen,
X¹ und X² für Fluor, den Rest V, eine gerad- oder verzweigtkettige Alkylengruppe mit 1 bis 10 Kohlenstoffatomen, die gegebenenfalls durch 1 bis 6 Fluoratome substituiert ist und am Ende ein Fluoratom oder den Rest V trägt, oder den Rest -N(R⁶)-CO-R⁸,
worin R⁸ einen gerad- oder verzweigtkettigen Alkylrest mit 1 bis 16 Kohlenstoffatomen, der gegebenenfalls durch 1 bis 6 Hydroxygruppen und 1 bis 12 Fluoratome substituiert und gegebenenfalls durch 1 bis 3 Sauerstoffatome unterbrochen ist, bedeutet,
m für die Ziffern 0, 1, 2, 3 oder 4,
n für die Ziffern 0 oder 1 stehen, wobei n und m nicht gleichzeitig 0 bedeuten sollen,
R² Wasserstoff, Fluor, den Rest N(R⁶)-CO-R⁸, eine 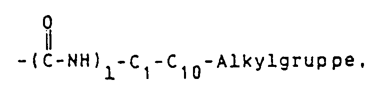 die gerad- oder verzweigtkettig und gegebenenfalls mit 1 bis 6 Fluoratomen substituiert ist, wobei 1 für die Ziffern 0 oder 1 steht, oder einen Rest 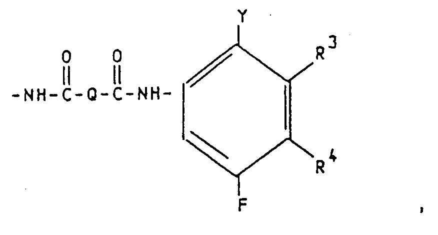 worin Q für eine (CF₂)ₖ- oder C₆F₄-Gruppe mit k in der Bedeutung der Ziffern 1, 2, 3, 4, 5 oder 6 steht,
R³ und R⁴ unabhängig voneinander Wasserstoff, Fluor, eine gerad- oder verzweigtkettige Alkylgruppe mit 1 bis 10 Kohlenstoffatomen, die gegebenenfalls durch 1 bis 6 Fluoratome substituiert ist, den Rest V oder -N(R⁶)-CO-R⁸ bedeuten,
wobei die gegebenenfalls im Molekül vorhandenen Reste V gleich oder verschieden sein können und freie Säuregruppen mit organischen oder anorganischen Basen versalzt sind, daß unter der Maßgabe, daß mindestens 2 Fluoratome im Molekül vorhanden sind.

4. Verfahren zur Herstellung von fluorsubstituierten Benzolderivaten der allgemeinen Formel I 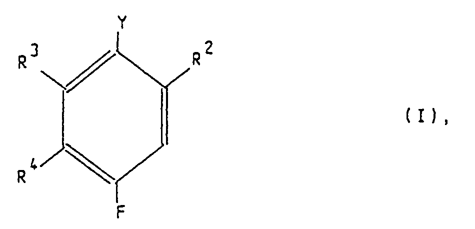 worin
Y eine -NHSO₂R¹-Gruppe mit
R¹ in der Bedeutung einer 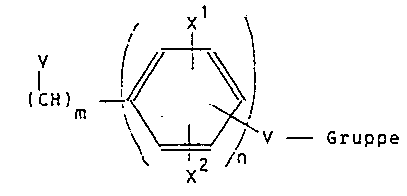 oder einer gerad- oder verzweigtkettigen Alkylengruppe mit 1 bis 10 Kohlenstoffatomen, die gegebenenfalls durch 1 bis 6 Fluoratome substituiert ist und am Ende ein Fluoratom oder den Rest V trägt,
worin V für Wasserstoff
oder die Reste U-OR⁵ oder 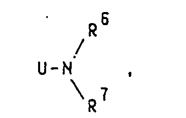 wobei
U einen CO- oder SO₂-Rest,
R⁵ Wasserstoff, einen gesättigten, ungesättigten, gerad- oder verzweigtkettigen oder cyclischen, gegebenenfalls durch 1 bis 6 Hydroxygruppen substituierten Kohlenwasserstoffrest mit bis zu 16 Kohlenstoffatomen,
R⁶ und R⁷ unabhängig voneinander Wasserstoff, eine gerad- oder verzweigtkettige, gegebenenfalls durch 1 bis 6 Hydroxygruppen oder 1 bis 12 Fluoratome substituierte Alkylgruppe mit 1 bis 16 Kohlenstoffatomen oder R⁶ und R⁷ gemeinsam mit dem Stickstoffatom einen gegebenenfalls ein weiteres Heteroatom enthaltenden gesättigten Fünf- oder Sechsring darstellen,
X¹ und X² für Fluor, den Rest V, eine gerad- oder verzweigtkettige Alkylengruppe mit 1 bis 10 Kohlenstoffatomen, die gegebenenfalls durch 1 bis 6 Fluoratome substituiert ist und am Ende ein Fluoratom oder den Rest V trägt, oder den Rest -N(R⁶)-CO-R⁸,
worin R⁸ einen gerad- oder verzweigtkettigen Alkylrest mit 1 bis 16 Kohlenstoffatomen, der gegebenenfalls durch 1 bis 6 Hydroxygruppen und 1 bis 12 Fluoratome substituiert und gegebenenfalls durch 1 bis 3 Sauerstoffatome unterbrochen ist, bedeutet,
m für die Ziffern 0, 1, 2, 3 oder 4,
n für die Ziffern 0 oder 1 stehen, wobei n und m nicht gleichzeitig 0 bedeuten sollen,
R² Wasserstoff, Fluor, den Rest N(R⁶)-CO-R⁸, eine 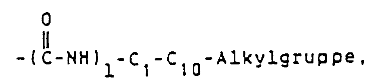 die gerad- oder verzweigtkettig und gegebenenfalls mit 1 bis 6 Fluoratomen substituiert ist, wobei 1 für die Ziffern 0 oder 1 steht, oder einen Rest 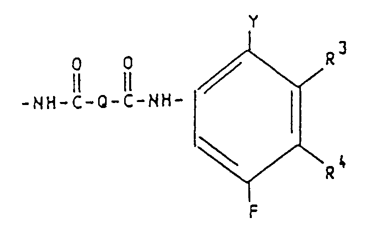 worin Q für eine (CF₂)ₖ- oder C₆F₄-Gruppe mit k in der Bedeutung der Ziffern 1, 2, 3, 4, 5 oder 6 steht,
R³ und R⁴ unabhängig voneinander Wasserstoff, Fluor, eine gerad- oder verzweigtkettige Alkylgruppe mit 1 bis 10 Kohlenstoffatomen, die gegebenenfalls durch 1 bis 6 Fluoratome substituiert ist, den Rest V oder -N(R⁶)-CO-R⁸ bedeuten, wobei die gegebenenfalls im Molekül vorhandenen Reste V gleich oder verschieden sein können und freie Säuregruppen mit organischen oder anorganischen Basen versalzt sind, unter den Maßgaben, daß mindestens 2 Fluoratome im Molekül vorhanden sind, daß für den Fall, daß R⁴ ein Wasserstoffatom und daß einer der beiden Reste R² und R³ für Fluor und der andere für Wasserstoff steht, R¹ nicht eine CH₃-Gruppe sein darf und daß für den Fall, daß R² ein Wasserstoffatom, ein Fluoratom, eine CH₃- oder CF₃-Gruppe, R³ ein Wasserstoffatom und R₄ ein Wasserstoffatom, eine CH₃- oder CF₃-Gruppe ist, das α-Kohlenstoffatom des Restes R¹ nicht fluoriert sein darf,
dadurch gekennzeichnet, daß
Verbindungen der allgemeinen Formel III 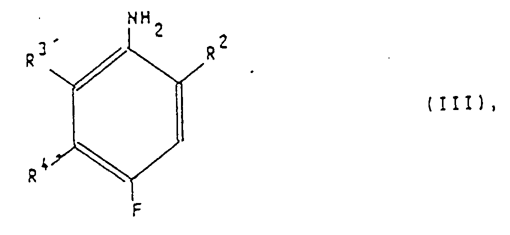 worin
R^{3'} und R^{4'} für R³ und R⁴ oder für in R³ und R⁴ umzuwandelnde Reste stehen. mit Verbindungen der allgemeinen Formel IV oder V
HalSO₂R^{1'} (IV),
(R^{1'}SO₂)₂O (V),
worin
Hal für Chlor, Brom oder Fluor und
R^{1'} für R¹ oder einen in R¹ umzuwandelnden Rest stehen,
umsetzt und gegebenenfalls in den so erhaltenen Reaktionsprodukten die Substituenten R^{1'}, R^{3'} und R^{4'} in die letztlich gewünschten Substituenten R¹, R³ und R⁴ überführt und gewünschtenfalls vorhandene freie Säuregruppen mit organischen oder anorganischen Basen versalzt werden.

5. Verfahren zur Herstellung der diagnostischen Mittel gemäß Anspruch 2, dadurch gekennzeichnet, daß man die in Wasser oder physiologischer Salzlösung gelöste oder suspendierte Benzolverbindung, gegebenenfalls mit den in der Galenik üblichen Zusätzen in eine für die enterale oder parenterale Applikation geeignete Form bringt.

## Claims

1. Fluorine-substituted benzene derivatives of the general formula I 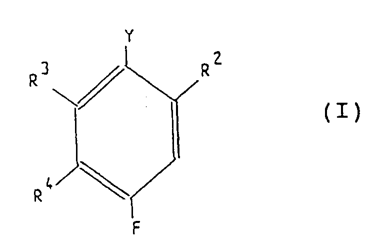 wherein
Y is a -NHSO₂R¹ group wherein
R¹ is a 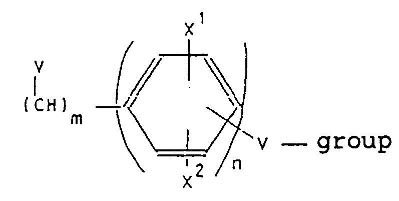 or a straight-chained or branched-chained alkylene group having from 1 to 10 carbon atoms which is optionally substituted by from 1 to 6 fluorine atoms and at its end carries a fluorine atom or the radical V,
wherein V is hydrogen
or the radical U-OR⁵ or 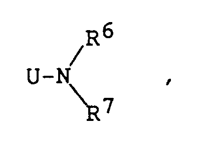 wherein
U is a CO or SO₂ radical,
R⁵ is hydrogen, a saturated, unsaturated, straight-chained or branched-chained or cyclic hydrocarbon radical having up to 16 carbon atoms that is optionally substituted by from 1 to 6 hydroxy groups,
R⁶ and R⁷ are each independently of the other hydrogen, a straight-chained or branched-chained alkyl group having from 1 to 16 carbon atoms that is optionally substituted by from 1 to 6 hydroxy groups or by from 1 to 12 fluorine atoms,
or R⁶ and R⁷, together with the nitrogen atom, form a saturated five- or six-membered ring that optionally contains a further hetero atom,
X¹ and X² are fluorine, the radical V, a straight-chained or branched-chained alkylene group having from 1 to 10 carbon atoms that is optionally substituted by from 1 to 6 fluorine atoms and at its end carries a fluorine atom or the radical V, or X¹ and X² are the radical -N(R⁶)-CO-R⁸,
wherein R⁸ is a straight-chained or branched-chained alkyl radical having from 1 to 16 carbon atoms that is optionally substituted by from 1 to 6 hydroxy groups and by from 1 to 12 fluorine atoms and is optionally interrupted by from 1 to 3 oxygen atoms,
m is 0, 1, 2, 3 or 4 and
n is 0 or 1; n and m may not simultaneously be 0,
R² is hydrogen, fluorine, the radical -N(R⁶)-CO-R⁸, a 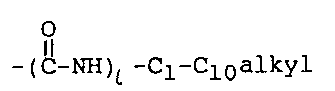 group that is straight-chained or branched-chained and is optionally substituted by from 1 to 6 fluorine atoms, wherein l is 0 or 1, or R² is a radical 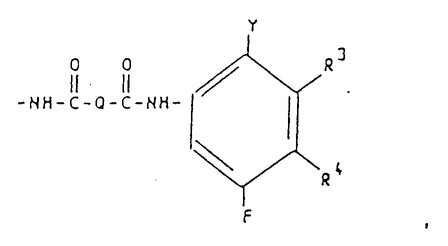 wherein Q is a (CF₂)ₖ group or a C₆F₄ group in which k is 1, 2, 3, 4, 5 or 6,
R³ and R⁴ are each independently of the other hydrogen, fluorine, a straight-chained or branched-chained alkyl group having from 1 to 10 carbon atoms that is optionally substituted by from 1 to 6 fluorine atoms, or the radical V or -N(R⁶)-CO-R⁸,
it being possible for the radicals V optionally present in the molecule to be identical or different, and free acid groups being hydrolysed by organic or inorganic bases,
with the following provisos:
that at least two fluorine atoms are present in the molecule,
that when R⁴ is a hydrogen atom and one of the two radicals R² and R³ is fluorine and the other is hydrogen, R¹ may not be a CH₃ group and
that when R² is a hydrogen atom, a fluorine atom, a CH₃ group or a CF₃ group, R₃ is a hydrogen atom and R₄ is a hydrogen atom, a CH₃ group or a CF₃ group, the α-carbon atom of the radical R¹ may not be fluorinated.

2. Diagnostic compositions comprising at least one compound of the general formula I 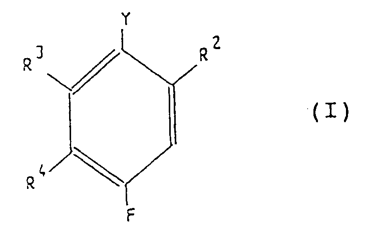 wherein
Y is a -NHSO₂R¹ group wherein
R¹ is a 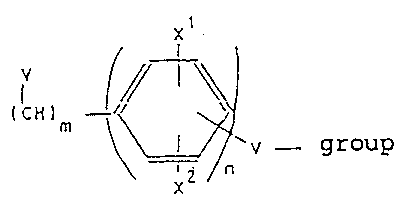 or a straight-chained or branched-chained alkylene group having from 1 to 10 carbon atoms which is optionally substituted by from 1 to 6 fluorine atoms and at its end carries a fluorine atom or the radical V,
wherein V is hydrogen or the radical U-OR⁵ or 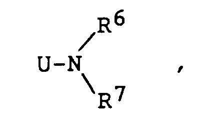 wherein
U is a CO or SO₂ radical,
R⁵ is hydrogen, a saturated, unsaturated, straight-chained or branched-chained or cyclic hydrocarbon radical having up to 16 carbon atoms that is optionally substituted by from 1 to 6 hydroxy groups,
R⁶ and R⁷ are each independently of the other hydrogen, a straight-chained or branched-chained alkyl group having from 1 to 16 carbon atoms that is optionally substituted by from 1 to 6 hydroxy groups or by from 1 to 12 fluorine atoms,
or R⁶ and R⁷, together with the nitrogen atom, form a saturated five- or six-membered ring that optionally contains a further hetero atom,
X¹ and X² are fluorine, the radical V, a straight-chained or branched-chained alkylene group having from 1 to 10 carbon atoms that is optionally substituted by from 1 to 6 fluorine atoms and at its end carries a fluorine atom or the radical V, or X¹ and X² are the radical -N(R⁶)-CO-R⁸,
wherein R⁸ is a straight-chained or branched-chained alkyl radical having from 1 to 16 carbon atoms that is optionally substituted by from 1 to 6 hydroxy groups and by from 1 to 12 fluorine atoms and is optionally interrupted by from 1 to 3 oxygen atoms,
m is 0, 1, 2, 3 or 4 and
n is 0 or 1; n and m may not simultaneously be 0,
R² is hydrogen, fluorine, the radical -N(R⁶)-CO-R⁸, a 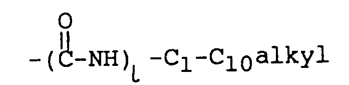 group that is straight-chained or branched-chained and is optionally substituted by from 1 to 6 fluorine atoms, wherein l is 0 or 1, or R² is a radical 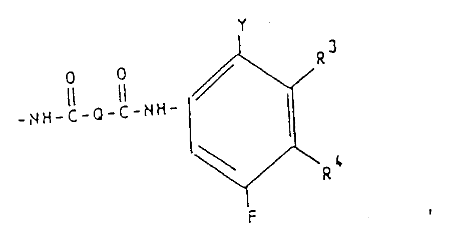 wherein Q is a (CF₂)ₖ group or a C₆F₄ group in which k is 1, 2, 3, 4, 5 or 6,
R³ and R⁴ are each independently of the other hydrogen, fluorine, a straight-chained or branched-chained alkyl group having from 1 to 10 carbon atoms that is optionally substituted by from 1 to 6 fluorine atoms, or the radical V or -N(R⁶)-CO-R⁸,
it being possible for the radicals V optionally present in the molecule to be identical or different, and free acid groups being hydrolysed by organic or inorganic bases,
with the following provisos:
that at least two fluorine atoms are present in the molecule,
that when R⁴ is a hydrogen atom and one of the two radicals R² and R³ is fluorine and the other is hydrogen, R¹ may not be a CH₃ group and
that when R² is a hydrogen atom, a fluorine atom, a CH₃ group or a CF₃ group, R₃ is a hydrogen atom and R₄ is a hydrogen atom, a CH₃ group or a CF₃ group, the α-carbon atom of the radical R¹ may not be fluorinated.

3. Use of compounds of the general formula I as NMR diagnostic agents 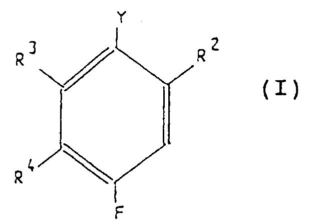 wherein
Y is a -NHSO₂R¹ group wherein
R¹ is a 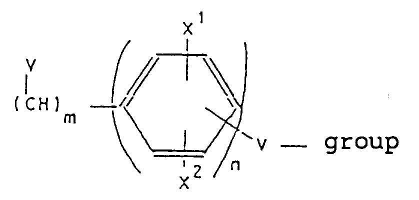 or a straight-chained or branched-chained alkylene group having from 1 to 10 carbon atoms which is optionally substituted by from 1 to 6 fluorine atoms and at its end carries a fluorine atom or the radical V,
wherein V is hydrogen
or the radical U-OR⁵ or 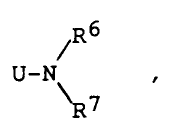 wherein
U is a CO or SO₂ radical,
R⁵ is hydrogen, a saturated, unsaturated, straight-chained or branched-chained or cyclic hydrocarbon radical having up to 16 carbon atoms that is optionally substituted by from 1 to 6 hydroxy groups,
R⁶ and R⁷ are each independently of the other hydrogen, a straight-chained or branched-chained alkyl group having from 1 to 16 carbon atoms that is optionally substituted by from 1 to 6 hydroxy groups or by from 1 to 12 fluorine atoms,
or R⁶ and R⁷, together with the nitrogen atom, form a saturated five- or six-membered ring that optionally contains a further hetero atom,
X¹ and X² are fluorine, the radical V, a straight-chained or branched-chained alkylene group having from 1 to 10 carbon atoms that is optionally substituted by from 1 to 6 fluorine atoms and at its end carries a fluorine atom or the radical V, or X¹ and X² are the radical -N(R⁶)-CO-R⁸,
wherein R⁸ is a straight-chained or branched-chained alkyl radical having from 1 to 16 carbon atoms that is optionally substituted by from 1 to 6 hydroxy groups and by from 1 to 12 fluorine atoms and is optionally interrupted by from 1 to 3 oxygen atoms,
m is 0, 1, 2, 3 or 4 and
n is 0 or 1; n and m may not simultaneously be 0,
R² is hydrogen, fluorine, the radical -N(R⁶)-CO-R⁸, a 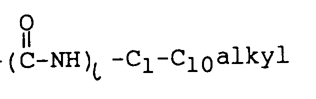 group that is straight-chained or branched-chained and is optionally substituted by from 1 to 6 fluorine atoms, wherein l is 0 or 1, or R² is a radical 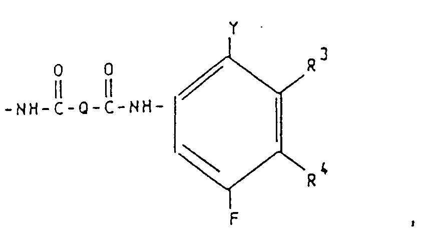 wherein Q is a (CF₂)ₖ group or a C₆F₄ group in which k is 1, 2, 3, 4, 5 or 6,
R³ and R⁴ are each independently of the other hydrogen, fluorine, a straight-chained or branched-chained alkyl group having from 1 to 10 carbon atoms that is optionally substituted by from 1 to 6 fluorine atoms, or the radical V or -N(R⁶)-CO-R⁸,
it being possible for the radicals V optionally present in the molecule to be identical or different, and free acid groups being hydrolysed by organic or inorganic bases,
with the following proviso:
that at least two fluorine atoms are present in the molecule.

4. Processes for the preparation of fluorine-substituted benzene derivatives of the general formula I 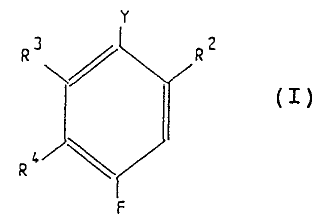 wherein
Y is a -NHSO₂R¹ group wherein
R¹ is a 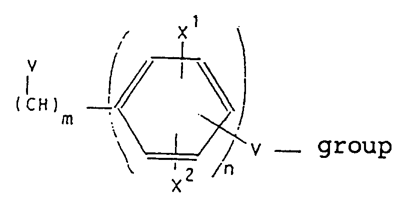 or a straight-chained or branched-chained alkylene group having from 1 to 10 carbon atoms which is optionally substituted by from 1 to 6 fluorine atoms and at its end carries a fluorine atom or the radical V,
wherein V is hydrogen
or the radical U-OR⁵ or 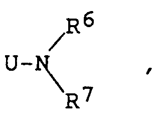 wherein
U is a CO or SO₂ radical,
R⁵ is hydrogen, a saturated, unsaturated, straight-chained or branched-chained or cyclic hydrocarbon radical having up to 16 carbon atoms that is optionally substituted by from 1 to 6 hydroxy groups,
R⁶ and R⁷ are each independently of the other hydrogen, a straight-chained or branched-chained alkyl group having from 1 to 16 carbon atoms that is optionally substituted by from 1 to 6 hydroxy groups or by from 1 to 12 fluorine atoms,
or R⁶ and R⁷, together with the nitrogen atom, form a saturated five- or six-membered ring that optionally contains a further hetero atom,
X¹ and X² are fluorine, the radical V, a straight-chained or branched-chained alkylene group having from 1 to 10 carbon atoms that is optionally substituted by from 1 to 6 fluorine atoms and at its end carries a fluorine atom or the radical V, or X¹ and X² are the radical -N(R⁶)-CO-R⁸,
wherein R⁸ is a straight-chained or branched-chained alkyl radical having from 1 to 16 carbon atoms that is optionally substituted by from 1 to 6 hydroxy groups and by from 1 to 12 fluorine atoms and is optionally interrupted by from 1 to 3 oxygen atoms,
m is 0, 1, 2, 3 or 4 and
n is 0 or 1; n and m may not simultaneously be 0,
R² is hydrogen, fluorine, the radical -N(R⁶)-CO-R⁸, a 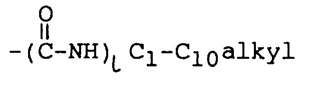 group that is straight-chained or branched-chained and is optionally substituted by from 1 to 6 fluorine atoms, wherein l is 0 or 1, or R² is a radical 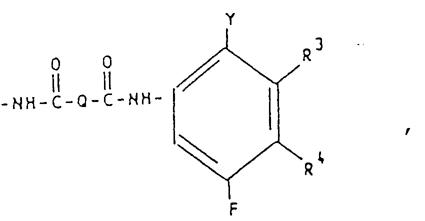 wherein Q is a (CF₂)ₖ group or a C₆F₄ group in which k is 1, 2, 3, 4, 5 or 6,
R³ and R⁴ are each independently of the other hydrogen, fluorine, a straight-chained or branched-chained alkyl group having from 1 to 10 carbon atoms that is optionally substituted by from 1 to 6 fluorine atoms, or the radical V or -N(R⁶)-CO-R⁸,
it being possible for the radicals V optionally present in the molecule to be identical or different, and free acid groups being hydrolysed by organic or inorganic bases,
with the following provisos:
that at least two fluorine atoms are present in the molecule,
that when R⁴ is a hydrogen atom and one of the two radicals R² and R³ is fluorine and the other is hydrogen, R¹ may not be a CH₃ group and
that when R² is a hydrogen atom, a fluorine atom, a CH₃ group or a CF₃ group, R₃ is a hydrogen atom and R₄ is a hydrogen atom, a CH₃ group or a CF₃ group, the α-carbon atom of the radical R¹ may not be fluorinated.

5. Process for the preparation of the diagnostic agents according to claim 2, characterised in that the benzene compound, dissolved or suspended in water or physiological salt solution, is brought into a form suitable for enteral or parenteral administration, optionally together with the additives customarily used in pharmaceuticals.

## Revendications

1. Dérivés substitués fluorés du benzène de formule générale I 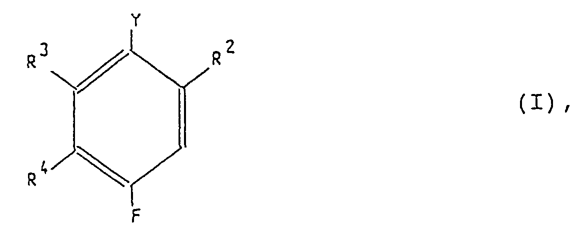 dans laquelle
Y est un groupe -NHSO₂R¹,
R¹ représentant un groupe 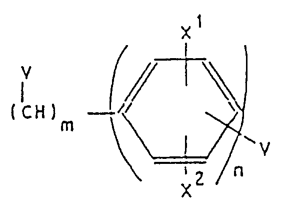 ou encore un groupe alkylène à chaîne droite ou ramifiée ayant de 1 à 10 atomes de carbone, et qui est éventuellement substitué par 1 à 6 atomes de fluor et porte en position terminale un atome de fluor ou le radical V,
où V est un hydrogène ou il représente l'un des radicaux U-OR⁵ ou 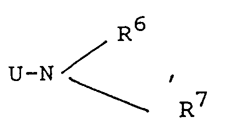 où U est un radical CO- ou SO₂ ; R⁵ est un hydrogène ou un radical hydrocarboné ayant jusqu'à 16 atomes de carbone, sature, insaturé, a chaîne droite ou ramifiée, ou cyclique, éventuellement substitué par 1 à 6 groupes hydroxy ;
R⁶ et R⁷, indépendamment l'un de l'autre, sont chacun un hydrogène ou un groupe alkyle ayant de 1 à 16 atomes de carbone, à chaîne droite ou ramifiée, éventuellement substitué par 1 à 6 groupes hydroxy ou 1 à 12 atomes de fluor, ou bien encore R⁶ et R⁷, avec l'atome d'azote, forment un noyau à 5 ou 6 chaînons, saturé, contenant éventuellement un autre hétéroatome ;
X¹ et X² sont chacun le fluor, le radical V ou un groupe alkylène à chaîne droite ou ramifiée ayant de 1 à 10 atomes de carbone, substitué par 1 à 6 atomes de fluor et portant en position terminale un atome de fluor ou le radical V, ou encore le radical -N(R⁶)-CO-R⁸, où R⁸ est un radical alkyle à chaîne droite ou ramifiée ayant de 1 à 16 atomes de carbone, éventuellement substitué par 1 à 6 groupes hydroxy et 1 à 12 atomes de fluor et éventuellement interrompu par 1 à 3 atomes d'oxygène ;
m est l'un des nombres 0, 1, 2, 3 ou 4,
n est l'un des nombres 0 ou 1, n et m ne devant pas être simultanément égaux à 0 ;
R² est un hydrogène, le radical N(R⁶)-CO-R⁸, un groupe 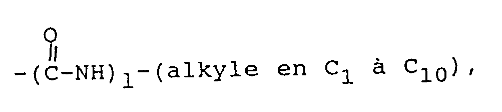 à chaîne droite ou ramifiée et éventuellement substitué par 1 à 6 atomes de fluor, l valant 0 ou 1, ou encore un radical 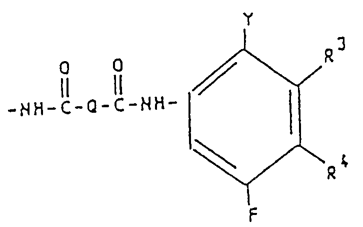 dans lequel Q est un groupe (CF₂)ₖ ou C₆F₄, k représentant l'un des nombres 1, 2, 3, 4, 5 ou 6,
R³ et R⁴, indépendamment l'un de l'autre, sont chacun un hydrogène, un fluor ou un groupe alkyle à chaîne droite ou ramifiée ayant de 1 à 10 atomes de carbone, éventuellement substitué par 1 à 6 atomes de carbone, le radical V ou encore -N(R⁶)-CO-R⁸, auquel cas les radicaux Y éventuellement présents dans la molécule peuvent être identiques ou différents, les groupes acides libres étant salifiés par des bases organiques ou minérales,
à condition qu'au moins deux atomes de fluor soient présents dans la molécule ; que, lorsque R⁴ est un atome d'hydrogène et que l'un des deux radicaux R² et R³ est un fluor et l'autre un hydrogène, R¹ ne soit pas un groupe CH₃, et que, lorsque R² est un atome d'hydrogène, un atome de fluor, un groupe CH₃ ou CF₃, que R³ est un atome d'hydrogène et R⁴ un atome d'hydrogène, un groupe CH₃ ou CF₃, l'atome de carbone α du radical R¹ ne soit pas fluoré,
conviennent d'une manière suprenante à la réalisation de diagnostics en RMN.

2. Agent de diagnostic, contenant au moins un composé de formule générale I 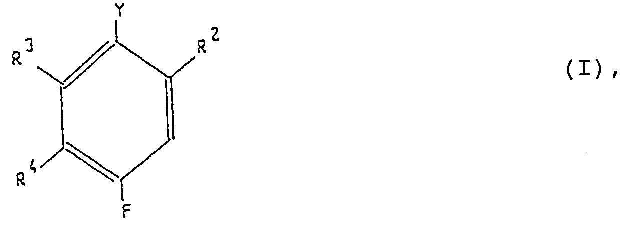 dans laquelle
Y est un groupe -NHSO₂R¹,
R¹ représentant un groupe 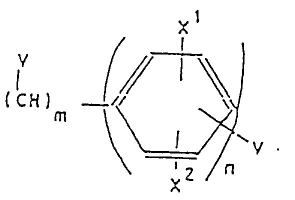 ou encore un groupe alkylène à chaîne droite ou ramifiée ayant de 1 à 10 atomes de carbone, et qui est éventuellement substitué par 1 à 6 atomes de fluor et porte en position terminale un atome de fluor ou le radical V,
où V est un hydrogène ou il représente l'un des radicaux U-OR⁵ ou 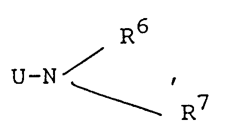 où U est un radical CO- ou SO₂ ; R⁵ est un hydrogène ou un radical hydrocarboné ayant jusqu'à 16 atomes de carbone, saturé, insaturé, à chaîne droite ou ramifiée, ou cyclique, éventuellement substitué par 1 à 6 groupes hydroxy ;
R⁶ et R⁷, indépendamment l'un de l'autre, sont chacun un hydrogène ou un groupe alkyle ayant de 1 à 16 atomes de carbone, à chaîne droite ou ramifiée, éventuellement substitué par 1 à 6 groupes hydroxy ou 1 à 12 atomes de fluor, ou bien encore R⁶ et R⁷, avec l'atome d'azote, forment un noyau à 5 ou 6 chaînons, saturé, contenant éventuellement un autre hétéroatome ;
X¹ et X² sont chacun le fluor, le radical V ou un groupe alkylène à chaîne droite ou ramifiée ayant de 1 à 10 atomes de carbone, substitué par 1 à 6 atomes de fluor et portant en position terminale un atome de fluor ou le radical V, ou encore le radical -N(R⁶)-CO-R⁸, où R⁸ est un radical alkyle à chaîne droite ou ramifiée ayant de 1 à 16 atomes de carbone, éventuellement substitué par 1 à 6 groupes hydroxy et 1 à 12 atomes de fluor et éventuellement interrompu par 1 à 3 atomes d'oxygène ;
m est l'un des nombres 0, 1, 2, 3 ou 4,
n est l'un des nombres 0 ou 1, n et m ne devant pas être simultanément égaux à 0 ;
R² est un hydrogène, le radical N(R⁶)-CO-R⁸, un groupe 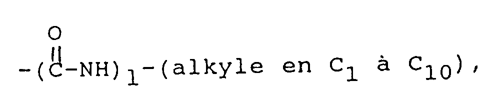 à chaîne droite ou ramifiée et éventuellement substitué par 1 à 6 atomes de fluor, l valant 0 ou 1, ou encore un radical 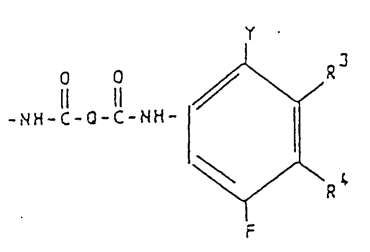 dans lequel Q est un groupe (CF₂)ₖ ou C₆F₄, k représentant l'un des nombres 1, 2, 3, 4, 5 ou 6,
R³ et R⁴, indépendamment l'un de l'autre, sont chacun un hydrogène, un fluor ou un groupe alkyle à chaîne droite ou ramifiée ayant de 1 à 10 atomes de carbone, éventuellement substitué par 1 à 6 atomes de carbone, le radical V ou encore -N(R⁶)-CO-R⁸, auquel cas les radicaux V éventuellement présents dans la molécule peuvent être identiques ou différents, les groupes acides libres étant salifiés par des bases organiques ou minérales,
à condition qu'au moins deux atomes de fluor soient présents dans la molécule ; que, lorsque R⁴ est un atome d'hydrogène et que l'un des deux radicaux R² et R³ est un fluor et l'autre un hydrogène, R¹ ne soit pas un groupe CH₃, et que, lorsque R² est un atome d'hydrogène, un atome de fluor, un groupe CH₃ ou CF₃, que R³ est un atome d'hydrogène et R⁴ un atome d'hydrogène, un groupe CH₃ ou CF₃, l'atome de carbone α du radical R¹ ne soit pas fluoré,
conviennent d'une manière suprenante à la réalisation de diagnostics en RMN.

3. Utilisation de composés de formule générale I en tant qu'agents de diagnostic en RMN, 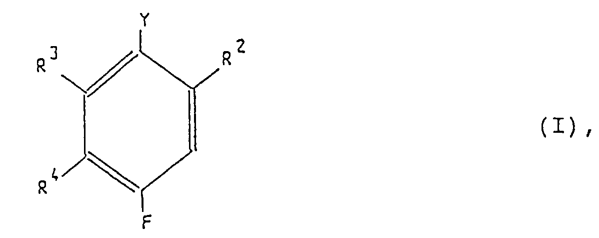 dans laquelle
Y est un groupe -NHSO₂R¹,
R¹ représentant un groupe 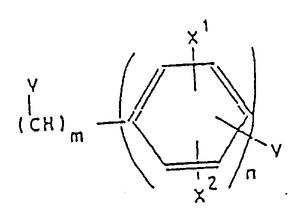 ou encore un groupe alkylène à chaîne droite ou ramifiée ayant de 1 à 10 atomes de carbone, et qui est éventuellement substitué par 1 à 6 atomes de fluor et porte en position terminale un atome de fluor ou le radical V,
où V est un hydrogène ou il représente l'un des radicaux U-OR⁵ ou 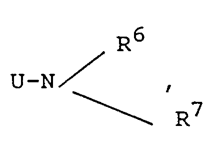 où U est un radical CO- ou SO₂ ; R⁵ est un hydrogène ou un radical hydrocarboné ayant jusqu'à 16 atomes de carbone, saturé, insaturé, à chaîne droite ou ramifiée, ou cyclique, éventuellement substitué par 1 à 6 groupes hydroxy ;
R⁶ et R⁷, indépendamment l'un de l'autre, sont chacun un hydrogène ou un groupe alkyle ayant de 1 à 16 atomes de carbone, à chaîne droite ou ramifiée, éventuellement substitué par 1 à 6 groupes hydroxy ou 1 à 12 atomes de fluor, ou bien encore R⁶ et R⁷, avec l'atome d'azote, forment un noyau a 5 ou 6 chaînons, saturé, contenant éventuellement un autre hétéroatome ;
X¹ et X² sont chacun le fluor, le radical V ou un groupe alkylène à chaîne droite ou ramifiée ayant de 1 à 10 atomes de carbone, substitué par 1 à 6 atomes de fluor et portant en position terminale un atome de fluor ou le radical V, ou encore le radical -N(R⁶)-CO-R⁸, où R⁸ est un radical alkyle à chaîne droite ou ramifiée ayant de 1 à 16 atomes de carbone, éventuellement substitué par 1 à 6 groupes hydroxy et 1 à 12 atomes de fluor et éventuellement interrompu par 1 à 3 atomes d'oxygène ;
m est l'un des nombres 0, 1, 2, 3 ou 4,
n est l'un des nombres 0 ou 1, n et m ne devant pas être simultanément égaux à 0 ;
R² est un hydrogène, le radical N(R⁶)-CO-R⁸, un groupe 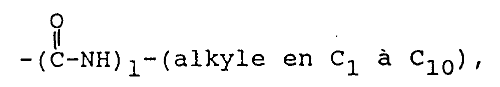 à chaîne droite ou ramifiée et éventuellement substitué par 1 à 6 atomes de fluor, l valant 0 ou 1, ou encore un radical 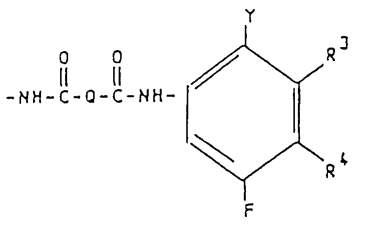 dans lequel Q est un groupe (CF₂)ₖ ou C₆F₄, k représentant l'un des nombres 1, 2, 3, 4, 5 ou 6,
R³ et R⁴, indépendamment l'un de l'autre, sont chacun un hydrogène, un fluor ou un groupe alkyle à chaîne droite ou ramifiée ayant de 1 à 10 atomes de carbone, éventuellement substitué par 1 à 6 atomes de carbone, le radical V ou encore -N(R⁶)-CO-R⁸, auquel cas les radicaux V éventuellement présents dans la molécule peuvent être identiques ou différents, les groupes acides libres étant salifiés par des bases organiques ou minérales,
à condition qu'au moins deux atomes de fluor soient présents dans la molécule.

4. Procédé pour préparer des dérivés fluorés du benzène de formule générale I 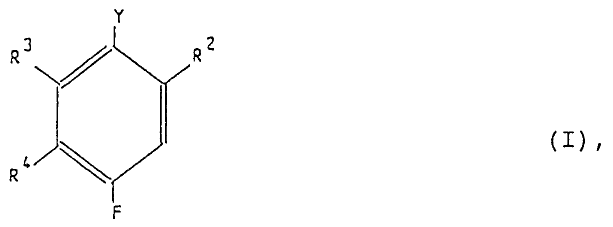 dans laquelle
Y est un groupe -NHSO₂R¹,
R¹ représentant un groupe 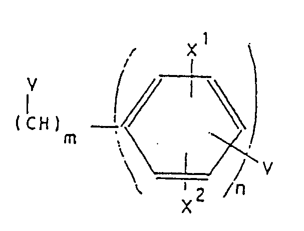 ou encore un groupe alkylène à chaîne droite ou ramifiée ayant de 1 à 10 atomes de carbone, et qui est éventuellement substitué par 1 à 6 atomes de fluor et porte en position terminale un atome de fluor ou le radical V,
où V est un hydrogène ou il représente l'un des radicaux U-OR⁵ ou 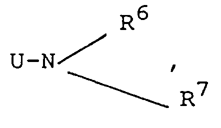 où U est un radical CO- ou SO₂ ; R⁵ est un hydrogène ou un radical hydrocarboné ayant jusqu'à 16 atomes de carbone, saturé, insaturé, a chaîne droite ou ramifiée, ou cyclique, éventuellement substitué par 1 à 6 groupes hydroxy ;
R⁶ et R⁷, indépendamment l'un de l'autre, sont chacun un hydrogène ou un groupe alkyle ayant de 1 à 16 atomes de carbone, à chaîne droite ou ramifiée, éventuellement substitué par 1 à 6 groupes hydroxy ou 1 à 12 atomes de fluor, ou bien encore R⁶ et R⁷, avec l'atome d'azote, forment un noyau à 5 ou 6 chaînons, saturé, contenant éventuellement un autre hétéroatome ;
X¹ et X² sont chacun le fluor, le radical V ou un groupe alkylène à chaîne droite ou ramifiée ayant de 1 à 10 atomes de carbone, substitué par 1 à 6 atomes de fluor et portant en position terminale un atome de fluor ou le radical V, ou encore le radical -N(R⁶)-CO-R⁸, où R⁸ est un radical alkyle à chaîne droite ou ramifiée ayant de 1 à 16 atomes de carbone, éventuellement substitué par 1 à 6 groupes hydroxy et 1 à 12 atomes de fluor et éventuellement interrompu par 1 à 3 atomes d'oxygène ;
m est l'un des nombres 0, 1, 2, 3 ou 4,
n est l'un des nombres 0 ou 1, n et m ne devant pas être simultanément égaux à 0 ;
R² est un hydrogène, le radical N(R⁶)-CO-R⁸, un groupe 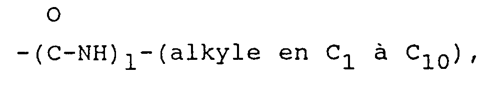 à chaîne droite ou ramifiée et éventuellement substitué par 1 à 6 atomes de fluor, l valant 0 ou 1, ou encore un radical 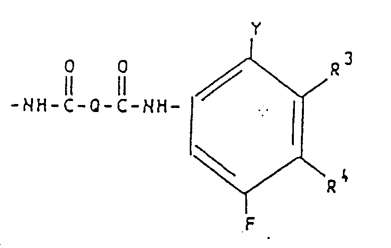 dans lequel Q est un groupe (CF₂)ₖ ou C₆F₄, k représentant l'un des nombres 1, 2, 3, 4, 5 ou 6,
R³ et R⁴, indépendamment l'un de l'autre, sont chacun un hydrogène, un fluor ou un groupe alkyle à chaîne droite ou ramifiée ayant de 1 à 10 atomes de carbone, éventuellement substitué par 1 à 6 atomes de carbone, le radical V ou encore -N(R⁶)-CO-R⁸, auquel cas les radicaux V éventuellement présents dans la molécule peuvent être identiques ou différents, les groupes acides libres étant salifiés par des bases organiques ou minérales,
à condition qu'au moins deux atomes de fluor soient présents dans la molécule ; que, quand R⁴ est un atome d'hydrogène et que l'un des deux radicaux R² et R³ est un fluor et l'autre un hydrogène, R¹ ne soit pas un groupe CH₃, et que, quand R² est un atome d'hydrogène, un atome de fluor, un groupe CH₃ ou CF₃, que R³ est un atome d'hydrogène et R⁴ un atome d'hydrogène, un groupe CH₃ ou CF₃, l'atome de carbone α du radical R¹ ne soit pas fluoré

5. Procédé pour préparer les agents diagnostiques selon la revendication 2, caractérisé en ce qu'on transforme en une forme convenant à une administration par voie entérale ou parentérale le composé du benzène, en solution ou en suspension dans l'eau ou dans une solution saline physiologique, éventuellement avec les additifs usuels orgaléniques.
